(19) Europäisches Patentamt / European Patent Office / Office européen des brevets

(11) **EP 4 032 539 A1**

(12) **EUROPEAN PATENT APPLICATION**
published in accordance with Art. 153(4) EPC

(43) Date of publication:
27.07.2022 Bulletin 2022/30

(21) Application number: 20865705.6

(22) Date of filing: 20.09.2020

(51) International Patent Classification (IPC):
$A61K\ 38/20^{(2006.01)}$    $A61K\ 39/395^{(2006.01)}$
$A61P\ 35/00^{(2006.01)}$    $C07K\ 14/54^{(2006.01)}$
$C07K\ 14/715^{(2006.01)}$    $C07K\ 16/28^{(2006.01)}$
$C07K\ 19/00^{(2006.01)}$    $C12N\ 15/13^{(2006.01)}$
$C12N\ 15/24^{(2006.01)}$    $C12N\ 15/62^{(2006.01)}$

(52) Cooperative Patent Classification (CPC):
A61K 38/19; A61K 38/20; A61K 39/395;
A61P 35/00; C07K 14/54; C07K 14/715;
C07K 16/28; C07K 19/00; C12N 15/62

(86) International application number:
PCT/RU2020/050233

(87) International publication number:
WO 2021/054867 (25.03.2021 Gazette 2021/12)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 19.09.2019 RU 2019129569

(71) Applicant: Joint Stock Company "Biocad"
St.Petersburg 198515 (RU)

(72) Inventors:
• ULITIN, Andrei Borisovich
 G.Puschino, 142290 (RU)
• KONONOV, Aleksey Vladimirovich
 g. Pushchino, 142290 (RU)

• AGEEV, Sergei Andreevich
 G. Chehov, 142301 (RU)
• GORDEEV, Aleksandr Andreevich
 Izhevsk, 426011 (RU)
• VINOGRADOVA, Elena Vladimirovna
 Saint Petersburg, 198504 (RU)
• EVDOKIMOV, Stanislav Rudolfovich
 Pushchino, 142290 (RU)
• SHMAKOVA, Aleksandra Pavlovna
 Pushkin, 196603 (RU)
• MITROSHIN, Ivan Vladimirovich
 Izhevsk, 426050 (RU)
• MOROZOV, Dmitry Valentinovich
 Saint Petersburg, 190000 (RU)

(74) Representative: Held, Stephan
 Meissner Bolte Patentanwälte
 Rechtsanwälte Partnerschaft mbB
 Widenmayerstraße 47
 80538 München (DE)

(54) **IMMUNOCYTOKINE COMPRISING HETERODIMERIC PROTEIN COMPLEX BASED ON IL-15/IL-15R ALPHA**

(57) The present invention relates to an immunocytokine comprising an heterodimeric protein complex based on IL-15/IL-15Rα, and to use thereof as a therapeutic agent, in particular as an agent for the treatment of cancer and an autoimmune disease. The present invention further relates to an immunocytokine comprising a heterodimeric protein complex based on an IL-15/IL-15Rα and an immunomodulatory antibody, and to use thereof as a therapeutic agent, in particular as an agent for the treatment of cancer and an autoimmune disease.

EP 4 032 539 A1

**Description**

FIELD OF INVENTION

The present invention relates to an immunocytokine comprising a heterodimeric protein complex based on IL-15/IL-15Rα, and to use thereof as a therapeutic agent, in particular as an agent for the treatment of cancer and an autoimmune disease. The present invention further relates to an immunocytokine comprising a heterodimeric protein complex based on IL-15/IL-15Rα and an immunomodulatory antibody, and to use thereof as a therapeutic agent, in particular as an agent for the treatment of cancer and an autoimmune disease.

BACKGROUND OF INVENTION

Cytokines are a category of signaling proteins and glycoproteins, which, like hormones and neurotransmitters, are extensively used in cellular communication. While hormones are secreted from specific organs to the blood, and neurotransmitters are related to neural activity, the cytokines are a more diverse class of compounds in terms of origin and purpose. They are produced by a wide variety of hematopoietic and non-hematopoietic cells and can effect both nearby cells and the whole organism, and sometimes are strongly dependent on the presence of other chemicals. The cytokine family consists mainly of small, water-soluble proteins and glycoproteins with a mass ranging between 8 and 30 kDa. Cytokines are critical for the development and functioning of both the innate and adaptive immune responses. They are often secreted by immune cells that have encountered a pathogen, thereby activating and recruiting further immune cells to increase the system's response to the pathogen.

Among the cytokines, interleukin 15 (IL-15) is a cytokine with structural similarity to IL-2; the former is secreted by mononuclear phagocytes (and some other cells) following infection with virus(es) or indirect stimulation by cells that are recognized as "non-self" or weakened. This cytokine induces cell proliferation of natural killer cells; cells of the innate immune system whose principal role is to kill virally infected cells. The protein encoded by this gene is a cytokine that regulates T cell and natural killer cell activation and proliferation.

Interleukin-15 (IL-15) is a 14-15 kDa glycoprotein simultaneously identified by two groups as a T cell-activating factor (Grabstein, K.H. et al., Science 1994, 264, 965; Burton, J.D. et al., Proc. Natl. Acad. Sci. USA 1994, 91, 4935). IL-15 mRNA is widely expressed in different cells and tissues, however, it is difficult to find the protein in these cells or in the cellular supernatant due to a strong post-transcriptional control of expression thereof at the level of translation and intracellular traffic (Bamford RN. et al., J. Immunol. 1998, 160: 4418-4426; Kurys G, et al., J. Biol. Chem. 2000, 275: 30653-30659). Furthermore, it has been shown that IL-15 may exist in an active form as a membrane protein (Musso et al., Blood 1999, Vol. 93, No 10 (May 15): pp 3531-3539), and recently it was noted that it may function either as ligand or as receptor (Budalgian et al., JBC 2004, vol 279, No 40: pp 42192-42201) inducing through this pathway secretion of pro-inflammatory cytokines. High expression level of the soluble protein has been associated to the pathogenesis of autoimmune and inflammatory diseases. IL-15 has been detected in several diseases including Crohn's disease (Kirman I., 1996, Am. J. Gastroenterol. 91, 1789), psoriasis (Ruckert R. 2000, 165: 2240-2250), leukemia (Yamada Y. 1999, Leukemia and Lymphoma, 35(1-2): 37-45) and rheumatoid arthritis (RA), (McInnes I.B. 1998, Immunology Today, 19, 75-79). Binding of the ligand to the T cell receptor induces expression of IL-15Rα and expression of several activation antigens such as CD69, CD25 and TNFRII. Furthermore, IL-15 is a chemoattractant for human blood T lymphocytes (Wilkinson 1995, J. Exp. Med. 181, 1255-1259). All these data suggest that IL-15 expressed by antigen presenting cells could be important on the early T cell activation at the inflammation site.

IL-15 is a member of the small four alpha-helix bundle family of cytokines. The IL-15 biological effects are mediated through its binding to a cell membrane receptor composed of three subunits α, β, and γ. The IL-15Rα is a subunit that is specific for this cytokine to whom it is bound with a very high affinity Kd $10^{-11}$, and may be found as a membrane receptor or in a soluble form (Budagian V. et al., JBC 2004, 279, 39: 40368-40375; Mortier et al., The Journal of Immunology, 2004, 173: 1681-1688). IL-15Ra contains a Sushi domain, which is capable of binding with IL-15, and is essential for biological functions of IL-15 after binding.

IL-15 has limitations related to low molecular weight, short in vivo half-life, hardly-controlled repeated dosage, and is likely to cause systemic immune side effect. There is an urgent need to find an approach which can increase the in vivo half-life, and promote or enhance the biological activity of IL-15 in vivo.

Recently, it was found that the complex formed by IL-15 and its receptor IL-15Rα can significantly enhance the biological activity of IL-15. Studies showed that the complex formed by IL-15 and the soluble receptor IL-15Rα is significantly superior to IL-15 alone in stimulating the proliferation of memory CD8+ T lymphocytes and NT/NKT cells. The IL-15/IL-15Rα complex is more than 10 times stronger than IL-15 alone in stimulating proliferation of memory CD8+ T cells and in maintaining their survival, and the mechanism may be associated with cis presentation.

International applications WO2007046006, WO2016095642 disclose a fusion protein intended for stimulating the IL-15Rbeta/gamma signaling pathway to thereby induce and/or stimulate the activation and/or proliferation of IL-15Rbeta/gamma-positive cells, such as NK and/or T cells, characterized in that it comprises an IL-15 indirectly linked by covalent bonds to a polypeptide which contains the sushi domain of the extracellular region of an IL-15Ralpha, and the use thereof in the treatment of cancer. However, WO2007046006 provides the experimental data and characteristics of the super-agonist only for IL-15 linked to IL-15Ralpha by a linker peptide through the N- and C-terminal amino acids, whereas WO2016095642 provides the experimental data and characteristics of the super-agonist only for that linked by a chimeric S-S bond formed by the Cys mutations in the IL-15 ligand and IL-15Ralpha themselves. The both do not provide any evidence for the activity and physicochemical characteristics of IL15 super-agonist variants comprising fusion protein domains linked by S-S bond(s) or without S-S bridges.

These objects have limitations related to low molecular weight, short in vivo half-life. They have a limited activity of interleukin 15, and the production thereof is very difficult, in particular, in Chinese hamster ovary cells, and associated with a low yield.

International applications WO 2015103928, WO2018071919 disclose variants to the solution of the above problems, in particular a heterodimeric IL-15 agonist protein comprising a protein (I) and a protein (II), wherein said protein (I) is formed by IL-15 fused with the first Fc variant, the first Fc variant being linked to the C-terminus of IL-15, and said protein (II) is the second Fc variant or is formed by IL-15Rα or a variant thereof fused with the second Fc variant, the second Fc variant being linked to the C-terminus of IL -15Rα. The protein (I) and the protein (II) form a stable heterodimeric protein through "knobs-into-holes" interactions between the first Fc variant and the second Fc variant.

The generation of IL-15-based immunocytokines is of particular interest for combining the advantageous properties of tumor-specific antibodies targeting tumors with the immunomodulatory effect of interleukin 15.

International applications WO2015018528, WO2018071918 disclose an immunocytokine comprising a conjugate and an immunomodulatory antibody or a fragment thereof, directly or indirectly linked by covalent bonds to said conjugate, wherein said conjugate comprises a polypeptide comprising the amino acid sequence of interleukin-15 or derivatives thereof, and an amino acid sequence of sushi domain of IL-15Rα or derivatives thereof.

International application WO 2012175222 discloses an immunocytokine for the treatment of cancer, which comprises a conjugate, and an antibody or a fragment thereof selected from the group comprising a Fab fragment, Fab' fragment, F(ab')2 fragment, Facb, Fd, and scFv, wherein said conjugate comprises a polypeptide comprising the amino acid sequence of the interleukin 15, and a polypeptide comprising the amino acid sequence of sushi domain of IL-15Rα. The conjugate and antibody or fragment thereof are covalently linked using bifunctional coupling agents that provide for protein conjugation.

International application WO2019006472 discloses a bifunctional heterodimeric protein comprising:

an IL-15/IL-15Ra fusion protein comprising an IL-15Ra protein, an IL-15 protein, and a first Fc domain; and

an antigen binding domain monomer which binds an antigen selected from the group consisting of human CD8, human NKG2A, and human NKG2D, comprising a heavy chain comprising a VH-CHl-hinge-CH2-CH3 monomer, wherein VH is a heavy chain variable domain and CH2- CH3 is a second Fc domain, and a light chain comprising a variable light chain (VL) and a light constant domain (CL),

wherein said first and said second Fc domains have a set of amino acid substitutions selected from the group consisting of:

S267K / L368D / K370S: S267K / S364K / E357Q;

S364K / E357Q: L368D / K370S;

L368D / K370S: S364K;

L368E / K370S: S364K;

T411T / K360E / Q362E: D401K;

L368D / K370S: S364K / E357L and

K370S:S364K / E357Q.

Despite the above prior art solutions for the complex of IL-15 and the receptor thereof IL-15Rα, there is still a need for an IL15-based molecule that will have high stability, prolonged in vivo half-life, increased in vivo biological activity and increased productivity in mammalian cells.

The novel immunocytokine format that was developed by the present inventors, which comprises IL-15 and IL-15Rα- based heterodimeric molecules, exhibits higher stability, increased productivity in mammalian cells, prolonged in vivo half-life and increased in vivo biological activity. Furthermore, they are a universal format for a bispecific immunocytokine having the property of IL15 activity. The novel immunocytokine format that was developed by the present inventors has a reduced toxicity according to the results of preclinical studies.

Brief description of invention

In one aspect, the present invention relates to an immunocytokine for stimulating the activation and/or proliferation of IL-15Rbeta/gamma-positive cells, which comprises an IL-15/IL-15Rα heterodimeric protein complex comprising:

1) IL-15Rα, which is linked to

a) an antibody light chain constant domain, or
b) antibody heavy chain constant domains comprising a first (CH1) heavy chain constant domain and an Fc fragment monomer comprising second (CH2) and third (CH3) heavy chain constant domains;

2) IL-15, which is linked to

a) antibody heavy chain constant domains comprising a first (CH1) heavy chain constant domain and an Fc fragment monomer comprising second (CH2) and third (CH3) heavy chain constant domains, or
b) an antibody light chain constant domain;

wherein the first antibody heavy chain constant domain and the antibody light chain constant domain within the heterodimeric complex have or do not have a covalent association through a natural S-S bridge, and
wherein, if the IL-15 or the IL-15Rα is linked to the antibody light chain constant domain, then the other portion, selected from IL-15Rα or IL-15, of the heterodimeric protein complex is linked to the antibody heavy chain constant domains.
In some embodiments, the immunocytokine comprises a first antibody heavy chain constant domain and an antibody light chain constant domain within a heterodimeric complex, which have a covalent association through a natural S-S bridge.
In some embodiments, the immunocytokine comprises a first antibody heavy chain constant domain and an antibody light chain constant domain within a heterodimeric complex, which do not have a covalent association through a natural S-S bridge.
In some embodiments, the immunocytokine comprises an antibody light chain constant domain selected from CK or CL.
In some embodiments, the immunocytokine comprises IL-15Rα having the amino acid sequence represented by SEQ ID NO:9, or any known mutant IL-15Rα variant with a similar biological activity.
In some embodiments, the immunocytokine comprises IL-15 having the amino acid sequence represented by SEQ ID NO:10, or any known mutant IL-15 variant with a similar biological activity.
In some embodiments, the immunocytokine comprises IL-15Rα that is linked to an antibody light chain constant domain.
In some embodiments, the immunocytokine comprises IL-15Rα linked to an antibody light chain constant domain that has the amino acid sequence represented by SEQ ID NO:1 or SEQ ID NO:19.
In some embodiments, the immunocytokine comprises IL-15 that is linked to an antibody light chain constant domain.
In some embodiments, the immunocytokine comprises IL-15 linked to an antibody light chain constant domain that has the amino acid sequence represented by SEQ ID NO:3 or SEQ ID NO:21.
In some embodiments, the immunocytokine comprises a first (CH1) heavy chain constant domain and an Fc fragment monomer comprising second (CH2) and third (CH3) heavy chain constant domains that are linked via a hinge.
In some embodiments, the immunocytokine comprises IL-15 or IL-15Rα that is linked to antibody heavy chain constant domains arranged in the following order: CH1-hinge-CH2-CH3.
In some embodiments, the immunocytokine comprises IL-15Rα that is linked to antibody heavy chain constant domains.
In some embodiments, the immunocytokine comprises IL-15Rα linked to antibody heavy chain constant domains that have the amino acid sequence represented by SEQ ID NO:4 or SEQ ID NO:22.
In some embodiments, the immunocytokine comprises IL-15 that is linked to antibody heavy chain constant domains.

In some embodiments, the immunocytokine comprises IL-15 linked to antibody heavy chain constant domains that have the amino acid sequence represented by SEQ ID NO:2 or SEQ ID NO:20.

In some embodiments, the immunocytokine comprises:

IL-15Rα linked to an antibody light chain constant domain that has the amino acid sequence represented by SEQ ID NO:1 or SEQ ID NO:19, and
IL-15 linked to antibody heavy chain constant domains that have the amino acid sequence represented by SEQ ID NO:2 or SEQ ID NO:20.

In some embodiments, the immunocytokine comprises:

IL-15Rα linked to antibody heavy chain constant domains that have the amino acid sequence represented by SEQ ID NO:4 or SEQ ID NO:22, and
IL-15 linked to an antibody light chain constant domain that has the amino acid sequence represented by SEQ ID NO:3 or SEQ ID NO:21.

In some embodiments, the immunocytokine comprises mutations in an Fc fragment monomer, which cause the deficiency of ADCC, CDC, and/or ADCP properties in said immunocytokine.

In some embodiments, the immunocytokine comprises an Fc fragment that belongs to IgG.

In some embodiments, the immunocytokine comprises an Fc fragment selected from the group comprising: human IgG1, IgG2, or IgG4.

In some embodiments, the immunocytokine comprises the above two IL-15/IL-15Rα heterodimeric protein complexes.

In some embodiments, the immunocytokine is used as a therapeutic agent for the treatment of cancer or an autoimmune disease.

In some embodiments, the immunocytokine is used as a therapeutic agent for the treatment of cancer.

In one aspect, the present invention relates to an immunocytokine for stimulating the activation and/or proliferation of IL-15Rbeta/gamma-positive cells, which comprises a heterodimeric protein complex based on IL-15/IL-15Rα and an immunomodulatory antibody or antigen-binding fragment thereof, which specifically inhibit the PD-1 pathway, wherein the IL-15/IL-15Rα heterodimeric protein complex comprises:

1) IL-15Rα, which is linked to

a) an antibody light chain constant domain, or
b) antibody heavy chain constant domains comprising a first (CH1) heavy chain constant domain and an Fc fragment monomer comprising second (CH2) and third (CH3) heavy chain constant domains;

2) IL-15, which is linked to

a) antibody heavy chain constant domains comprising a first (CH1) heavy chain constant domain and an Fc fragment monomer comprising second (CH2) and third (CH3) heavy chain constant domains, or
b) an antibody light chain constant domain;

wherein the first antibody heavy chain constant domain and the antibody light chain constant domain within the heterodimeric complex have or do not have a covalent association through a natural S-S bridge, and
wherein, if the IL-15 or the IL-15Rα is linked to the antibody light chain constant domain, then the other portion, selected from IL-15Rα or IL-15, of the heterodimeric protein complex is linked to the antibody heavy chain constant domains.

In some embodiments, the immunocytokine comprises a first antibody heavy chain constant domain and an antibody light chain constant domain within a heterodimeric complex, which have a covalent association through a natural S-S bridge.

In some embodiments, the immunocytokine comprises a first antibody heavy chain constant domain and an antibody light chain constant domain within a heterodimeric complex, which do not have a covalent association through a natural S-S bridge.

In some embodiments, the immunocytokine comprises an antibody light chain constant domain selected from CK or CL.

In some embodiments, the immunocytokine comprises IL-15Rα having the amino acid sequence represented by SEQ ID NO:9, or any known mutant IL-15Rα variant with a similar biological activity.

In some embodiments, the immunocytokine comprises IL-15 having the amino acid sequence represented by SEQ ID NO:10, or any known mutant IL-15 variant with a similar biological activity.

In some embodiments, the immunocytokine comprises IL-15Rα that is linked to an antibody light chain constant domain.

In some embodiments, the immunocytokine comprises IL-15Rα linked to an antibody light chain constant domain that has the amino acid sequence represented by SEQ ID NO:1 or SEQ ID NO:19.

In some embodiments, the immunocytokine comprises IL-15 that is linked to an antibody light chain constant domain.

In some embodiments, the immunocytokine comprises IL-15 linked to an antibody light chain constant domain that has the amino acid sequence represented by SEQ ID NO:3 or SEQ ID NO:21.

In some embodiments, the immunocytokine comprises a first (CH1) heavy chain constant domain and an Fc fragment monomer comprising second (CH2) and third (CH3) heavy chain constant domains that are linked via a hinge.

In some embodiments, the immunocytokine comprises IL-15 or IL-15Rα that is linked to antibody heavy chain constant domains arranged in the following order: CH1-hinge-CH2-CH3.

In some embodiments, the immunocytokine comprises IL-15Rα that is linked to antibody heavy chain constant domains.

In some embodiments, the immunocytokine comprises IL-15Rα linked to antibody heavy chain constant domains that have the amino acid sequence represented by SEQ ID NO:6 or SEQ ID NO:24.

In some embodiments, the immunocytokine comprises IL-15 that is linked to antibody heavy chain constant domains.

In some embodiments, the immunocytokine comprises IL-15 linked to antibody heavy chain constant domains that have the amino acid sequence represented by SEQ ID NO:5 or SEQ ID NO:23.

In some embodiments, the immunocytokine comprises an immunomodulatory antibody or an antigen binding fragment thereof, which specifically inhibit the PD-1 pathway, and is an antibody that specifically binds to PD-1.

In some embodiments, the immunocytokine comprises an immunomodulatory antibody comprising:

a) a light chain comprising a light chain variable domain and a light chain constant domain;
b) a heavy chain comprising a heavy chain variable domain and antibody heavy chain constant domains comprising a first (CH1) heavy chain constant domain and an Fc fragment monomer comprising second (CH2) and third (CH3) heavy chain constant domains.

In some embodiments, the immunocytokine comprises a light chain variable domain comprising LCDRs 1, 2, and 3 (hypervariable regions 1, 2, and 3), which are represented by the amino acid sequences of SEQ ID NO:14, SEQ ID NO:15, and SEQ ID NO:16, respectively.

In some embodiments, the immunocytokine comprises a light chain variable domain comprising the amino acid sequence represented by SEQ ID NO:18.

In some embodiments, the immunocytokine comprises a heavy chain variable domain comprising HCDRs 1, 2, and 3 (hypervariable regions 1, 2, and 3), which are represented by the amino acid sequences of SEQ ID NO:11, SEQ ID NO:12, and SEQ ID NO:13, respectively.

In some embodiments, the immunocytokine comprises a heavy chain variable domain comprising the amino acid sequence represented by SEQ ID NO:17.

In some embodiments, the immunocytokine comprises:

1) a light chain variable domain comprising LCDRs 1, 2, and 3 (hypervariable regions 1, 2, and 3), which are represented by the amino acid sequences of SEQ ID NO:14, SEQ ID NO:15, and SEQ ID NO:16, respectively.

2) a heavy chain variable domain comprising HCDRs 1, 2, and 3 (hypervariable regions 1, 2, and 3), which are represented by the amino acid sequences of SEQ ID NO:11, SEQ ID NO:12, and SEQ ID NO:13, respectively.

In some embodiments, the immunocytokine comprises:

1) a light chain variable domain comprising the amino acid sequence represented by SEQ ID NO:18.

2) a heavy chain variable domain comprising the amino acid sequence represented by SEQ ID NO:17.

In some embodiments, the immunocytokine comprises an immunomodulatory antibody that comprises a light chain comprising the amino acid sequence represented by SEQ ID NO:8.

In some embodiments, the immunocytokine comprises an immunomodulatory antibody that comprises a heavy chain comprising the amino acid sequence represented by SEQ ID NO:7.

In some embodiments, the immunocytokine comprises an immunomodulatory antibody comprising:

a heavy chain comprising the amino acid sequence represented by SEQ ID NO:7;

a light chain comprising the amino acid sequence represented by SEQ ID NO:8.

In some embodiments, the immunocytokine comprises an immunomodulatory antibody or an antigen binding fragment thereof, which specifically inhibit the PD-1 pathway, which is an antibody that specifically binds to PD-L1.
In some embodiments, the immunocytokine comprises:

a) a heterodimeric protein complex based on IL-15/IL-15Rα comprising:

IL-15Rα linked to an antibody light chain constant domain that has the amino acid sequence represented by SEQ ID NO:1 or SEQ ID NO:19, and
IL-15 linked to antibody heavy chain constant domains that have the amino acid sequence represented by SEQ ID NO:5 or SEQ ID NO:23;

b) an immunomodulatory antibody comprising:

a heavy chain comprising the amino acid sequence represented by SEQ ID NO:7;

a light chain comprising the amino acid sequence represented by SEQ ID NO:8.

In some embodiments, the immunocytokine comprises:

a) IL-15Rα linked to antibody heavy chain constant domains that have the amino acid sequence represented by SEQ ID NO:6 or SEQ ID NO:24, and
IL-15 linked to an antibody light chain constant domain that has the amino acid sequence represented by SEQ ID NO:3 or SEQ ID NO:21;
b) an immunomodulatory antibody comprising:

a heavy chain comprising the amino acid sequence represented by SEQ ID NO:7;

a light chain comprising the amino acid sequence represented by SEQ ID NO:8.

In some embodiments, the immunocytokine has mutations in antibody constant domains, which induce the heterodimerization of two different portions, one of which comprises covalently or non-covalently associated IL-15Rα linked to antibody constant domains and IL-15 linked to an antibody constant domain, and the other portion comprises covalently or non-covalently associated light and heavy chains of antibody.
In some embodiments, the immunocytokine comprises:
a first Fc monomer and a second Fc monomer, which are selected from the following group: the first Fc monomer is a Knob modified Fc, and the second Fc monomer is a Hole modified Fc, or when the second Fc monomer is a Knob modified Fc, and the first Fc monomer is a Hole modified Fc.
In some embodiments, the immunocytokine comprises a first Fc monomer that has the amino acid substitutions S354C/T366W, and a second Fc monomer that has the amino acid substitutions Y349C/T366S/L368A/Y407V.
In some embodiments, the immunocytokine comprises a first Fc monomer that has the amino acid substitutions Y349C/T366S/L368A/Y407, and a second Fc monomer that has the amino acid substitutions S354C/T366W.
In some embodiments, the immunocytokine comprises an Fc fragment that belongs to IgG.
In some embodiments, the immunocytokine comprises an Fc fragment, wherein the Fc fragment isotype is selected from the group comprising: human IgG1, IgG2, or IgG4.
In some embodiments, the immunocytokine comprises mutations in the Fc fragment monomer, which cause the deficiency of ADCC, CDC, and/or ADCP properties in said immunocytokine.
In some embodiments, the immunocytokine is used for the treatment of an oncological or autoimmune disease.
In some embodiments, the immunocytokine is used for the treatment of an oncological disease.
In one aspect, the present invention relates to an isolated nucleic acid that encodes any of the above immunocytokines.
In some embodiments, the nucleic acid is DNA.
In one aspect, the present invention relates to an expression vector comprising the above nucleic acid.

In one aspect, the present invention relates to a method for production of a host cell for production of the above immunocytokine, which comprises transformation of the cell with the above vector.

In one aspect, the present invention relates to a host cell for production of the above immunocytokine, which comprises the above nucleic acid.

In one aspect, the present invention relates to a method for production of a drug comprising the above immunocytokine, which comprises culturing of the above host cell in a culture medium under conditions sufficient to produce said immunocytokine, if necessary, followed by isolation and purification of the resulting immunocytokine.

In one aspect, the present invention relates to a pharmaceutical composition for stimulating the activation and/or proliferation of IL-15Rbeta/gamma-positive cells, which comprises the above immunocytokine in a therapeutically effective amount, in combination with one or more pharmaceutically acceptable excipients.

In some embodiments, the pharmaceutical composition is used for the treatment of an oncological or autoimmune disease.

In some embodiments, the pharmaceutical composition is used for the treatment of an oncological disease.

In some embodiments, the pharmaceutical composition is used for the treatment of an oncological disease that is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

In one aspect, the present invention relates to a method for treatment of an oncological disease, which comprises administering to a subject in need of such treatment said immunocytokine or said pharmaceutical composition, in a therapeutically effective amount.

In some embodiments of the method for treatment, the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

In one aspect, the present invention relates to a method for activating the biological activity of a T cell population or NK cell population in a subject in need of such activation, which comprises administering to the subject an effective amount of the above immunocytokine or the above pharmaceutical composition.

In one aspect, the present invention relates to the use of said immunocytokine or said pharmaceutical composition for the treatment in a subject in need of such treatment of an oncological disease.

In some uses, the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

Brief description of drawings

Fig. 1. The immunocytokine format comprising an IL-15/IL-15Rα heterodimeric protein complex (CK_IL15Ra_Hc_IL15CH1FcLALA).
Fig. 2. The immunocytokine format comprising an IL-15/IL-15Rα heterodimeric protein complex (CK_IL15_Hc_IL15RaCH1FcLALA).

Fig. 3. The immunocytokine format comprising an IL-15/IL-15Rα heterodimeric protein complex and immunomodulatory antibody that specifically inhibits the PD-1 pathway (Fab-CK_IL15_Hc_IL15RaCH1FcknobLALA).

"Knob" is understood to mean mutations forming a "knob-into-hole: structure between the first Fc variant and the second Fc variant in the CH3 domain.

Fig. 4. The immunocytokine format comprising an IL-15/IL-15Rα heterodimeric protein complex and immunomodulatory antibody that specifically inhibits the PD-1 pathway (Fab-CK_IL15Ra_Hc_IL15CHlFcknobLALA).

"Knob" is understood to mean mutations forming a "knob-into-hole: structure between the first Fc variant and the second Fc variant in the CH3 domain.

Fig. 5. pEE-IL15Ra-CK vector.

AmpR is a beta-lactamase gene that provides resistance to ampicillin,
pUC origin is a pUC replication origin in bacteria, CMV-Promoter is the promoter of cytomegalovirus early genes,
leader is a leader sequence,
IL15Ra is a gene encoding interleukin 15 receptor subunit alpha,
CK is a gene encoding a light chain constant domain,
Poly A is a polyadenylation signal sequence, for increasing mRNA stability,
OriP is a replication origin in bacteria, or
a high-copy number ColE1/pMB1/pBR322/pUC origin of replication in bacterial cells.

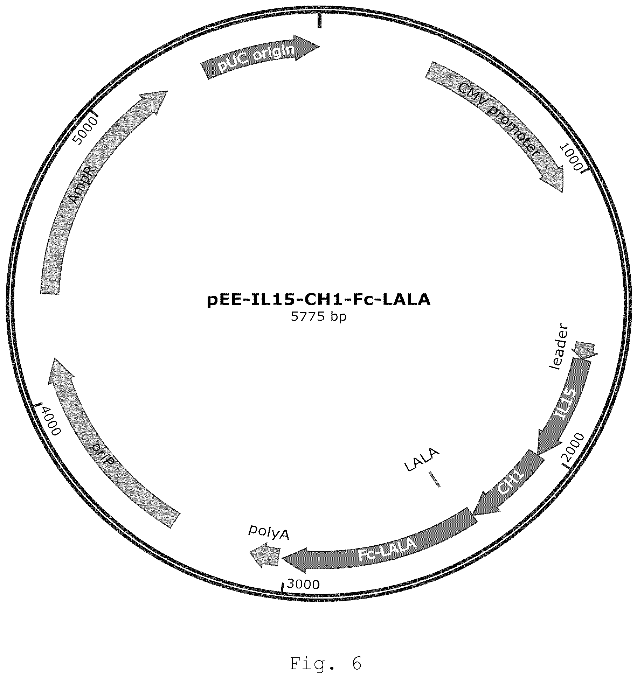

Fig. 6. pEE-IL15-CH1-Fc-LALA vector.

AmpR is a beta-lactamase gene that provides resistance to ampicillin,
pUC origin is a pUC replication origin in bacteria, CMV-Promoter is the promoter of cytomegalovirus early genes,
leader is a leader sequence,
IL15 is a gene encoding interleukin 15,
CH1 is a gene encoding a first heavy chain constant domain,
Fc-LALA is a gene encoding an Fc fragment carrying LALA mutations (L234A and L235A mutations in the CH2 constant domain of the Fc fragment),
Poly A is a polyadenylation signal sequence, for increasing mRNA stability,
OriP is a replication origin in bacteria, or
a high-copy number ColE1/pMB1/pBR322/pUC origin of replication in bacterial cells.

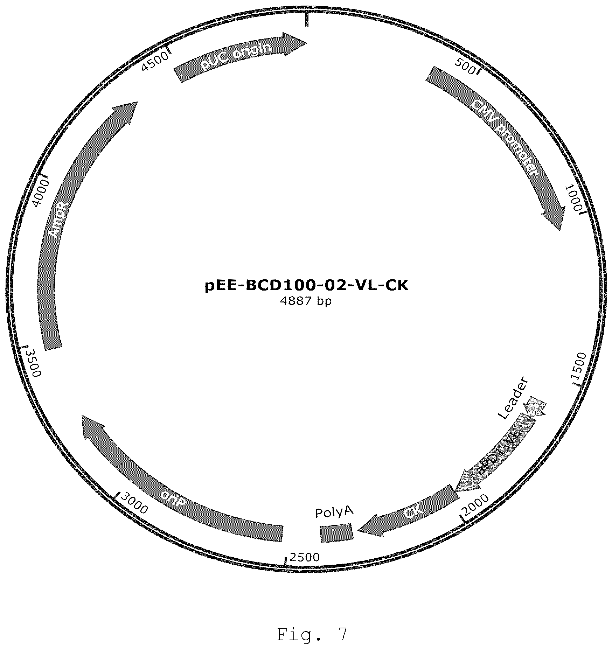

Fig. 7. pEE-BCD100-02-VL-CK vector.

AmpR is a beta-lactamase gene that provides resistance to ampicillin,
pUC origin is a pUC replication origin in bacteria, CMV-Promoter is the promoter of cytomegalovirus early genes,
leader is a leader sequence,
aPD1-VL is a gene encoding the light chain of antibody that specifically binds to PD1,
CK is a gene encoding a light chain constant domain,
Poly A is a polyadenylation signal sequence, for increasing mRNA stability,
OriP is a replication origin in bacteria, or
a high-copy number ColE1/pMB1/pBR322/pUC origin of replication in bacterial cells.

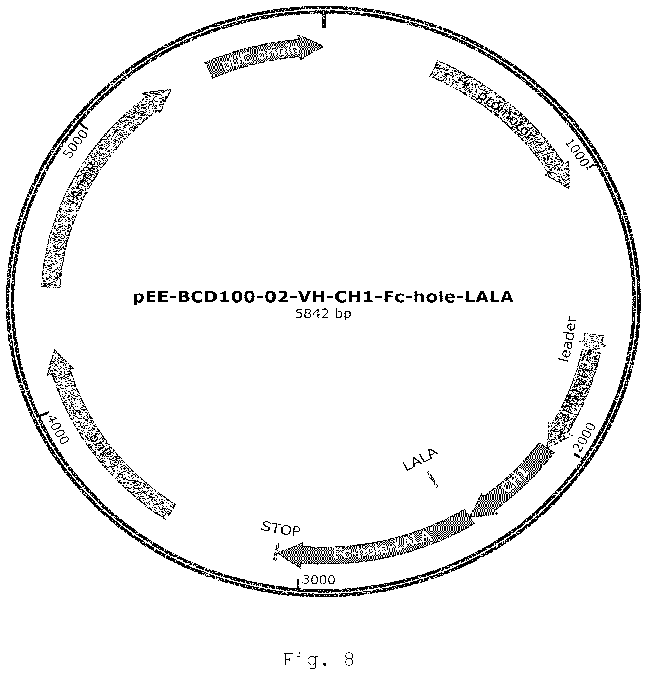

Fig. 8. pEE-BCD100-02-VH-CH1-Fc-hole-LALA vector.

AmpR is a beta-lactamase gene that provides resistance to ampicillin,
pUC origin is a pUC replication origin in bacteria,
promoter is the promoter of cytomegalovirus early genes,
leader is a leader sequence,
aPD1-VH is a gene encoding the variable domain of the heavy chain of antibody that specifically binds to PD1,
CH1 is a gene encoding a first heavy chain constant domain,
Fc-hole-LALA is a gene encoding an Fc fragment carrying mutations to form a "hole" and LALA mutations (L234A and L235A mutations in the CH2 constant domain of the Fc fragment),
STOP is a stop codon,
OriP is a replication origin in bacteria, or
a high-copy number ColE1/pMB1/pBR322/pUC origin of replication in bacterial cells.

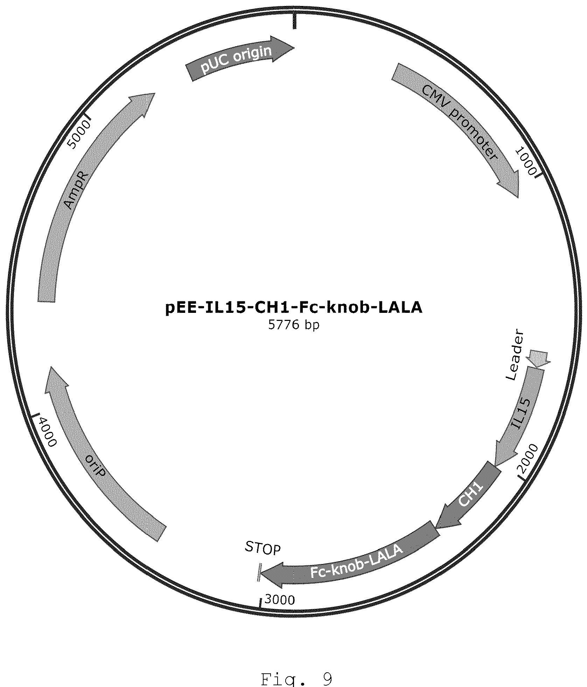

Fig. 9. pEE-IL15-CH1-Fc-knob-LALA vector.

AmpR is a beta-lactamase gene that provides resistance to ampicillin,
pUC origin is a pUC replication origin in bacteria,
CMV-Promoter is the promoter of cytomegalovirus early genes,
leader is a leader sequence,
IL15 is a gene encoding interleukin 15,
CH1 is a gene encoding a first heavy chain constant domain,
Fc-knob-LALA is a gene encoding an Fc fragment carrying mutations to form a "knob" and LALA mutations
(L234A and L235A mutations in the CH2 constant domain of the Fc fragment),
STOP is a stop codon,
OriP is a replication origin in bacteria, or
a high-copy number ColE1/pMB1/pBR322/pUC origin of replication in bacterial cells.

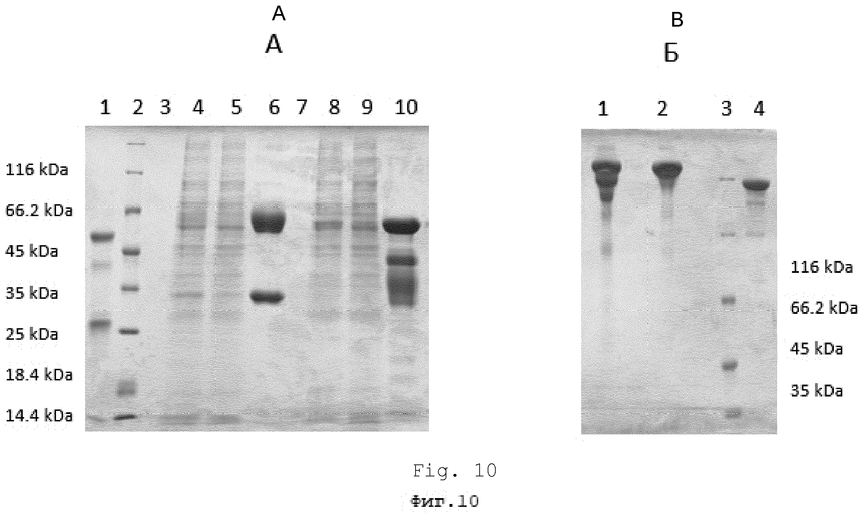

Fig. 10 A. SDS gel electrophoresis with betamercaptoethanol

1.Control antibody 5μl
2.Fermentas unstained marker
3. -
4.Growth medium with CK_IL15Ra_Hc_IL15CH1FcLALA prior to purification 10 μl
5.Growth medium with CK_IL15Ra_Hc_IL15CH1FcLALA following purification 10 μl
6.Purified CK_IL15Ra_Hc_IL15CH1FcLALA 10 μl
7. -
8.Growth medium with CK_IL15_Hc_IL15RaCH1FcLALA prior to purification 10 μl
9.Growth medium with CK_IL15_Hc_IL15RaCH1FcLALA following purification 10 μl
10.Purified CK_IL15_Hc_IL15RaCH1FcLALA 10 μl

Fig. 10 B. SDS gel electrophoresis without betamercaptoethanol

1.Purified CK_IL15Ra_Hc_IL15CH1FcLALA 10 μl
2.Purified CK_IL15_Hc_IL15RaCH1FcLALA 10 μl
3.Fermentas unstained marker
4. Control antibody 5ul

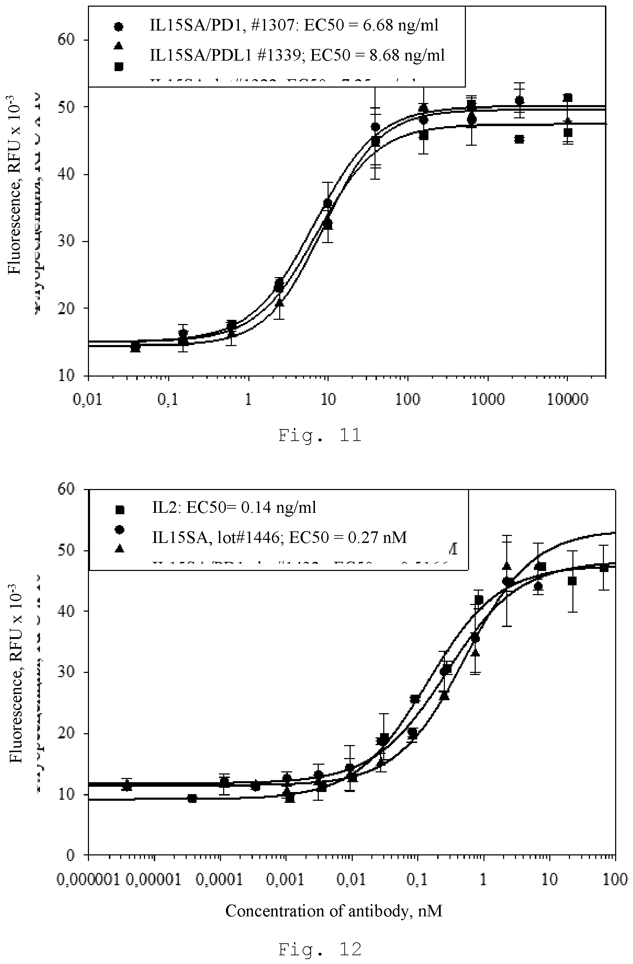

Fig. 11. Measurement of proliferation activity. The NK-92 cell line was used in the assay. The assay was conducted in a 96-well culture plate. The suspension comprised the NK-92 cells, the test antibody at a concentration as indicated in the graph. All suspension components were prepared in RPMI-1640 medium supplemented with fetal bovine serum and glutamine. After all components were added, the plates were incubated at 37°C, 5% CO2. Alamar Blue was then added to the wells. Following incubation, the fluorescence intensity in the wells was measured.

Fig. 12. Measurement of proliferation activity. The assay used isolated natural killer cells isolated from PBMCs of healthy donors by negative selection. The assay was conducted in a 96-well culture plate. The suspension comprised natural killer cells, a test antibody at a concentration as indicated in the graph. All suspension components were prepared in a culture medium supplemented with autologous human plasma. After all components were added, the plates were incubated at 37°C, 5% CO2. Alamar Blue was then added to the wells. Following incubation, the fluorescence intensity in the wells was measured.

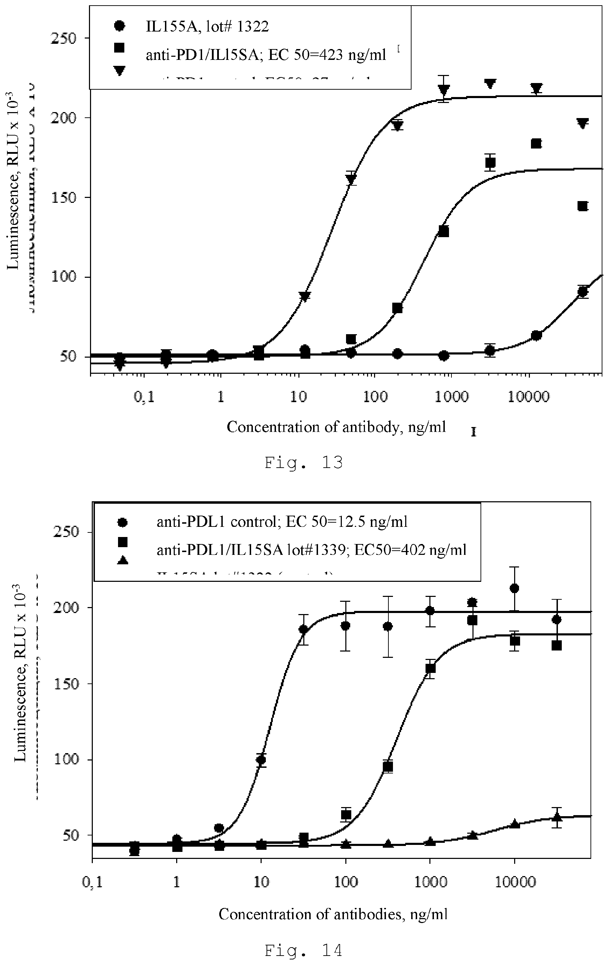

Fig. 13. Measurement of aPD1 specific activity For the assay, we used the Jurkat NFAT-FLuc PD-1 cell line generated based on the Jurkat cell line and stably expressing PD-1 on the surface thereof and containing the firefly luciferase encoding gene under control of NFAT promoter; and the Raji PDL1 cell line generated based on the Raji cell line and stably expressing PDL1 on the surface thereof. The assay was conducted in a 96-well culture plate. The suspension in each well contained Jurkat NFAT-FLuc PD-1 cells, Raji PDL1 cells, aCD3/aTAA1 cells at a concentration of 1 ng/ml, and a test antibody at a concentration as indicated in the graph. After all components were added, the plates were incubated at 37°C, 5% CO2, and the luciferase intensity in the wells was then measured using a luminescence assay kit.

Fig. 14. Determination of specific activity of anti-PDL1/IL15SA antibodies on a reporter cell line.

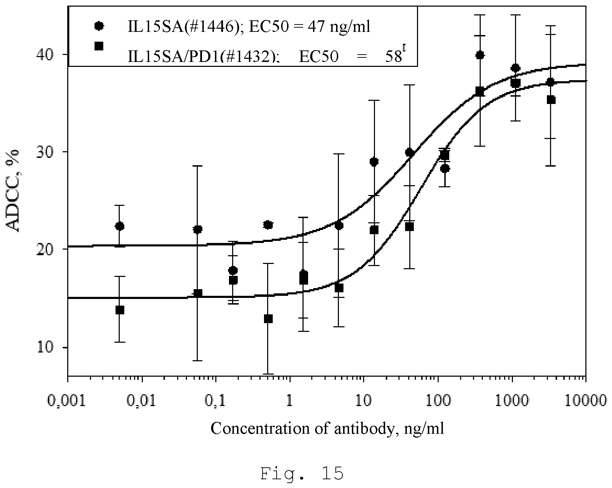

Fig. 15. Determination of effect of natural killer cells on ADCC. The assay used natural killer cells isolated from PBMCs of healthy donors by negative selection. The assay was conducted in a 96-well culture plate. The suspension contained natural killer cells and Raji cells, the effector antibody Rituximab, and a test antibody at a concentration as indicated in the graph. All suspension components were prepared in a culture medium supplemented with fetal bovine serum and glutamine. After all components were added, the plates were incubated at 37°C, 5% CO2. The culture liquid was then collected, and lactate dehydrogenase content was measured using an LDH assay kit.

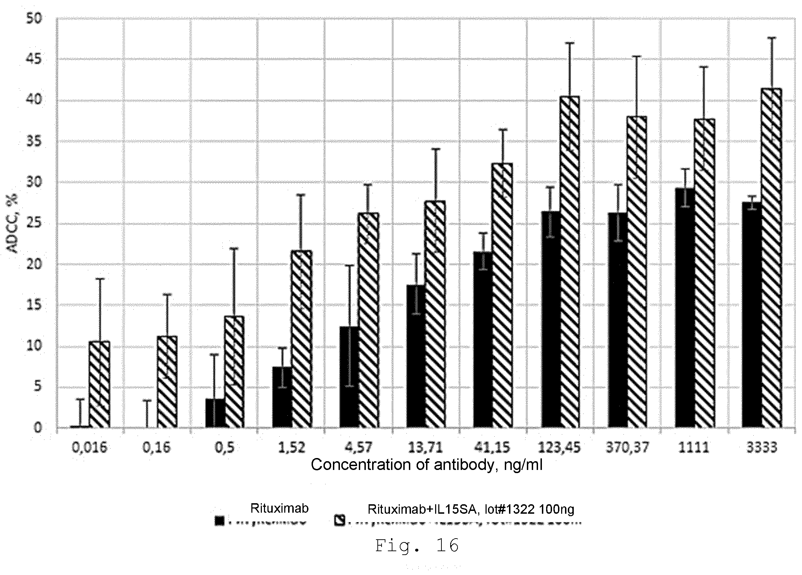

Fig. 16. Results of determination of specific activity of immunocytokines relative to enhancing ADCC ability of rituximab against the TAA-reporter line.

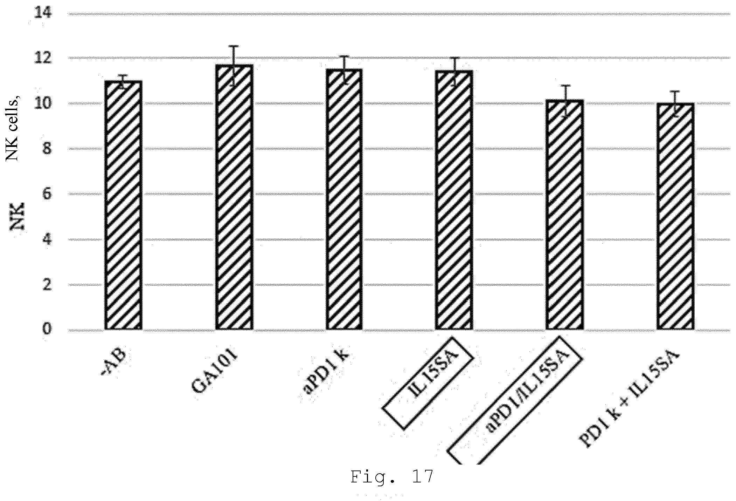

Fig. 17. Study of cytotoxicity for variants of immunocytokine comprising an IL-15/IL-15Rα (IL15SA) heterodimeric protein complex, on NK cells.

The results demonstrate no significant cytotoxic effect of the candidates, i.e. no more than 10% decrease in the number of immunoreactive cells as compared to the AB negative control (absence of any exogenous antibodies).

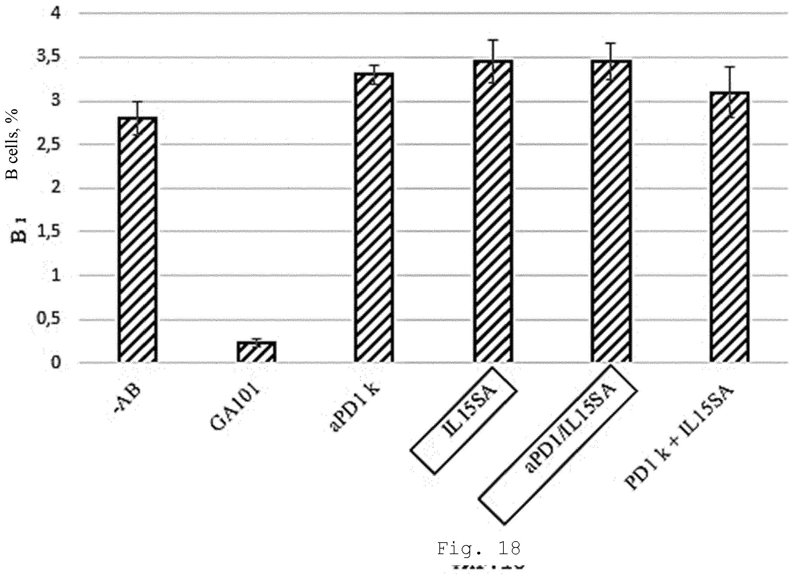

Fig. 18. Study of cytotoxicity for IL15SA variants on B cells.

The results demonstrate no significant cytotoxic effect of the candidates, i.e. no more than 10% decrease in the number of immunoreactive cells as compared to the AB negative control (absence of any exogenous antibodies).

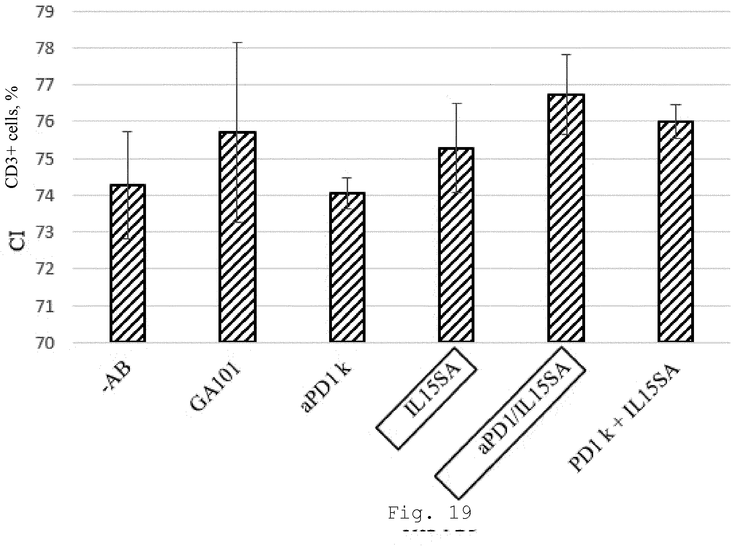

Fig. 19. Study of cytotoxicity for IL15SA variants on CD3+ cells.

The results demonstrate no significant cytotoxic effect of the candidates, i.e. no more than 10% decrease in the number of immunoreactive cells as compared to the AB negative control (absence of any exogenous antibodies).

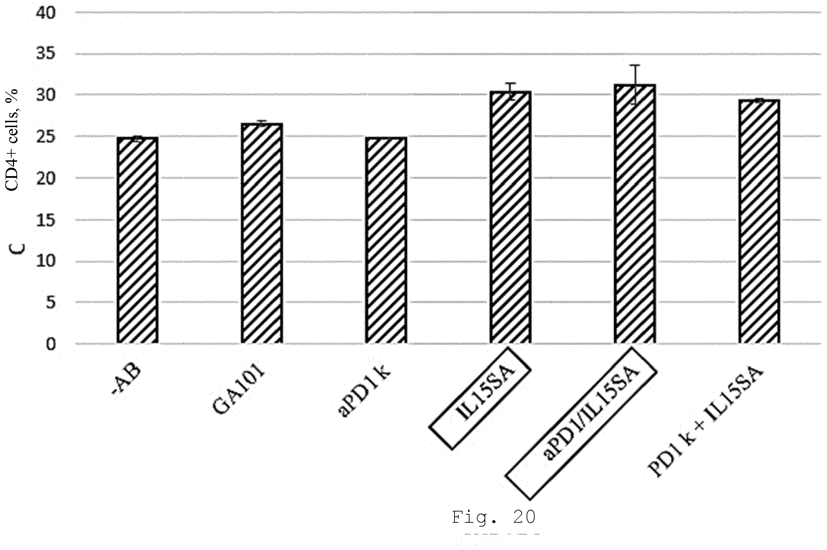

Fig. 20. Study of cytotoxicity for IL15SA variants on CD4+ cells.

The results demonstrate no significant cytotoxic effect of the candidates, i.e. no more than 10% decrease in the number of immunoreactive cells as compared to the AB negative control (absence of any exogenous antibodies).

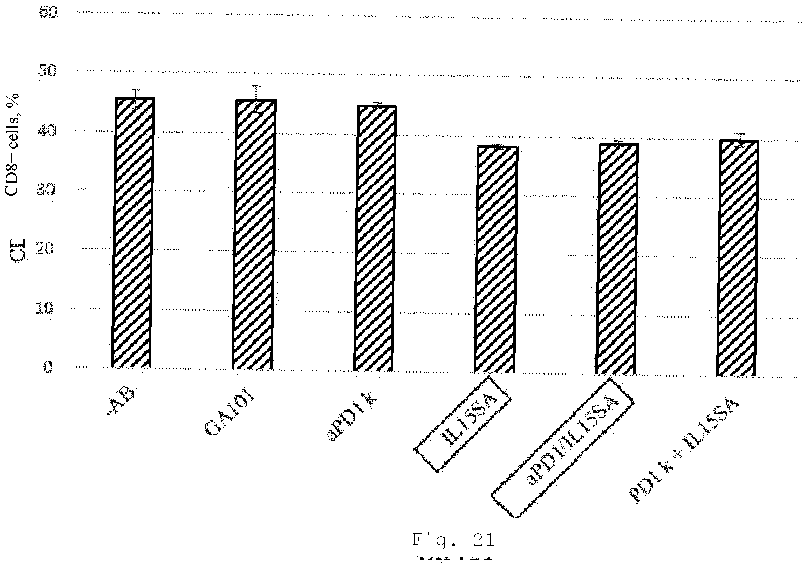

Fig. 21. Study of cytotoxicity for IL15SA variants on CD8+ cells.

The results demonstrate no significant cytotoxic effect of the candidates, i.e. no more than 10% decrease in the number of immunoreactive cells as compared to the AB negative control (absence of any exogenous antibodies).

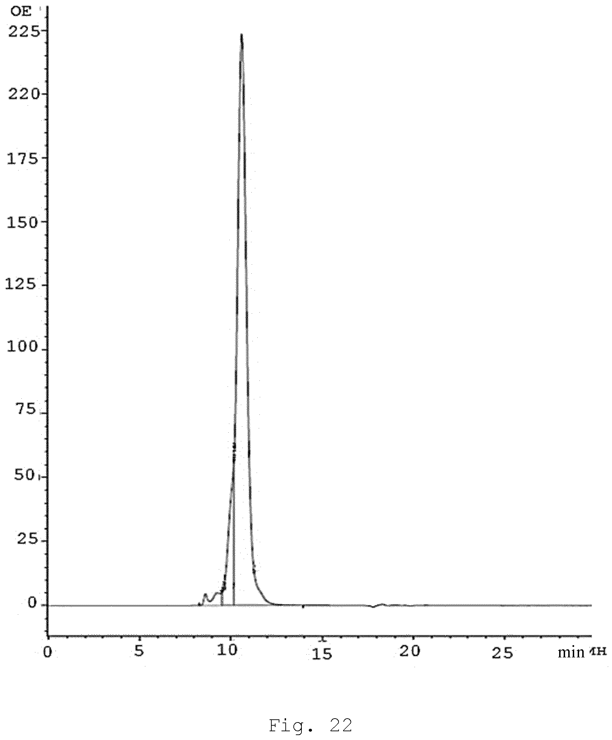

Fig. 22. Chromatogram. This chromogram demonstrates high homogeneity (the aggregate content in solution was no more than 5%) of immunocytokine comprising a heterodimeric protein complex based on IL-15/IL-15Rα (IL15 super-agonist).

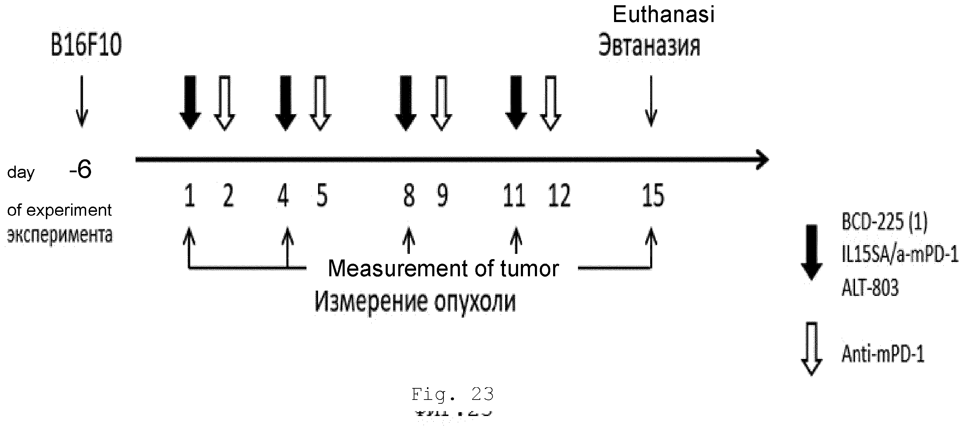

Fig. 23. Scheme of experiment for determination of antitumor activity of immunocytokine products, which immunocytokine comprises a heterodimeric protein complex based on IL-15/IL-15Rα (BCD-225) in a murine in vivo tumor model.

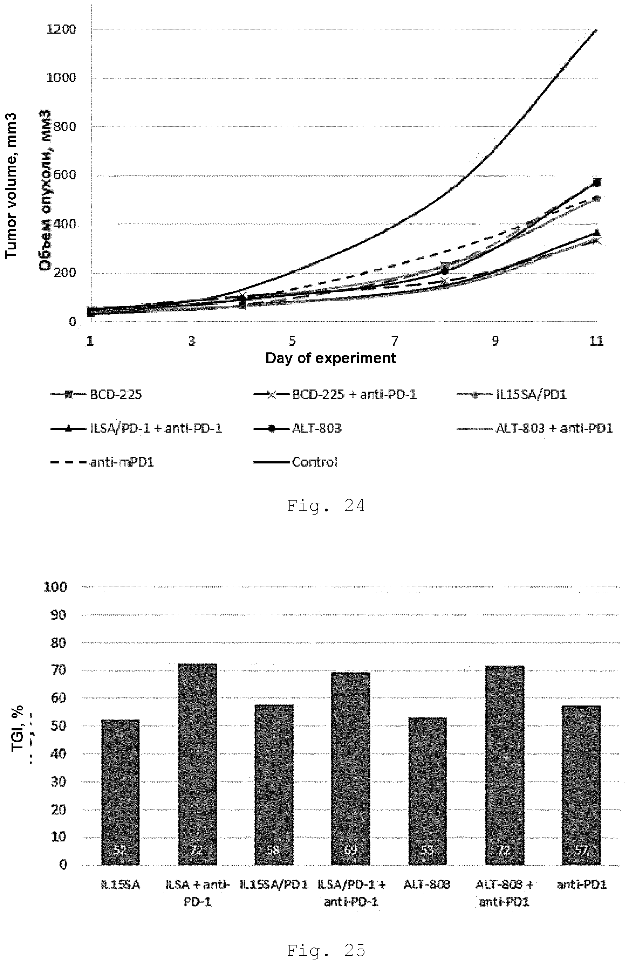

Fig. 24. Results of experiment for determination of antitumor activity of immunocytokine product variants, which immunocytokine comprises a heterodimeric protein complex based on IL-15/IL-15Rα (BCD-225) (graph) in a murine in vivo tumor model.

Fig. 25. Results of experiment for determination of antitumor activity of immunocytokine product variants, which immunocytokine comprises a heterodimeric protein complex based on IL-15/IL-15Rα (BCD-225) (diagram) in a murine in vivo tumor model.

Description of invention

General definitions and general methods

Unless defined otherwise, all technical and scientific terms used herein have the same meaning as is commonly understood by one of ordinary skill in the art.

Further, unless otherwise required by context, singular terms shall include pluralities and plural terms shall include the singular. Typically, the classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known and widely used by those skilled in the art. Enzyme reactions and purification methods are performed according to the manufacturer's guidelines, as is common in the art, or as described herein.

Unless specified otherwise, the term "biologically active" and "biological activity" and "biological characteristics" with respect to a polypeptide of the invention means having the ability to bind to a biological molecule.

The term "recombinant protein" means a protein (polypeptide) that is expressed in a cell or cell line comprising nucleotide sequence(s) encoding said protein, wherein said nucleotide sequence(s) is not naturally associated with the cell.

The term "bispecific antibody" refers to an antibody having an antigen-binding domain(s) that are capable of specific binding to two distinct epitopes on a single biological molecule or capable of specific binding to epitopes on two distinct biological molecules. A bispecific antibody is also referred to herein as having "dual specificity" or as being a "dual specificity" antibody.

The term "monoclonal antibody" or "mAb" refers to an antibody that is synthesized and isolated by a separate clonal population of cells. The clonal population can be a clonal population of immortalized cells. In some embodiments, the immortalized cells in a clonal population are hybrid cells - hybridomas - typically produced by the fusion of individual B lymphocytes from immunized animals with individual cells from a lymphocytic tumour. Hybridomas are a type of constructed cells and do not exist in nature.

The term "Ka" as used herein refers to the association (on) rate of a particular antibody-antigen interaction.

The term "Kd" as used herein refers to the dissociation (off) rate of a particular antibody-antigen interaction.

"Binding affinity" generally refers to the strength of the sum total of noncovalent interactions between a single binding site of a molecule (e.g. an antibody) and its binding partner (e.g. an antigen). Unless specified otherwise, "binding affinity" refers to intrinsic (characteristic, true) binding affinity which reflects a 1:1 interaction between members of a binding pair (e.g. antibody and antigen). The affinity of a molecule X for its binding partner Y can generally be represented by the dissociation constant (Kd). The preferred Kd value is about 200 nM, 150 nM, 100 nM, 60 nM, 50 nM, 40 nM, 30 nM, 20 nM, 10 nM, 8 nM, 6 nM, 4 nM, 2 nM, 1 nM, or less. Affinity can be measured by common methods known in the art, including those described herein. A variety of methods of measuring binding affinity are known in the art, any of which can be used for the purposes of the present invention.

The term "peptide linker" as used herein is intended to mean any peptide having the ability to combine domains, with a length which depends on the domains which it binds to each other, and comprising any amino acid sequence. Preferably, the peptide linker has a length of more than 5 amino acids and consists of any set of amino acids selected from G, A, S, P, E, T, D, K.

The term "in vitro" refers to a biological entity, a biological process, or a biological reaction outside the body under artificial conditions. For example, a cell grown in vitro is to be understood as a cell grown in an environment outside the body, e.g. in a test tube, a culture vial, or a microtiter plate.

As used herein, the words "comprise," "have," "include," or variations such as "comprises," "comprising," "has," "having," "includes" or "including", and all grammatical variations thereof will be understood to imply the inclusion of a stated integer or group of integers but not the exclusion of any other integer or group of integers.

Detailed description of the invention

Immunocytokine

In one aspect, the present invention relates to an immunocytokine for stimulating the activation and/or proliferation of IL-15Rbeta/gamma-positive cells, which comprises a heterodimeric protein complex based on IL-15/IL-15Rα comprising:

> 1) IL-15Rα, which is linked to
>
> > a) an antibody light chain constant domain, or
> > b) antibody heavy chain constant domains comprising a first (CH1) heavy chain constant domain and an Fc fragment monomer comprising second (CH2) and third (CH3) heavy chain constant domains;
>
> 2) IL-15, which is linked to
>
> > a) antibody heavy chain constant domains comprising a first (CH1) heavy chain constant domain and an Fc fragment monomer comprising second (CH2) and third (CH3) heavy chain constant domains, or
> > b) an antibody light chain constant domain;

wherein the first antibody heavy chain constant domain and the antibody light chain constant domain within the heterodimeric complex have or do not have a covalent association through a natural S-S bridge, and

wherein, if the IL-15 or the IL-15Rα is linked to the antibody light chain constant domain, then the other portion, selected from IL-15Rα or IL-15, of the heterodimeric protein complex is linked to the antibody heavy chain constant domains.

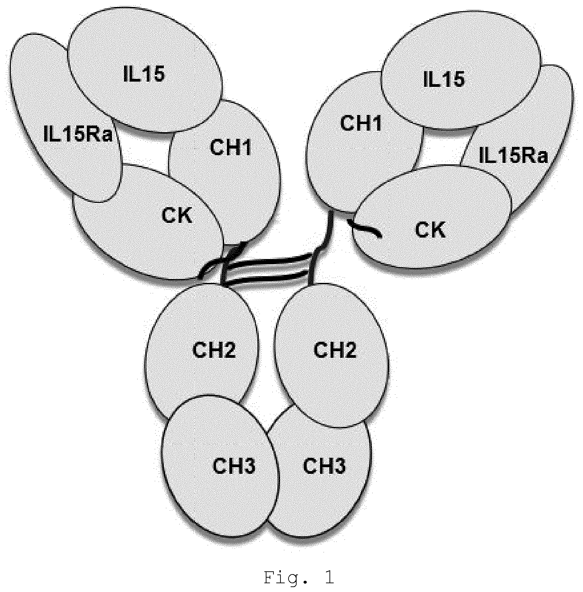

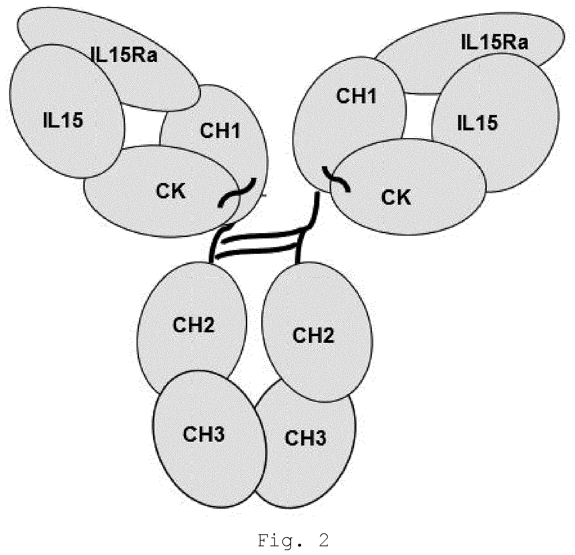

The format of the above immunocytokine is shown in Figures 1 and 2.

The term "immunocytokine" means a molecule comprising an antibody or fragments thereof directly or indirectly linked by covalent bonds to a cytokine or derivatives thereof. Said antibody and said cytokine can be linked by a linker peptide.

An immunocytokine of the invention is contemplated to refer to an isolated immunocytokine.

The term "isolated" used to describe various immunocytokines of this description means an immunocytokine which has been identified and separated and/or regenerated from a cell or cell culture, in which the immunocytokine is expressed. Impurities (contaminant components) from its natural environment are materials which would interfere with diagnostic or therapeutic uses of the polypeptide, and may include enzymes, hormones, and other proteinaceous or nonproteinaceous solutes. In preferred embodiments, the immunocytokine is purified (1) to a degree sufficient to obtain at least 15 residues of N-terminal or internal amino acid sequence by use of a spinning cup sequenator (Edman sequenator), or (2) to homogeneity by SDS-PAGE under nonreducing or reducing conditions using Coomassie Brilliant Blue, or preferably silver stain. The isolated immunocytokine includes the immunocytokine in situ within recombinant cells since at least one component of the polypeptide's natural environment will not be present. The isolated polypeptide is typically prepared by at least one purification step.

As used herein, "heterodimeric protein complex" refers to a protein formed from the combination of IL-15 and IL-15Rα.

As used herein, "IL-15" or "IL-15 peptide" or "interleukin-15" can be any IL-15 (interleukin-15) or a mutant thereof, such as human or non-human mammalian IL-15 or non-mammalian IL-15. Exemplary non-human mammals include mammals such as pigs, rabbits, monkeys, chimpanzees, mice, and the like; and non-mammals include chickens and the like. Preferably, human interleukin-15 mature molecule is found in the database UniProtKB, Accession Number P40933, 49-162aa. The term "IL-15 variant" refers to a variant molecule with increased or decreased affinity to the receptor thereof, or increased or decreased activity in stimulating T cells or NK cells, due to one or more amino acid substitutions, additions or deletions.

"IL-15Rα" means interleukin 15 receptor subunit alpha (α), and, according to the present invention, can be any IL-15Rα compound or a functional fragment thereof, such as human IL-15Rα or non-human mammalian IL-15Rα or non-mammalian IL-15Rα. Exemplary non-human mammals include mammals such as pigs, rabbits, monkeys, chimpanzees, mice, and the like; and non-mammals include chickens and the like. The preferred IL-15Rα molecule is found in the database UniProtKB, accession number Q13261 (https://www.uniprot.org/uniprot/Q13261).

The fragment crystallizable region ("Fc region, Fc") of an immunoglobulin is the "tail" region of an immunoglobulin molecule that interacts with cell surface Fc-receptor, as well as some proteins of the complement system. This property allows antibodies to activate the immune system. In IgG, IgA and IgD isotypes, the Fc region is composed of two identical protein fragments, respectively, from the second and third constant domains of the two heavy chains; in IgM and IgE isotypes, the Fc region contains three heavy chain constant domains (CH2, CH3, and CH4 domains) in each polypeptide chain.

"Fc fragment monomer" is understood to mean an Fc region from the second and third constant domains of either one of the two heavy chains (for IgG, IgA and IgD isotypes); for IgM and IgE isotypes, the Fc monomer comprises three constant domains of one of the two heavy chains (CH2, CH3 and CH4 domains).

In some embodiments, the immunocytokine comprises a first antibody heavy chain constant domain and an antibody light chain constant domain within a heterodimeric complex, which have a covalent association through a natural S-S bridge.

In some embodiments, the immunocytokine comprises a first antibody heavy chain constant domain and an antibody light chain constant domain within a heterodimeric complex, which do not have a covalent association through a natural S-S bridge.

In some embodiments, the immunocytokine comprises an antibody light chain constant domain selected from CK or CL.

In mammals, known are only two types of light chain denoted by lambda (λ) and kappa (κ). The constant domain of the lambda light chain is designated CL, and that of the kappa light chain is designated CK.

In some embodiments, the immunocytokine comprises IL-15Rα having the amino acid sequence represented by DTCPPPMSVEHADIWVKSYSLYSRERYICNSGFKRKAGTSSLTECVLNKATNVAHWTTPSLK CIRDPALVHQRPAPPSTVTTAGVTPQPESLSPSGKEPAASS (SEQ ID NO:9), or any known variant of IL-15Rα containing mutations with similar biological activity.

Mutant IL-15Rα, IL-15Rα with a similar biological activity are understood to refer to any IL-15Rα known in the art that comprises mutations and has a similar biological activity as compared to that of the "wild-type" IL-15Rα.

In some embodiments, the immunocytokine comprises IL-15 having the amino acid sequence represented by DNWVNVISDLKKIEDLIQSMHIDATLYTESDVHPSCKVTAMKCFLLELQVISLESGDASIHD TVENLIILANDSLSSNGNVTESGCKECEELEEKNIKEFLQSFVHIVQMFINTS (SEQ ID NO:10), or any known variant of IL-15 containing mutations with similar biological activity.

Mutant IL-15, IL-15 with a similar biological activity mean any IL-15 known in the art that comprises mutations and has a similar biological activity as compared to the "wild-type" IL-15.

In some embodiments, the immunocytokine comprises IL-15Rα that is linked to an antibody light chain constant domain.

In some embodiments, the immunocytokine comprises IL-15Rα linked to an antibody light chain constant domain that has the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEC (SEQ ID NO:1).
```

In some embodiments, the immunocytokine comprises IL-15Rα linked to an antibody light chain constant domain that has the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEA (SEQ ID NO:19).
```

The amino acid sequence represented by SEQ ID NO:19 is modified from the amino acid sequence represented by SEQ ID NO:1 by a single amino acid substitution of Cysteine (C) to Alanine (A) at position 216. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises IL-15 that is linked to an antibody light chain constant domain.

In some embodiments, the immunocytokine comprises IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO:3).
```

In some embodiments, the immunocytokine comprises IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEA (SEQ ID NO:21).
```

The amino acid sequence represented by SEQ ID NO:21 is modified from the amino acid sequence represented by SEQ ID NO:3 by a single amino acid substitution of Cysteine (C) to Alanine (A) at position 233. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises a first (CH1) heavy chain constant domain and an Fc fragment monomer comprising second (CH2) and third (CH3) heavy chain constant domains that are linked via a hinge.

In some embodiments, the immunocytokine comprises IL-15 or IL-15Rα that is linked to antibody heavy chain constant domains arranged in the following order: CH1-hinge-CH2-CH3.

In some embodiments, the immunocytokine comprises IL-15Rα that is linked to antibody heavy chain constant domains.

In some embodiments, the immunocytokine comprises IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTNQVSLTCLVKGFYPSDIAVEW
ESNGQPENNYKTTPPVLDSDGSFFLYSKLTVDKSRWQQGNVFSCSVMHEALHNHYTQKSLSL
SP (SEQ ID NO:4).
```

In some embodiments, the immunocytokine comprises IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSADKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTNQVSLTCLVKGFYPSDIAVEW
ESNGQPENNYKTTPPVLDSDGSFFLYSKLTVDKSRWQQGNVFSCSVMHEALHNHYTQKSLSL
SP (SEQ ID NO:22).
```

The amino acid sequence represented by SEQ ID NO:22 is modified from the amino acid sequence represented by SEQ ID NO:4 by a single amino acid substitution of Cysteine (C) to Alanine (A) at position 212. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises IL-15 that is linked to antibody heavy chain constant domains.

In some embodiments, the immunocytokine comprises IL-15 linked to antibody heavy chain constant domains that have the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSP (SEQ ID NO:2).
```

In some embodiments, the immunocytokine comprises IL-15 linked to antibody heavy chain constant domains that have the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSADKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSP (SEQ ID NO:20).
```

The amino acid sequence represented by SEQ ID NO:20 is modified from the amino acid sequence represented by SEQ ID NO:2 by a single amino acid substitution of Cysteine (C) to Alanine (A) at position 229. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises:

IL-15Rα linked to an antibody light chain constant domain that has the amino acid sequence represented by DTCPPPMSVEHADIWVKSYSLYSRERYICNSGFKRKAGTSSLTECVLNKATNVAHWTTPSLKCIRDPALVHQRPA

PPSTVTTAGVTPQPESLSPSGKEPAASSGAEGGGRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEC (SEQ ID NO:1) or
DTCPPPMSVEHADIWVKSYSLYSRERYICNSGFKRKAGTSSLTECVLNKATNVAHWTTPSLKCIRDPALVHQRPA PPSTVTTAGVTPQPESLSPSGKEPAASSGAEGGGRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEA (SEQ ID NO:19), and

IL-15 linked to antibody heavy chain constant domains that have the amino acid sequence represented by

DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSP                    (SEQ            ID            NO:2)


    or


DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSADKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSP (SEQ ID NO:20).


In some embodiments, the immunocytokine comprises:

    IL-15Rα linked to an antibody light chain constant domain that has the amino acid sequence represented by

DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEC (SEQ ID NO:1),


    and
    IL-15 linked to antibody heavy chain constant domains that have the amino acid sequence represented by

DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSP (SEQ ID NO:2).


In some embodiments, the immunocytokine comprises:

IL-15Rα linked to an antibody light chain constant domain that has the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEA (SEQ ID NO:19),
```

and
IL-15 linked to antibody heavy chain constant domains that have the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSADKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSP (SEQ ID NO:20).
```

In some embodiments, the immunocytokine comprises:

IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTNQVSLTCLVKGFYPSDIAVEW
ESNGQPENNYKTTPPVLDSDGSFFLYSKLTVDKSRWQQGNVFSCSVMHEALHNHYTQKSLSL
SP                    (SEQ            ID             NO:4)
```

or

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSADKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTNQVSLTCLVKGFYPSDIAVEW
ESNGQPENNYKTTPPVLDSDGSFFLYSKLTVDKSRWQQGNVFSCSVMHEALHNHYTQKSLSL
SP (SEQ ID NO:22),
```

and
IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO:3)
```

or

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEA (SEQ ID NO:21).
```

In some embodiments, the immunocytokine comprises:

IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTNQVSLTCLVKGFYPSDIAVEW
ESNGQPENNYKTTPPVLDSDGSFFLYSKLTVDKSRWQQGNVFSCSVMHEALHNHYTQKSLSL
SP (SEQ ID NO:4), a
```

and
IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO:3).
```

In some embodiments, the immunocytokine comprises:

IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
```

```
KSTSGGTAALGCLVKDYFPEPVTVSWNSGALTSGVHTFPAVLQSSGLYSLSSVVTVPSSSLG
TQTYICNVNHKPSNTKVDKRVEPKSADKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTNQVSLTCLVKGFYPSDIAVEW
ESNGQPENNYKTTPPVLDSDGSFFLYSKLTVDKSRWQQGNVFSCSVMHEALHNHYTQKSLSL
SP (SEQ ID NO:22),
```

and
IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEA (SEQ ID NO:21).
```

In some embodiments, the immunocytokine comprises mutations in an Fc fragment monomer, which cause the deficiency of ADCC, CDC, and/or ADCP properties in said immunocytokine.

Mutations in Fc fragment are understood to mean modification(s) of the amino acid sequences of antibodies described herein. Variants of the amino acid sequence of antibody are produced by introducing appropriate nucleotide changes into the antibody nucleic acid, or by peptide synthesis. Such modifications include, for example, deletions, and/or insertions and/or substitutions of residues within the amino acid sequences of antibody. Any combination of deletion, insertion, and substitution is made to arrive at the final construct, provided that the final construct possesses the desired characteristics.

A variant of modification of the amino acid sequences of antibodies using amino acid substitutions is the substitution of at least one amino acid residue in the antibody molecule with another residue.

Conservative substitutions are shown in Table A under "preferred substitutions".

| Table A | | |
|---|---|---|
| Original residue | Exemplary substitutions | Preferred substitutions |
| Ala (A) | Val; Leu; Ile | Val |
| Arg (R) | Lys; Gin; Asn | Lys |
| Asn (N) | Gin; His; Asp, Lys; Arg | Gin |
| Asp (D) | Glu; Asn | Glu |
| Cys (C) | Ser; Ala | Ser |
| Gln (Q) | Asn; Glu | Asn |
| Glu (E) | Asp; Gin | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gin; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

The term "effector function" of antibody refers to biological activities attributable to the Fc-region (native Fc-region sequence or Fc-region amino acid variants) of antibody, which vary with the antibody isotype. Examples of antibody effector functions include: $Cl_q$ binding and complement dependent cytotoxicity; Fc receptor binding; antibody-dependent cell-mediated cytotoxicity (ADCC); phagocytosis; down regulation of cell surface receptors (e.g. B-cell receptor, BCR), and B-cell activation.

"Antibody-dependent cellular cytotoxicity" or "ADCC" refers to a cell-mediated response, in which nonspecific cytotoxic cells that express Fc receptors (FcR) (for example, natural killer (NK) cells, neutrophils, and macrophages) recognize bound antibody on a target cell and subsequently cause lysis or phagocytosis of the target cell. The primary cells for mediating ADCC, NK cells, express FcγRJII only, whereas monocytes express FcγRI, FcγRII and FcγRIII. FcR expression on hematopoietic cells is summarized in Table 3 on page 464 of Ravetch and Kinet, Annu. Rev. Immunol 9: 457-92 (1991). To assess ADCC activity of a molecule of interest, an in vitro ADCC assay, such as that described in U.S. Patent Nos. 5,500,362 or 5,821,337 may be performed. Useful effector cells for such assays include peripheral blood mononuclear cells (PBMCs) and Natural Killer (NK) cells. Alternatively, or additionally, ADCC activity of the molecule of interest may be assessed in vivo, e.g. in an animal model such as that disclosed in Clynes et al. PNAS (USA) 95: 652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMCs), natural killer (NK) cells, monocytes, cytotoxic T cells and neutrophils; with PBMCs and NK cells being preferred. The effector cells may be isolated from a native source thereof, e.g. from blood or PBMCs as described in the present publication.

"Complement dependent cytotoxicity" and "CDC" refer to the ability of a molecule to lyse a target in the presence of complement. The complement activation pathway is initiated by the binding of the first component of the complement system (C1q) to a molecule {e.g. an antibody) complexed with a cognate antigen. To assess complement activation, a CDC assay may be performed, e.g. as described in Gazzano-Santoro et al., J. Immunol. Methods 202: 163 (1996).

In some embodiments, the immunocytokine comprises an Fc fragment that belongs to IgG.

In some embodiments, the immunocytokine comprises an Fc fragment selected from the group comprising: human IgG1, IgG2, or IgG4.

In some embodiments, the immunocytokine comprises the above two heterodimeric protein complexes based on IL-15/IL-15Rα.

IL15 super-agonist (also referred to as BCD225 or IL15SA or (IL15SA)2-Fc) of the present invention is understood to refer to CK_IL15Ra_Hc_IL15CH1FcLALA and CK_IL15_Hc_IL15RaCH1FcLALA.

The meanings of the designations CK, IL15Ra, IL15, CH1FcLALA are given throughout the text of the application.

CK_IL15Ra_Hc_IL15CH1FcLALA is the above immunocytokine comprising two heterodimeric protein complexes based on IL-15/IL-15Rα, which comprise IL15Ra linked to the antibody light chain constant domain CK, and IL15 linked to antibody heavy chain constant domains comprising a first (CH1) heavy chain constant domain and an Fc fragment monomer comprising second (CH2) and third (CH3) heavy chain constant domains, wherein the Fc comprises LALA mutations.

CK_IL15_Hc_IL15RaCH1FcLALA is the above immunocytokine comprising two heterodimeric protein complexes based on IL-15/IL-15Rα, which comprise IL15 linked to the antibody light chain constant domain CK, and IL15Ra linked to antibody heavy chain constant domains comprising a first (CH1) heavy chain constant domain and an Fc fragment monomer comprising second (CH2) and third (CH3) heavy chain constant domains, wherein the Fc comprises LALA mutations.

The above immunocytokines can be made both in the format, where the first antibody heavy chain constant domain and the antibody light chain constant domain within the heterodimeric complex have a covalent association through a natural S-S bridge, and in the format, where the first antibody heavy chain constant domain and the antibody light chain constant domain within the heterodimeric complex do not have a covalent association through the natural S-S bridge; in a particular case, to this end, the both cysteines are replaced by alanine.

Experimental studies of the above immunocytokines both in the format, where the first antibody heavy chain constant domain and the antibody light chain constant domain within the heterodimeric complex have a covalent association through a natural S-S bridge, and in the format, where the first antibody heavy chain constant domain and the antibody light chain constant domain within the heterodimeric complex do not have a covalent association through a natural S-S bridge, demonstrated no significant difference in the activity of the above immunocytokines.

The format of the above immunocytokine is shown in Figures 1 and 2.

In some embodiments, the immunocytokine is used as a therapeutic agent for the treatment of cancer or an autoimmune disease.

In some embodiments, the immunocytokine is used as a therapeutic agent for the treatment of cancer.

In one aspect, the present invention relates to an immunocytokine for stimulating the activation and/or proliferation of IL-15Rbeta/gamma-positive cells, which comprises a heterodimeric protein complex based on IL-15/IL-15Rα, and an immunomodulatory antibody or antigen-binding fragment thereof, which specifically inhibit the PD-1 pathway, wherein the heterodimeric protein complex based on IL-15/IL-15Rα comprises:

1) IL-15Rα, which is linked to

a) an antibody light chain constant domain, or
b) antibody heavy chain constant domains comprising a first (CH1) heavy chain constant domain and an Fc fragment monomer comprising second (CH2) and third (CH3) heavy chain constant domains;

2) IL-15, which is linked to

a) antibody heavy chain constant domains comprising a first (CH1) heavy chain constant domain and an Fc fragment monomer comprising second (CH2) and third (CH3) heavy chain constant domains, or
b) an antibody light chain constant domain;

wherein the first antibody heavy chain constant domain and the antibody light chain constant domain within the heterodimeric complex have or do not have a covalent association through a natural S-S bridge, and

wherein, if the IL-15 or the IL-15Rα is linked to the antibody light chain constant domain, then the other portion, selected from IL-15Rα or IL-15, of the heterodimeric protein complex is linked to the antibody heavy chain constant domains.

The term "antibody" or "immunoglobulin" (Ig) as used herein includes whole antibodies and any antigen binding fragment (i.e., "antigen-binding portion") or single chains thereof. The term "antibody" refers to a glycoprotein comprising at least two heavy (H) chains and two light (L) chains interconnected by disulfide bonds, or an antigen-binding portion thereof. Each heavy chain comprises a heavy chain variable region (abbreviated referred to herein as VH) and a heavy chain constant region. Known are five types of mammalian Ig heavy chain denoted by Greek letters: α, δ, ε, γ and μ. The type of a heavy chain present defines the class of an antibody; these chains are found in IgA, IgD, IgE, IgG, and IgM antibodies, respectively. Distinct heavy chains differ in size and composition; α and γ contain approximately 450 amino acids, while μ and ε have approximately 550 amino acids. Each heavy chain has two regions, the constant region and the variable region. The constant region is identical in all antibodies of the same isotype, but differs in antibodies of different isotypes. Heavy chains γ, α and δ have a constant region composed of three constant domains CH1, CH2 and CH3 (in a line), and a hinge region for added flexibility (Woof J., Burton D., Nat Rev Immunol 4, 2004, cc.89-99); heavy chains μ and ε have a constant region composed of four constant domains CH1, CH2, CH3 and CH4. In mammals, known are only two types of light chain denoted by lambda (λ) and kappa (κ). Each light chain consists of a light chain variable region (abbreviated referred to herein as VL) and light chain constant region. The approximate length of a light chain is 211 to 217 amino acids. Preferably, the light chain is a kappa (κ) light chain, and the constant domain CL is preferably C kappa (κ).

"Antibody" according to the invention can be of any class (e.g. IgA, IgD, IgE, IgG, and IgM, preferably IgG), or subclass (e.g. IgG1, IgG2, IgG3, IgG4, IgA1 and IgA2, preferably IgG1).

VL and VH regions can be further subdivided into hyper-variability regions called complementarity determining regions (CDRs), interspersed between regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g. effector cells) and the first component (Clq) of the classical complement system.

The term "antigen-binding portion" of an antibody or "antigen-binding fragment" (or simply "antibody portion" or "antibody fragment"), as used herein, refers to one or more fragments of an antibody that retain the ability to specifically bind to an antigen. It has been shown that the antigen-binding function of an antibody can be performed by fragments of a full length antibody. Examples of binding fragments which are included within the term "antigen-binding portion" of an antibody include (i) Fab-fragment monovalent fragment consisting of the VL, VH, CL and CH 1 domains; (ii) F(ab') 2 fragment, a bivalent fragment comprising two Fab-fragments linked by a disulfide bridge at the hinge region; (iii) Fd-fragment consisting of the VH and CH1 domains; (iv) Fv-fragment consisting of the VL and VH domains of a single arm of an antibody; (v) dAb-fragment (Ward et al., (1989) Nature 341:544-546), which consists of a VH/VHH domain; and (vi) extracted complementarity determining region (CDR). In addition, two regions of the Fv-fragment, VL and VH, are encoded by separate genes, they can be joined using recombinant methods using a synthetic linker that enables them to receive a single protein chain in which the VL and VH region are paired to form monovalent molecules (known as single chain Fv (scFv); see e.g. Bird et al. (1988) Science 242:423-426; and Huston et al. (1988) Proc. Natl. Acad. Sci. USA 85:5879-5883). It is assumed that such single-stranded molecules are also included within the term "antigen-binding portion" of an antibody. These antibody fragments are obtained using conventional techniques known to those skilled in the art, and the fragments are screened in the same manner as are intact antibodies.

Preferably, the CDR of antigen-binding portion, or the whole antigen binding portion of antibodies of the invention is derived from mouse, lama or human donor library or substantially of human origin with certain amino acid residues altered, e.g. substituted with different amino acid residues so as to optimize the specific properties of antibody, e.g. KD, koff, IC50, EC50, ED50. Preferably, the framework regions of antibody of the invention are of human origin or substantially of human origin (at least 80, 85, 90, 95, 96, 97, 98 or 99% of human origin).

In other embodiments, the antigen binding portion of antibody of the invention may be derived from other non-human species including mouse, lama, rabbit, rat or hamster, but not limited thereto. Alternatively, the antigen binding region can be derived from the human species.

The term "variable" refers to the fact that certain portions of the variable domains greatly differ in sequence among antibodies. The V domain mediates antigen binding and determines specificity of each particular antibody for its particular antigen. However, the variability is not evenly distributed across the 110-amino acid span of the variable domains. Instead, the V regions consist of invariant fragments called framework regions (FRs) of 15-30 amino acids separated by shorter regions of extreme variability called "hypervariable regions" or CDR. The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by the FRs and, with the hypervariable regions from the other chain, contribute to the formation of the antigen-binding site of antibodies. The constant domains are not involved directly in binding an antibody to an antigen, but exhibit various effector functions, such as participation of the antibody in antibody-dependent cellular cytotoxicity (ADCC).

The term "hypervariable region" as used in the present description refers to the amino acid residues of an antibody which are responsible for antigen binding. The hypervariable region generally comprises amino acid residues from a "complementarity determining region" or "CDR" and/or those residues from a "hypervariable loop".

In certain cases, it may also be desirable to alter one or more CDR amino acid residues so as to improve binding affinity to the target epitope. This is known as "affinity maturation" and may optionally be performed in connection with humanization, for example in situations where humanization of an antibody leads to reduced binding specificity or affinity and it is not possible to sufficiently improve the binding specificity or affinity by back mutations alone. Various affinity maturation methods are known in the art, for example the in vitro scanning saturation mutagenesis method described by Burks et al., Proc Natl Acad Sci USA, 94:412-417 (1997) and the stepwise in vitro affinity maturation method by Wu et al., Proc Natl Acad Sci USA 95:6037 6042 (1998).

"Framework regions" (FR) are those variable domain residues other than the CDR residues. Each variable domain typically has four FRs identified as FR1, FR2, FR3 and FR4. If the CDRs are defined according to Kabat, the light chain FR residues are positioned about at residues 1-23 (LCFR1), 35-49 (LCFR2), 57-88 (LCFR3), and 98-107 (LCFR4) and the heavy chain FR residues are positioned about at residues 1-30 (HCFR1), 36-49 (HCFR2), 66-94 (HCFR3), and 103-113 (HCFR4) in the heavy chain. If the CDRs comprise amino acid residues from hypervariable loops, the light chain FR residues are positioned about at residues 1-25 (LCFR1), 33-49 (LCFR2), 53-90 (LCFR3), and 97-107 (LCFR4) in the light chain and the heavy chain FR residues are positioned about at residues 1-25 (HCFR1), 33-52 (HCFR2), 56-95 (HCFR3), and 102-113 (HCFR4) in the heavy chain residues. In some instances, when the CDR comprises amino acids from both a CDR as defined by Kabat and those of a hypervariable loop, the FR residues will be adjusted accordingly. For example, when CDRH1 includes amino acids H26-H35, the heavy chain FR1 residues are at positions 1-25 and the FR2 residues are at positions 36-49.

An antibody of the present invention "which binds" a target antigen refers to an antibody capable of binding the antigen with sufficient affinity such that the antibody can be used as a diagnostic and/or therapeutic agent targeting a protein or cell or tissue expressing said antigen, and slightly cross-reacts with other proteins. According to analytical methods: fluorescence-activated cell sorting (FACS), radioimmunoassay (RIA) or ELISA, in such embodiments, the degree of antibody binding to a non-target protein is less than 10 % of antibody binding to a specific target protein. With regard to the binding of an antibody to a target molecule, the term "specific binding" or "specifically binds to" or "is specific for" a particular polypeptide or an epitope on a particular polypeptide target means binding that is measurably different from a non-specific interaction.

In some embodiments, the immunocytokine comprises a first antibody heavy chain constant domain and an antibody light chain constant domain within a heterodimeric complex, which have a covalent association through a natural S-S bridge.

In some embodiments, the immunocytokine comprises a first antibody heavy chain constant domain and an antibody light chain constant domain within a heterodimeric complex, which do not have a covalent association through a natural S-S bridge.

In some embodiments, the immunocytokine comprises an antibody light chain constant domain selected from CK or CL.

In some embodiments, the immunocytokine comprises IL-15Rα having the amino acid sequence represented by DTCPPPMSVEHADIWVKSYSLYSRERYICNSGFKRKAGTSSLTECVLNKATNVAHWTTPSLK CIRDPALVHQRPAPPSTVTTAGVTPQPESLSPSGKEPAASS (SEQ ID NO:9), or any known variant of IL-15Rα containing mutations with similar biological activity.

Mutant IL-15Rα, IL-15Rα with a similar biological activity are understood to refer to any IL-15Rα known in the art that comprises mutations and has a similar biological activity as compared to that of the "wild-type" IL-15Rα.

In some embodiments, the immunocytokine comprises IL-15 having the amino acid sequence represented by DNWVNVISDLKKIEDLIQSMHIDATLYTESDVHPSCKVTAMKCFLLELQVISLESGDASIHD TVENLIILANDSLSSNGNVTESGCKECEELEEKNIKEFLQSFVHIVQMFINTS (SEQ ID NO:10), or any known variant of IL-15 containing mutations with similar biological activity.

Mutant IL-15, IL-15 with a similar biological activity mean any IL-15 known in the art that comprises mutations and has a similar biological activity as compared to the "wild-type" IL-15.

In some embodiments, the immunocytokine comprises IL-15Rα that is linked to an antibody light chain constant domain.

In some embodiments, the immunocytokine comprises IL-15Rα linked to an antibody light chain constant domain that has the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEC (SEQ ID NO:1).
```

In some embodiments, the immunocytokine comprises IL-15Rα linked to an antibody light chain constant domain that has the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEA (SEQ ID NO:19).
```

The amino acid sequence represented by SEQ ID NO:19 is modified from the amino acid sequence represented by SEQ ID NO:1 by a single amino acid substitution of Cysteine (C) to Alanine (A) at position 216. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises IL-15 that is linked to an antibody light chain constant domain.

In some embodiments, the immunocytokine comprises IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO:3).
```

In some embodiments, the immunocytokine comprises IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEA (SEQ ID NO:21).
```

The amino acid sequence represented by SEQ ID NO:21 is modified from the amino acid sequence represented by SEQ ID NO:3 by a single amino acid substitution of Cysteine (C) to Alanine (A) at position 233. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises a first (CH1) heavy chain constant domain and an Fc fragment monomer comprising second (CH2) and third (CH3) heavy chain constant domains that are linked via a hinge.

In some embodiments, the immunocytokine comprises IL-15 or IL-15Rα that is linked to antibody heavy chain constant domains arranged in the following order: CH1-hinge-CH2-CH3.

In some embodiments, the immunocytokine comprises IL-15Rα that is linked to antibody heavy chain constant domains.

In some embodiments, the immunocytokine comprises IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHYTQKSLS
LSP (SEQ ID NO:6).
```

In some embodiments, the immunocytokine comprises IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSADKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE

YKCKVSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHYTQKSLS
LSP (SEQ ID NO:24).
```

The amino acid sequence represented by SEQ ID NO:24 is modified from the amino acid sequence represented by SEQ ID NO:6 by a single amino acid substitution of Cysteine (C) to Alanine (A) at position 212. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises IL-15 that is linked to antibody heavy chain constant domains.

In some embodiments, the immunocytokine comprises IL-15 linked to antibody heavy chain constant domains that have the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPCRDELTKNQV
SLWCLVKGFYPSDIAVEWESNGQPENNYKTTPPVLDSDGSFFLYSKLTVDKSRWQQGNVFSC
SVMHEALHNHYTQKSLSLSP (SEQ ID NO:5).
```

In some embodiments, the immunocytokine comprises IL-15 linked to antibody heavy chain constant domains that have the amino acid sequence represented by

EP 4 032 539 A1

DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSADKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPCRDELTKNQV
SLWCLVKGFYPSDIAVEWESNGQPENNYKTTPPVLDSDGSFFLYSKLTVDKSRWQQGNVFSC
SVMHEALHNHYTQKSLSLSP (SEQ ID NO:23).

The amino acid sequence represented by SEQ ID NO:23 is modified from the amino acid sequence represented by SEQ ID NO:5 by a single amino acid substitution of Cysteine (C) to Alanine (A) at position 229. This substitution enables to produce an immunocytokine that comprises a first antibody heavy chain constant domain and an antibody light chain constant domain within a heterodimeric complex, which do not have a covalent association through a natural S-S bridge.

In some embodiments, the immunocytokine comprises an immunomodulatory antibody or an antigen binding fragment thereof, which specifically inhibit the PD-1 pathway, and is an antibody that specifically binds to PD-1.

Specific binding can be measured, for example, by determining binding of a molecule compared to binding of a control molecule. For example, specific binding can be determined by competition with a control molecule that is similar to the target, for example, an excess of non-labeled target. In this case, specific binding is indicated if the binding of the labeled target to a probe is competitively inhibited by excess unlabeled target. As used in the present description, the term "specific binding" or "specifically binds to" or is "specific for" a particular polypeptide or an epitope on a particular polypeptide target can be described by a molecule having a Kd for the target of at least about 200 nM, or at least about 150 nM, or at least about 100 nM, or at least about 60 nM, or at least about 50 nM, or at least about 40 nM, or at least about 30 nM, or at least about 20 nM, or at least about 10 nM, or at least about 8 nM, or at least about 6 nM, or at least about 4 nM, or at least about 2 nM, or at least about 1 nM, or greater. In one embodiment, the term "specific binding" refers to binding where a molecule binds to a particular polypeptide or epitope on a particular polypeptide without substantially binding to any other polypeptide or polypeptide epitope.

In some embodiments, the immunocytokine comprises an immunomodulatory antibody comprising:

a) a light chain comprising a light chain variable domain and a light chain constant domain;
b) a heavy chain comprising a heavy chain variable domain and antibody heavy chain constant domains comprising a first (CH1) heavy chain constant domain and an Fc fragment monomer comprising second (CH2) and third (CH3) heavy chain constant domains.

In some embodiments, the immunocytokine comprises a light chain variable domain comprising LCDRs 1, 2, and 3 (hypervariable regions 1, 2, and 3), which are represented by the amino acid sequences GGNNIGSKNVH (SEQ ID NO:14), RDSNRPS (SEQ ID NO:15), and CQVWDSSTAV (SEQ ID NO:16), respectively.

In some embodiments, the immunocytokine comprises a light chain variable domain comprising the amino acid sequence represented by

QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQ (SEQ ID NO: 18).

In some embodiments, the immunocytokine comprises a heavy chain variable domain comprising HCDRs 1, 2, and 3 (hypervariable regions 1, 2, and 3), which are represented by the amino acid sequences FTFSSYWMY (SEQ ID NO:11), AIDTGGGRTYYADSVKG (SEQ ID NO:12), and CARDEGGGTGWGVLKDWPYGLDA (SEQ ID NO:13), respectively.

In some embodiments, the immunocytokine comprises a heavy chain variable domain comprising the amino acid sequence represented by

QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSS (SEQ ID NO: 17).

In some embodiments, the immunocytokine comprises:

1) a light chain variable domain comprising LCDRs 1, 2, and 3 (hypervariable regions 1, 2, and 3), which are represented by the amino acid sequences GGNNIGSKNVH (SEQ ID NO: 14), RDSNRPS (SEQ ID NO: 15), and CQVWDSSTAV (SEQ ID NO: 16), respectively;

2) a heavy chain variable domain comprising HCDRs 1, 2, and 3 (hypervariable regions 1, 2, and 3), which are represented by the amino acid sequences FTFSSYWMY (SEQ ID NO: 11), AIDTGGGRTYYADSVKG (SEQ ID NO: 12) and CARDEGGGTGWGVLKDWPYGLDA (SEQ ID NO: 13), respectively.

In some embodiments, the immunocytokine comprises:

1) a light chain variable domain comprising the amino acid sequence represented by

```
QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQ (SEQ ID NO:18);
```

2) a heavy chain variable domain comprising the amino acid sequence represented by

```
QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSS (SEQ ID NO:17).
```

In some embodiments, the immunocytokine comprises an immunomodulatory antibody that comprises a light chain comprising the amino acid sequence represented by

```
QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO 8).
```

In some embodiments, the immunocytokine comprises an immunomodulatory antibody that comprises a heavy chain comprising the amino acid sequence represented by

```
QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVCTLPPSRDELTK
NQVSLSCAVKGFYPSDIAVEWESNGQPENNYKTTPPVLDSDGSFFLVSKLTVDKSRWQQGNV
FSCSVMHEALHNHYTQKSLSLSP (SEQ ID NO 7).
```

In some embodiments, the immunocytokine comprises an immunomodulatory antibody comprising:

a heavy chain comprising the amino acid sequence represented by

```
QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
```

VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVCTLPPSRDELTK
NQVSLSCAVKGFYPSDIAVEWESNGQPENNYKTTPPVLDSDGSFFLVSKLTVDKSRWQQGNV
FSCSVMHEALHNHYTQKSLSLSP (SEQ ID NO 7);

a light chain comprising the amino acid sequence represented by

QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO 8).

In some embodiments, the immunocytokine comprises the immunomodulatory antibody prolgolimab (BCD100, WO2018013017).

In some embodiments, the immunocytokine comprises an immunomodulatory antibody or an antigen binding fragment thereof, which specifically inhibit the PD-1 pathway, which is an antibody that specifically binds to PD-L1.

In some embodiments, the antibody that specifically binds to PD-L1 is BCD135, which is disclosed in RU2665790 (WO2018194496).

In some embodiments, the immunocytokine comprises:

a) a heterodimeric protein complex based on IL-15/IL-15Rα comprising:

IL-15Rα linked to an antibody light chain constant domain that has the amino acid sequence represented by

DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEC (SEQ ID NO:1),

and
IL-15 linked to antibody heavy chain constant domains that have the amino acid sequence represented by

DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPCRDELTKNQV
SLWCLVKGFYPSDIAVEWESNGQPENNYKTTPPVLDSDGSFFLYSKLTVDKSRWQQGNVFSC
SVMHEALHNHYTQKSLSLSP (SEQ ID NO:5);

b) an immunomodulatory antibody comprising:

a heavy chain comprising the amino acid sequence represented by

QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVCTLPPSRDELTK

NQVSLSCAVKGFYPSDIAVEWESNGQPENNYKTTPPVLDSDGSFFLVSKLTVDKSRWQQGNV
FSCSVMHEALHNHYTQKSLSLSP (SEQ ID NO 7);

a light chain comprising the amino acid sequence represented by

QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO 8).

In some embodiments, the immunocytokine comprises:

a) a heterodimeric protein complex based on IL-15/IL-15Rα comprising:

IL-15Rα linked to an antibody light chain constant domain that has the amino acid sequence represented by

DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGRTVAAPSVFIFPPSD
EQLKSGTASVVCLLNNFYPREAKVQWKVDNALQSGNSQESVTEQDSKDSTYSLSSTLTLSKA
DYEKHKVYACEVTHQGLSSPVTKSFNRGEA (SEQ ID NO:19),

and
IL-15 linked to antibody heavy chain constant domains that have the amino acid sequence represented by

DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSADKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPCRDELTKNQV
SLWCLVKGFYPSDIAVEWESNGQPENNYKTTPPVLDSDGSFFLYSKLTVDKSRWQQGNVFSC
SVMHEALHNHYTQKSLSLSP (SEQ ID NO:23);

b) an immunomodulatory antibody comprising:

a heavy chain comprising the amino acid sequence represented by

QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVCTLPPSRDELTK
NQVSLSCAVKGFYPSDIAVEWESNGQPENNYKTTPPVLDSDGSFFLVSKLTVDKSRWQQGNV
FSCSVMHEALHNHYTQKSLSLSP (SEQ ID NO 7);

a light chain comprising the amino acid sequence represented by

QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO 8).

In some embodiments, the immunocytokine comprises:

a) IL-15Rα linked to antibody heavy chain constant domains that have the amino acid sequence represented by

DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE

YKCKVSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHYTQKSLS
LSP (SEQ ID NO:6),

and IL-15 linked to an antibody light chain constant domain that has the amino acid sequence represented by

DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO:3);

b) an immunomodulatory antibody comprising:

a heavy chain comprising the amino acid sequence represented by

QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVCTLPPSRDELTK
NQVSLSCAVKGFYPSDIAVEWESNGQPENNYKTTPPVLDSDGSFFLVSKLTVDKSRWQQGNV
FSCSVMHEALHNHYTQKSLSLSP (SEQ ID NO 7);

a light chain comprising the amino acid sequence represented by

```
QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO 8).
```

In some embodiments, the immunocytokine comprises:

a) IL-15Rα linked to antibody heavy chain constant domains that have the amino acid sequence represented by

```
DTCPPPMSVEHADIWVKSYSLYSRERYICNSGFKRKAGTSSLTECVLNKATNVAHWTTPSLK
CIRDPALVHQRPAPPSTVTTAGVTPQPESLSPSGKEPAASSGAEGGGASTKGPSVFPLAPSS
KSTSGGTAALGCLVKDYFPEPVTVSWNSGALTSGVHTFPAVLQSSGLYSLSSVVTVPSSSLG
TQTYICNVNHKPSNTKVDKRVEPKSADKTHTCPPCPAPEAAGGPSVFLFPPKPKDTLMISRT
PEVTCVVVDVSHEDPEVKFNWYVDGVEVHNAKTKPREEQYNSTYRVVSVLTVLHQDWLNGKE
YKCKVSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHYTQKSLS
LSP (SEQ ID NO:24),
```

and
IL-15 linked to an antibody light chain constant domain that has the amino acid sequence represented by

```
DNWVNVISDLKKIEDLIQSMHIDATLYTESDVHPSCKVTAMKCFLLELQVISLESGDASIHD
TVENLIILANDSLSSNGNVTESGCKECEELEEKNIKEFLQSFVHIVQMFINTSAGGKPGGSA
GGRTVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEA (SEQ ID NO:21);
```

b) an immunomodulatory antibody comprising:

a heavy chain comprising the amino acid sequence represented by

```
QVQLVQSGGGLVQPGGSLRLSCAASGFTFSSYWMYWVRQVPGKGLEWVSAIDTGGGRTYYAD
SVKGRFAISRVNAKNTMYLQMNSLRAEDTAVYYCARDEGGGTGWGVLKDWPYGLDAWGQGTL
VTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPEAAG
GPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVCTLPPSRDELTK
NQVSLSCAVKGFYPSDIAVEWESNGQPENNYKTTPPVLDSDGSFFLVSKLTVDKSRWQQGNV
FSCSVMHEALHNHYTQKSLSLSP (SEQ ID NO 7);
```

a light chain comprising the amino acid sequence represented by

```
QPVLTQPLSVSVALGQTARITCGGNNIGSKNVHWYQQKPGQAPVLVIYRDSNRPSGIPERFS
GSNSGNTATLTISRAQAGDEADYYCQVWDSSTAVFGTGTKLTVLQRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO 8).
```

In some embodiments, the immunocytokine has mutations in antibody constant domains, which induce the heterodimerization of two different portions, one of which comprises covalently or non-covalently associated IL-15Rα linked to antibody constant domains and IL-15 linked to an antibody constant domain, and the other portion comprises covalently or non-covalently associated light and heavy chains of antibody.

In some embodiments, the immunocytokine comprises:

a first Fc monomer and a second Fc monomer, which are selected from the following group: the first Fc monomer is a Knob modified Fc, and the second Fc monomer is a Hole modified Fc, or when the second Fc monomer is a Knob modified Fc, and the first Fc monomer is a Hole modified Fc.

"Knobs -into-holes" (interactions of the "knobs -into-holes" type) is an approach that enables to circumvent the problem associated with mispaired byproducts. This approach aims at forcing the pairing of two different antibody heavy chains by introducing mutations into the CH3 domains to modify the contact interfaces. On one chain, bulky amino acids were replaced by amino acids with short side chains to create a "hole". Conversely, amino acids with large side chains were introduced into the other CH3 domain to create a "knob". By co-expressing these two heavy chains, a high yield of heterodimer formation ("knob-hole") versus homodimer formation ("hole-hole" or "knob-knob") was observed (Ridgway, J. B., Presta L G, Carter P; and WO 1996/027011). The percentage of heterodimer can be further increased by remodeling the interfaces of the two CH3 domains using a phage display technology and the introduction of a disulfide bridge to stabilize the heterodimers (Merchant, A. M., et al, Nature Biotech 16 (1998) 677-681; Atwell, S., Ridgway, J. B., Wells, J. A., Carter, P., J Mol Biol 270 (1997) 26-35).

In some embodiments, the immunocytokine comprises a first Fc monomer that has the amino acid substitutions S354C/T366W, and a second Fc monomer that has the amino acid substitutions Y349C/T366S/L368A/Y407V.

In some embodiments, the immunocytokine comprises a first Fc monomer that has the amino acid substitutions Y349C/T366S/L368A/Y407, and a second Fc monomer that has the amino acid substitutions S354C/T366W.

In some embodiments, the immunocytokine comprises an Fc fragment that belongs to IgG.

In some embodiments, the immunocytokine comprises an Fc fragment, wherein the Fc fragment isotype is selected from the group comprising: human IgG1, IgG2, or IgG4.

In some embodiments, the immunocytokine comprises mutations in the Fc fragment monomer, which cause the deficiency of ADCC, CDC, and/or ADCP properties in said immunocytokine.

Further, the bispecific IL15 super-agonist or bispecific IL15 immunocytokine or anti-PD1/IL15SA immunocytokine (bispecific monoclonal antibody comprising IL-15SA and anti-PD-1 Fab fragment) is understood to mean:

Anti-PD-1 Fab-CK _IL15Ra_Hc _IL15CH1FcknobLALA,
Anti-PD-1 Fab- CK_IL15_Hc_IL15RaCH1FcknobLALA.

Further, the anti-PD-L1/IL15SA immunocytokine is understood to mean:

Anti-PD-L1 Fab-CK _IL15Ra_Hc _IL15CH1FcknobLALA,
Anti-PD-L1 Fab- CK_IL15_Hc_IL15RaCH1FcknobLALA.

The meanings of the designations Anti-PD-1 Fab, Anti-PD-LI Fab, CK, IL15Ra, IL15, CHIFcknobLALA are given throughout the text of the application.

Anti-PD-1 Fab-CK_IL15Ra_Hc _IL15CH1FcknobLALA is the above immunocytokine comprising an antigen binding Fab fragment of an anti-PD1 antibody, IL15Ra, linked to the antibody light chain constant domain CK, and IL15 linked to the antibody heavy chain constant domains comprising a first (CH1) heavy chain constant domain and an Fc fragment monomer comprising second (CH2) and third (CH3) heavy chain constant domains, wherein the Fc comprises mutations to form heterodimerization (e.g. knob) and LALA.

Anti-PD-1 Fab- CK_IL15_Hc_IL15RaCH1FcknobLALA is the above immunocytokine comprising an antigen binding Fab fragment of an anti-PD1 antibody, IL15, linked to the antibody light chain constant domain CK, and IL15Ra linked to the antibody heavy chain constant domains comprising a first (CH1) heavy chain constant domain and an Fc fragment monomer comprising second (CH2) and third (CH3) heavy chain constant domains, wherein the Fc comprises mutations to form heterodimerization (e.g. knob) and LALA.

Anti-PD-L1 Fab-CK _IL15Ra_Hc _IL15CH1FcknobLALA is the above immunocytokine comprising an antigen binding Fab fragment of an anti-PD-LI antibody, IL15Ra, linked to the antibody light chain constant domain CK, and IL15 linked to the antibody heavy chain constant domains comprising a first (CH1) heavy chain constant domain and an Fc fragment monomer comprising second (CH2) and third (CH3) heavy chain constant domains, wherein the Fc comprises mutations to form heterodimerization (e.g. knob) and LALA.

Anti-PD-LI Fab- CK_IL15_Hc_IL15RaCH1FcknobLALA is the above immunocytokine comprising an antigen binding Fab fragment of an anti-PD-LI antibody, IL15, linked to the antibody light chain constant domain CK, and IL15Ra linked to the antibody heavy chain constant domains comprising a first (CH1) heavy chain constant domain and an Fc fragment monomer comprising second (CH2) and third (CH3) heavy chain constant domains, wherein the Fc comprises mutations to form heterodimerization (e.g. knob) and LALA.

The above immunocytokines can be made both in the format, where the first antibody heavy chain constant domain and the antibody light chain constant domain within the heterodimeric complex have a covalent association through a natural S-S bridge, and in the format, where the first antibody heavy chain constant domain and the antibody light chain constant domain within the heterodimeric complex do not have a covalent association through the natural S-S bridge; in a particular case, to this end, the both cysteines are replaced by alanine.

Experimental studies of the above immunocytokines both in the format, where the first antibody heavy chain constant domain and the antibody light chain constant domain within the heterodimeric complex have a covalent association through a natural S-S bridge, and in the format, where the first antibody heavy chain constant domain and the antibody light chain constant domain within the heterodimeric complex do not have a covalent association through a natural S-S bridge, demonstrated no significant difference in the activity of the above cytokines.

"Knob", as described above, is understood to mean mutations forming a "knob-into-hole: structure between the first Fc variant and the second Fc variant in the CH3 domain.

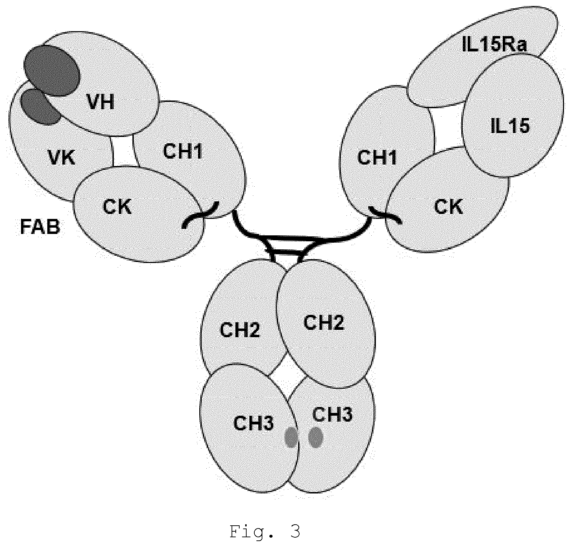

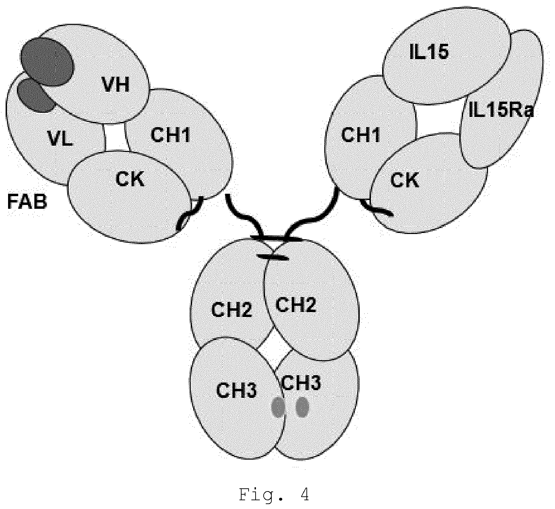

The format of the above immunocytokine is shown in Figures 3 and 4.

In some embodiments, the immunocytokine is used for the treatment of an oncological or autoimmune disease.

In some embodiments, the immunocytokine is used for the treatment of an oncological disease.

Nucleic acid

In one aspect, the present invention relates to an isolated nucleic acid that encodes any of the above immunocytokines.

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-strand DNA or RNA, a single-strand DNA or RNA, or transcription products of said DNAs.

It should also be included here that the present invention does not relate to nucleotide sequences in their natural chromosomal environment, i.e. in a natural state. The sequences of the present invention have been isolated and/or purified, i.e., they were sampled directly or indirectly, for example by a copy, their environment having been at least partially modified. Thus, isolated nucleic acids obtained by recombinant genetics, by means, for example, of host cells, or obtained by chemical synthesis should also be mentioned here.

An "isolated" nucleic acid molecule is one which is identified and separated from at least one nucleic acid molecule-impurity, which the former is bound to in the natural source. An isolated nucleic acid molecule is different from the form or set in which it is found under natural conditions. Thus, an isolated nucleic acid molecule is different from a nucleic acid molecule that exists in cells under natural conditions. An isolated nucleic acid molecule however includes a nucleic acid molecule located in cells in which the immunocytokine is normally expressed, for example, if the nucleic acid molecule has a chromosomal localization that is different from its localization in cells under natural conditions.

In some embodiments, the nucleic acid is DNA.

In one embodiment, the present invention relates to a nucleic acid molecule comprising a nucleotide sequence that encodes an amino acid sequence selected from SEQ ID NOs: 1, 2, 3, 4, 5, 6, 7, 8, 9, 10, 11, 12, 13, 14, 15, 16, 17, 18, 19, 20, 21, 22, 23, or 24. A nucleic acid molecule can also comprise any combination of the above nucleotide sequences.

As would be appreciated by those skilled in the art, because of the redundancy of the genetic code, a variety of different DNA sequences can encode the peptide with an amino acid sequence selected from SEQ ID NOs: 1, 2, 3, 4, 5, 6, 7, 8, 9, 10, 11, 12, 13, 14, 15, 16, 17 , 18, 19, 20, 21, 22, 23, or 24. It is well within the skill of a person trained in the art to create these alternative DNA sequences encoding the same amino acid sequences. Such variant DNA sequences are within the scope of the present invention.

Expression vector

In one aspect, the present invention relates to an expression vector comprising the above nucleic acid.

The term "vector" as used herein means a nucleic acid molecule capable of transporting another nucleic acid to which it has been linked. In some embodiments, a vector is a plasmid, i.e., a circular double stranded piece of DNA into which additional DNA segments may be ligated. In some embodiments, a vector is a viral vector, wherein additional DNA segments may be ligated into the viral genome. In some embodiments, vectors are capable of autonomous replication in a host cell into which they are introduced (e.g. bacterial vectors having a bacterial origin site of replication and episomal mammalian vectors). In further embodiments, vectors (e.g. non-episomal mammalian vectors) can be integrated into the genome of a host cell upon introduction into a host cell, and thereby are replicated along with the host gene. Moreover, certain vectors are capable of directing the expression of genes to which they are operably linked. Such vectors are referred to herein as "recombinant expression vectors" (or simply, "expression vectors").

The present invention relates to vectors comprising the above nucleic acid molecules that encode any of the amino acid sequences of the above immunocytokine or structural portions thereof as described herein.

In some embodiments, the immunocytokine of the present invention is expressed by inserting DNA encoding partially or entirely the sequence of the first and second immunocytokine domains (e.g. a domain comprising IL15 or IL15Ra linked to antibody constant domain(s), and/or antibody light and heavy chains), which is produced as described above in expression vectors such that the genes are operably linked to the desired expression control sequences, such as transcriptional and translational control sequences. Expression vectors include plasmids, retroviruses, adenoviruses, adeno-associated viruses (AAV), plant viruses, such as cauliflower mosaic virus, tobacco mosaic virus, cosmids, YACs, EBV derived episomes, and the like. DNA molecules may be ligated into a vector such that transcriptional and translational control sequences within the vector serve their intended function of regulating the transcription and translation of the DNA. An expression vector and expression control sequences may be chosen to be compatible with the expression host cell used. DNA molecules partially or fully encoding the sequences of first and second binding domains (for example, heavy and light chain sequences where a binding domain comprises a heavy and light chain sequence) can be introduced into individual vectors. In one embodiment, any combination of said DNA molecules is introduced into the same expression vector. DNA molecules can be introduced into an expression vector by standard methods (e.g. ligation of complementary restriction sites on an antibody gene fragment and vector, or blunt end ligation if no restriction sites are present).

A suitable vector is one that encodes functionally complete human CH or CL/CK immunoglobulin sequences, with appropriate restriction site engineering so that any sequence, VH or VL or IL15 or IL15Rα, can easily be inserted and expressed, as described above. HC- and LC-encoding of genes in such vectors may contain intron sequences, resulting in enhanced overall antibody protein yields by stabilizing the corresponding mRNA. The intron sequences are flanked by splice donor and splice acceptor sites, which determine where RNA splicing will occur. Location of intron sequences can be either in variable or constant regions of antibody chains, or in both variable and constant regions when multiple introns are used. Polyadenylation and transcription termination may occur at a native chromosomal site downstream of coding regions. A recombinant expression vector can also encode a signal peptide that facilitates secretion of an antibody chain from a host cell. The gene for the antibody chain or the gene for the chain comprising IL15 or IL15Ra can be cloned into the vector in such a way that the signal peptide is linked with the reading frame of the amino terminus of the immunoglobulin chain. The signal peptide can be an immunoglobulin signal peptide or a heterologous signal peptide (i.e., a signal peptide from a non-immunoglobulin protein).

In addition to the genes for the antibody chain or the gene for the chain comprising IL15 or IL15Rα, the recombinant expression of the vectors of the invention may comprise regulatory sequences, which control expression of the genes in a host cell. It will be understood by those skilled in the art that the design of an expression vector, including the selection of regulatory sequences, may depend on such factors as the choice of a host cell to be transformed, the level of expression of a desired protein, and so forth. Preferred control sequences for an expression host cell in mammals include viral elements that ensure high levels of protein expression in mammalian cells, such as promoters and/or enhancers derived from a retroviral LTR, cytomegalovirus (CMV) (such as a CMV promoter/enhancer), simian virus 40 (SV40) (such as a SV40 promoter/enhancer), adenovirus, (e.g. the major late promoter adenovirus (AdMLP)), polyomavirus and strong mammalian promoters such as native immunoglobulin promoter or actin promoter. For further description of viral control elements and sequences thereof, see, e.g. US patents Nos. 5,168,062, 4,510,245 and 4,968,615. Methods for expressing polypeptides in bacterial cells or fungal cells, e.g. yeast cells, are also well known in the art.

In addition to the above genes and regulatory sequences, recombinant expression vectors of the invention may carry additional sequences, such as sequences that regulate replication of a vector in host cells (e.g. origins of replication) and selectable marker genes. The selectable marker gene facilitates the selection of host cells into which a vector has been introduced (see e.g. U.S. Patent Nos. 4,399,216, 4,634,665 and 5,179,017). For example, the selectable marker gene typically confers resistance to medicinal agents, such as G418, hygromycin or methotrexate, on a host cell into which the vector has been introduced. For example, selectable marker genes include a dihydrofolate reductase (DHFR) gene (for use in dhfr-host cells during methotrexate selection/amplification), a neo gene (for G418 selection), and a glutamate synthetase gene.

The term "expression control sequence" as used in the present description refers to polynucleotide sequences that are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "control sequences" is intended to include at least all components, the presence of which is essential for expression and processing, and can also include additional components, the presence of which is advantageous, for example, leader sequences and fusion partner sequences.

The term "control sequences" refers to DNA sequences necessary for the expression of an operably linked coding sequence in a particular host organism. The control sequences that are suitable for prokaryotes, for example, include a promoter, optionally an operator sequence and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader sequence is operably linked to DNA for a polypeptide if it is expressed as a preprotein that participates in the secretion of the polypeptide; a promoter or enhancer is operably linked to a coding sequence if it affects the transcription of the sequence; a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous.

Host cells and method for production thereof

In one aspect, the present invention relates to a method for production of a host cell for production of the above immunocytokine, which comprises transformation of the cell with the above vector.

Nucleic acid molecules encoding immunocytokines of the invention and vectors comprising these nucleic acid molecules can be used for transfection of a suitable mammalian or cell thereof, plant or cell thereof, bacterial or yeast host cell. Transformation can be by any known technique for introducing polynucleotides into a host -cell. Methods for introduction of heterologous polynucleotides into mammalian cells are well known in the art and include dextran--mediated transfection, cationic polymer-nucleic acid complex transfection, calcium phosphate precipitation, polybrene--mediated transfection, protoplast fusion, encapsulation of the polynucleotide(s) in liposomes, and direct microinjection of DNA into nuclei. In addition, nucleic acid molecules may be introduced into mammalian cells by viral vectors. Methods for transfecting cells are well known in the art. See, e.g. U.S. patent Nos. 4,399,216, 4,912,040, 4,740,461 and 4,959,455. Methods for transforming plant cells are well known in the art, including, e.g. Agrobacterium-mediated transformation, biolistic transformation, direct injection, electroporation and viral transformation. Methods for transforming bacterial and yeast cells are also well known in the art.

In one aspect, the present invention relates to a host cell for production of the above immunocytokine, which comprises the above nucleic acid.

In one aspect, the present invention relates to a method for production of a drug comprising the above immunocytokine, which comprises culturing of the above host cell in a culture medium under conditions sufficient to produce said immunocytokine, if necessary, followed by isolation and purification of the resulting immunocytokine.

The term "recombinant host cell" (or simply "host cell") as used herein refers to a cell into which a recombinant expression vector has been introduced. The present invention relates to host cells, which may include, for example, a vector according to the invention described above. The present invention further relates to host cells comprising, for example, a nucleotide sequence encoding the immunocytokine or the above portions thereof of the present invention. It should be understood that "recombinant host cell" and "host cell" refer not only to a particular subject cell but to the progeny of such a cell as well. Since modifications may occur in succeeding generations due to either mutation or environmental influences, such progeny may not, in fact, be identical to a parental cell; however, such cells are still included within the scope of the term "host cell" as used herein.

Mammalian cell lines used as hosts for transformation are well known in the art and include a plurality of immortalized cell lines available. These include, e.g., Chinese hamster ovary (CHO) cells, NS0 cells, SP2 cells, HEK-293T cells, FreeStyle 293 cells (Invitrogen), NIH-3T3 cells, HeLa cells, baby hamster kidney (BHK) cells, African green monkey kidney cells (COS), human hepatocellular carcinoma cells (e.g., Hep G2), A549 cells, and a number of other cell lines. Cell lines are selected by determining which cell lines have high expression levels and provide for necessary characteristics of the protein produced. Other cell lines that may be used are insect cell lines, such as Sf9 or Sf21 cells. When the recombinant expression vectors encoding the immunocytokine of the invention or a portion thereof are introduced into mammalian host cells, the immunocytokines are produced by culturing the host cells for a period of time sufficient to express the immunocytokine or a portion thereof of the invention in the host cells, or, more preferably, secrete the immunocytokine into the culture medium in which the host cells are cultured. Immunocytokines can be isolated from the culture medium using standard protein purification techniques. Plant host cells include, e.g. Nicotiana, Arabidopsis, duckweed, corn, wheat, potato, etc. Bacterial host cells include Escherichia and Streptomyces species. Yeast host cells include Schizosaccharomyces pombe, Saccharomyces cerevisiae and Pichia pastoris.

Furthermore, level of production of immunocytokine of the invention from a producing cell line can be enhanced using a number of known techniques. For example, the glutamine synthetase gene expression system (the GS system) is a common approach for enhancing expression under certain conditions. The GS system is discussed in whole or part in connection with EP Nos. 0216846, 0256055, 0323997 and 0338841.

It is likely that the immunocytokine or a portion thereof of the invention in different cell lines or host cells will have different glycosylation patterns from each other. However, immunocytokine as disclosed herein or a portion (domain) thereof comprising the amino acid sequences provided herein are part of the present invention, regardless of the glycosylation of the binding molecules, and, in general, regardless of the presence or absence of post-translational modifications.

Pharmaceutical composition

In one aspect, the present invention relates to a pharmaceutical composition for stimulating the activation and/or proliferation of IL-15Rbeta/gamma-positive cells, which comprises the above immunocytokine in a therapeutically effective amount, in combination with one or more pharmaceutically acceptable excipients.

"Pharmaceutical composition" means a composition comprising immunocytokine of the present invention and at least one of components selected from the group consisting of pharmaceutically acceptable and pharmacologically compatible excipients, such as fillers, solvents, diluents, carriers, auxiliary, distributing agents, delivery agents, preservatives, stabilizers, emulsifiers, suspending agents, thickeners, prolonged delivery controllers, the choice and proportions of which depend on the type and route of administration and dosage. Pharmaceutical compositions of the present invention and methods for preparation thereof will be undoubtedly apparent to those skilled in the art. Pharmaceutical compositions should preferably be manufactured in compliance with the GMP (Good Manufacturing Practice) requirements. A composition may comprise a buffer composition, tonicity agents, stabilizers and solubilizers. Prolonged action of a composition may be achieved by agents slowing down absorption of active pharmaceutical ingredient, for example, aluminum monostearate and gelatine. Examples of suitable carriers, solvents, diluents and delivery agents include water, ethanol, polyalcohols and their mixtures, oils, and organic esters for injections.

"Therapeutically effective amount" refers to that amount of the therapeutic agent being administered which will relieve to some extent one or more of the symptoms of the disorder being treated.

"Medicament (drug)" is a compound or a mixture of compounds as a pharmaceutical composition in the form of tablets, capsules, powders, lyophilisates, injections, infusions, ointments and other ready forms intended for restoration, improvement or modification of physiological functions in humans and animals, and for treatment and preventing of diseases, for diagnostics, anesthesia, contraception, cosmetology and others. Any method for administering peptides, proteins or antibodies accepted in the art may be suitably employed for immunocytokine of the invention.

The term "pharmaceutically acceptable" refers to one or more compatible liquid or solid components that are suitable for administration in a mammal, preferably a human.

The term "excipient" is used herein to describe any ingredient other than the above ingredients of the invention. These are substances of inorganic or organic nature which are used in the pharmaceutical manufacturing in order to give drug products the necessary physicochemical properties.

The term "buffer", "buffer composition", "buffering agent" refers to a solution, which is capable of resisting changes in pH by the action of its acid-base conjugate components, and which allows the immunocytokine product of the invention to resist changes in pH. Generally, the pharmaceutical composition preferably has a pH in the range from 4.0 to 8.0. Examples of buffers used include, but are not limited to, acetate, phosphate, citrate, histidine, succinate, etc. buffer solutions.

The terms "tonic agent", "osmolyte" or "osmotic agent", as used herein, refer to an excipient that can increase the osmotic pressure of a liquid antibody formulation. "Isotonic" drug is a drug that has an osmotic pressure equivalent to that of human blood. Isotonic drugs typically have an osmotic pressure from about 250 to 350 mOsm/kg. Isotonic agents used include, but are not limited to, polyols, saccharides and sucrose, amino acids, metal salts, for example, sodium chloride, etc.

"Stabilizer" refers to an excipient or a mixture of two or more excipients that provide the physical and/or chemical stability of the active agent. Stabilizers include amino acids, for example, but are not limited to, arginine, histidine, glycine, lysine, glutamine, proline; surfactants, for example, but are not limited to, polysorbate 20 (trade name: Tween 20), polysorbate 80 (trade name: Tween 80), polyethylene-polypropylene glycol and copolymers thereof (trade names: Poloxamer, Pluronic, sodium dodecyl sulfate (SDS); antioxidants, for example, but are not limited to, methionine, acetylcysteine, ascorbic acid, monothioglycerol, sulfurous acid salts, etc.; chelating agents, for example, but are not limited to, ethylenediaminetetraacetic acid (EDTA), diethylenetriaminepentaacetic acid (DTPA), sodium citrate, etc.

A pharmaceutical composition is "stable" if the active agent retains physical stability and/or chemical stability and/or biological activity thereof during the specified shelf life at storage temperature, for example, of 2-8 °C. Preferably, the active agent retains both physical and chemical stability, as well as biological activity. Storage period is adjusted based on the results of stability test in accelerated or natural aging conditions.

A pharmaceutical composition of the invention can be manufactured, packaged, or widely sold in the form of a single unit dose or a plurality of single unit doses in the form of a ready formulation. The term "single unit dose" as used herein refers to a discrete quantity of a pharmaceutical composition containing a predetermined quantity of an active ingredient. The quantity of the active ingredient typically equals the dose of the active ingredient to be administered in a subject, or a convenient portion of such dose, for example, half or a third of such dose.

The pharmaceutical compositions according to the present invention are typically suitable for parenteral administration as sterile formulations intended for administration in a human body through the breach in skin or mucosal barriers, bypassing the gastrointestinal tract by virtue of injection, infusion and implantation. For example, parenteral administration is intended to include, inter alia, subcutaneous, intraperitoneal, intramuscular, intrasternal, intravenous, intraarterial, intrathecal, intraventricular, intraurethral, intracranial, intrasynovial, transdermal injection or infusions; and kidney dialytic infusion techniques. Intra-tumor delivery, for example, intra-tumor injection, can also be employed. Regional perfusion is also provided. Preferred embodiments include intravenous and subcutaneous routes. Any method for administering peptides or proteins accepted in the art may be suitably employed for the immunocytokine of the invention.

Injectable formulations may be prepared, packaged, or sold, without limitation, in unit dosage form, such as in ampoules, vials, in plastic containers, pre-filled syringes, autoinjection devices. Formulations for parenteral administration include, inter alia, suspensions, solutions, emulsions in oily or aqueous bases, pastes, and the like.

In another embodiment, the invention provides a composition for parenteral administration comprising a pharmaceutical composition which is provided in dry (i.e. powder or granular) form for reconstitution with a suitable base (e.g. sterile pyrogen-free water) prior to administration. Such formulation may be prepared by, for example, lyophilisation process, which is known in the art as freeze drying, and which involves freezing a product followed by removal of solvent from frozen material.

An immunocytokine of the invention can also be administered intranasally or by inhalation, either alone, as a mixture with a suitable pharmaceutically acceptable excipient from an inhaler, such as a pressurized aerosol container, pump, spray, atomiser, or nebuliser, wherein a suitable propellant is used or not used, or as nasal drops, or spray.

Dosage forms for parenteral administration may be formulated to be immediate or modified release. Modified release formulations include delayed, sustained, pulsed, controlled, targeted and programmed release.

In some embodiments, the pharmaceutical composition is used for the treatment of an oncological or autoimmune disease.

In some embodiments, the pharmaceutical composition is used for the treatment of an autoimmune disease.

The term "autoimmune disease" as used in this description refers to a non-malignant disease or disorder arising from and directed against an individual's own (self) antigens and/or tissues.

The definition of "autoimmune disease" encompasses, but is not limited to, rheumatoid arthritis, osteoarthritis, juvenile chronic arthritis, septic arthritis, Lyme osteoarthritis, psoriatic arthritis, reactive arthritis, spondyloarthropathy, systemic lupus erythematosus, Crohn's disease, ulcerative colitis, inflammatory bowel diseases, diabetes mellitus, thyroiditis, asthma, allergic diseases, psoriasis, atopic dermatitis, scleroderma, reaction "graft versus host", organ transplant rejection, acute or chronic immune diseases associated with transplantation, sarcoidosis, Kawasaki disease, Graves disease, nephrotic syndrome, chronic fatigue syndrome, Wegener's granulomatosis, Henoch-Schonlein purpura, microscopic renal vasculitis, chronic active hepatitis, uvenita, septic shock, toxic shock syndrome, sepsis syndrome, cachexia, acquired immunodeficiency syndrome, acute transverse myelitis, Huntington's chorea, Parkinson's disease, Alzheimer's disease, stroke, primary biliary cirrhosis, hemolytic anemia, adult (acute) respiratory distress syndrome, alopecia, alopecia areata, seronegative arthropathy, arthropathy, Reiter's disease, psoriatic arthropathy associated with ulcerative colitis arthropathy, atopic allergies, autoimmune bullous diseases, pemphigus vulgaris, sheet-like pemphigus, pemphigoid disease, linear IgA, autoimmune hemolytic anemia, Coombs-positive hemolytic anemia, pernicious anemia, juvenile pernicious anemia, arthritis, primary sclerosing hepatitis A, cryptogenic autoimmune hepatitis, fibrosis lung disease, cryptogenic fibrosis alveolitis, post-inflammatory interstitial lung diseases, interstitial pneumonitis, chronic eosinophilic pneumonia, post-infectious interstitial lung diseases, gouty arthritis, autoimmune hepatitis, autoimmune hepatitis type I (classical autoimmune hepatitis or lupoid), autoimmune hepatitis type II, osteoarthritis, primary sclerosing cholangitis, psoriasis type I, psoriasis type II, idiopathic leucopenia, autoimmune neutropenia, renal NOS-diseases, glomerulonephritis, microscopic renal vasculitis, discoid lupus erythematosus, idiopathic or NOS-male infertility, autoimmunity to sperm, multiple sclerosis (all subtypes), sympathetic ophthalmia, pulmonary hypertension secondary to connective tissue disease, Goodpasture syndrome, pulmonary manifestations of polyarthritis nodosa, acute rheumatic fever, rheumatoid spondylitis, ankylosing spondylitis, Still's disease, systemic sclerosis, Sjogren syndrome, Takayasu's disease, autoimmune thrombocytopenia, idiopathic thrombocytopenia, autoimmune thyroid disease, hyperthyroidism, Hashimoto's disease, autoimmune atrophic hypothyroidism, primary myxedema, phacogenic uveitis, primary vasculitis, vitiligo, acute liver diseases, chronic liver diseases, allergies, asthma, psychiatric disorders (including depression and schizophrenia), type Th2/type Th1-mediated diseases, conjunctivitis, allergic contact dermatitis, allergic rhinitis, deficiency of alpha-1-antitrypsin, amyotrophic lateral sclerosis, anemia, cystic fibrosis, disorders associated with cytokine therapy, demyelinating disease, dermatitis, iridocyclitis/uveitis/optic neuritis, ischemia-reperfusion injury, ischemic stroke, juvenile rheumatoid arthritis, autoimmune enteropathy, autoimmune hearing loss, autoimmune lymphoproliferative syndrome, autoimmune myocarditis, autoimmune premature ovarian failure, and blepharitis.

In some embodiments, the pharmaceutical composition is used for the treatment of an oncological disease.

The terms "oncological disease", "cancer" and "cancerous" refer to a physiological condition or describe a physiological condition in mammals that is typically characterized by unregulated growth/proliferation of cells. Examples of oncological diseases include, but are not limited to, carcinoma, lymphoma, blastoma, sarcoma, and leukemia. More particular examples of such cancerous diseases include squamous cell cancer, small-cell lung cancer, non-small cell lung cancer, adenocarcinoma of the lung and squamous carcinoma of the lung, peritoneal cancer, hepatocellular cancer, stomach cancer including gastrointestinal cancer, pancreatic cancer, glioblastoma, glioma, cervical cancer, ovarian cancer, liver cancer, bladder cancer, breast cancer, colon cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, and various head and neck cancers.

In some embodiments, the pharmaceutical composition is used for the treatment of an oncological disease that is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

The term "antiproliferative activity" is intended to refer to stopping or inhibiting growth of cells, such as cancer cells.

The term "$IC_{50}$" (inhibitory concentration 50%) refers to concentrations of drug, at which a measurable activity or response, for example, growth/proliferation of cells such as tumor cells, is inhibited by 50%. $IC_{50}$ value can be calculated using appropriate dose-response curves, using special statistical software for curve fitting.

The term GI50 (50% growth inhibition) refers to concentrations of drug, at which proliferation of cells, such as tumor cells, is inhibited by 50%.

The term "ED50" (EC50) (50% effective dose/concentration) refers to concentrations of drug producing 50% biological effect (which may include cytoxicity).

Method for treatment/Use for treatment

In one aspect, the present invention relates to a method for treatment of an oncological disease, which comprises administering to a subject in need of such treatment said immunocytokine or said pharmaceutical composition, in a therapeutically effective amount.

"Treat", "treatment" and "therapy" refer to a method of alleviating or abrogating a biological disorder and/or at least one of attendant symptoms thereof. As used herein, to "alleviate" a disease, disorder or condition means reducing the severity and/or occurrence frequency of the symptoms of a disease, disorder, or condition. Further, references herein to "treatment" include references to curative, palliative and prophylactic treatment.

In one aspect, the subject of treatment, or patient, is a mammal, preferably a human subject. Said subject may be either male or female, of any age.

The term "disease" means any condition that would benefit from treatment according to the present invention. The definition of this term includes chronic and acute disorders or diseases.

In some embodiments of the method for treatment, the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

In one aspect, the present invention relates to a method for activating the biological activity of a T cell population or NK cell population in a subject in need of such activation, which comprises administering to the subject an effective amount of the above immunocytokine or the above pharmaceutical composition.

In one aspect, the present invention relates to the use of said immunocytokine or said pharmaceutical composition for the treatment in a subject in need of such treatment of an oncological disease.

In some uses, the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T-and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

In the case of a tumor (for example, cancer), the therapeutically effective amount of an immunocytokine of the invention may reduce the number of cancer cells; reduce the initial tumor size; inhibit (i.e. slow to some extent and preferably stop) cancer cell infiltration into peripheral organs; inhibit (i.e. slow to some extent and preferably stop) tumor metastasis; inhibit to some extent tumor growth; and/or relieve to some extent one or more of the symptoms associated with the disorder. An immunocytokine of the invention may to some extent prevent growth and/or kill existing cancer cells, it may be cytostatic and/or cytotoxic. In the treatment of cancer, in vivo efficacy can, for example, be measured by assessing survival, time to tumor progression (TTP), tumor response rate to treatment (RR), duration of response and/or quality of life.

Immunocytokines of the invention can be administered without further therapeutic treatment, i.e. as an independent therapy. Furthermore, treatment by an immunocytokine of the invention may comprise at least one additional therapeutic treatment (combination therapy). In some embodiments, the immunocytokine may be administered jointly or formulated with another medication/preparation for the treatment of cancer.

As used herein, the terms "co-administration", "co-administered" and "in combination with", referring to an immunocytokine of the invention and one or more other therapeutic agents, are contemplated to mean, refer to or include the following:

1) simultaneous administration of such combination of an immunocytokine of the invention and a therapeutic agent to a patient in need of treatment, when such components are formulated together into a single dosage form which releases said components at substantially the same time to said patient,
2) simultaneous administration of such combination of an immunocytokine of the invention and a therapeutic agent to a patient in need of treatment, when such components are formulated apart from each other into separate dosage forms which are taken at substantially the same time by said patient, whereupon said components are released at substantially the same time to said patient,
3) sequential administration of such combination of an immunocytokine of the invention and a therapeutic agent to a patient in need of treatment, when such components are formulated apart from each other into separate dosage forms which are taken at consecutive times by said patient with a significant time interval between each administration, whereupon said components are released at substantially different times to said patient; and
4) sequential administration of such combination of an immunocytokine of the invention and a therapeutic agent to a patient in need of treatment, when such components are formulated together into a single dosage form which releases said components in a controlled manner, whereupon they are concurrently, consecutively, and/or overlappingly released at the same and/or different times to said patient, where each part may be administered by either the same or a different route.

An immunocytokine of the present invention can be combined with a therapeutic agent selected from the group comprising: a cytotoxic agent, a chemotherapeutic agent, an anti-hormonal agent, or another therapeutic antibody.

The term "cytotoxic agent" as used in this description refers to a substance that inhibits or prevents the function of cells and/or causes destruction of cells. The term is intended to include radioactive isotopes (e.g. $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclophosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylmelamine; acetogenins (e.g. bullatacin and bullatacinone); delta-9- tetrahydrocannabinol (dronabinol, MARINOL®); beta-lapachone; lapachol; colchicines; betulinic acid; camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its synthetic analogues adozelesin, carzelesin and bizelesin); podophyllotoxin; podophyllinic acid; teniposide; cryptophycins (e.g. cryptophycin 1 and cryptophycin 8);

dolastatin; duocarmycin (including the synthetic analogues KW-2189 and CB1-TM1); eleutherobin; pancratistatin; sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. calicheamicin such as calicheamicin gamma II and calicheamicin omega II (see, e.g., Agnew, Chem. Intl. Ed. Engl., 33: 183-186 (1994)); dynemicin, including dynemicin A; esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXOL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate, gemcitabine (GEMZAR®), tegafur (UFTORAL®), capecitabine (XELODA®), epothilone, and 5-fluorouracil (5-FU); folic acid analogues such as denopterin, methotrexate, pteropterin,trimetrexate; purine analogs such as fludarabine, 6-mercaptopurine, thiamiprine, thioguanine;pyrimidine analogs such as ancitabine, azacitidine, 6-azauridine, carmofur, cytarabine,dideoxyuridine, doxifluridine, enocitabine, floxuridine; anti-adrenals such as aminoglutethimide, mitotane, trilostane; folic acid replenisher such as frolinic acid; aceglatone; aldophosphamideglycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (e.g. T-2 toxin, verracurin A, roridin A and anguidine); urethan; dacarbazine; mannomustine; mitobronitol; mitolactol; pipobroman; gacytosine; arabinoside ("Ara-C"); thiotepa; taxoid, e.g. paclitaxel (TAXOL®), albumin-engineered nanoparticle formulation of paclitaxel (ABRAXANETM), and docetaxel (TAXOTERE®); chlorambucil; 6-thioguanine; mercaptopurine; methotrexate; platinum agents such as cisplatin, oxaliplatin, and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluorometlhylornithine (DMFO); retinoids such as retinoic acid, including bexarotene (TARGRETIN®); bisphosphonates such as clodronate (e.g. BONEFOS® or OSTAC®), etidronate (DIDROCAL®), NE-58095, zoledronic acid/zoledronate (ZOMETA®), alendronate (FOSAMAJX®), pamidronate (AREDIA®), tiludronate (SKELID®), or risedronate (ACTONEL®); troxacitabine (1,3-dioxolane nucleoside cytosine analogue); antisense oligonucleotides, e.g. those that inhibit expression of genes in signaling pathways implicated in aberrant cell proliferation, such as e.g. PKC-alpha, Raf, H-Ras, and epidermal growth factor receptor (EGF-R); vaccines such as THERATOPE® vaccine and gene therapy vaccines, e.g. ALLOVECTIN® vaccine, LEUVECTIN® vaccine, and VAXID® vaccine; topoisomerase 1 inhibitor (e.g. LURTOTECAN®); rmRH (e.g. ABARELIX®); BAY439006 (sorafenib; Bayer); SU-11248(Pfizer); perifosine, COX-2 inhibitor (e.g. celecoxib or etoricoxib), proteosome inhibitor (e.g. PS341); bortezomib (VELCADE®); CCI-779; tipifarnib (811577); orafenib, ABT510; Bcl-2inhibitor such as oblimersen sodium (GENASENSE®); pixantrone; EGFR inhibitors (see definition below); tyrosine kinase inhibitors (see definition below); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATINTM) combined with 5-FU and leucovorin.

Also included in this definition are anti-hormonal agents that act to regulate or inhibit hormone action on tumors, such as anti-estrogens with mixed agonist/antagonist profile, including, tamoxifen (NOLVADEX®), 4-hydroxytamoxifen, trioxifene, toremifene (FARESTON®), idoxifene, droloxifene, raloxifene (EVTSTA®), trioxifene, keoxifene, and selective estrogen receptor modulators (SERMs), such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors, such as formestane and exemestane (AROMASIN®), and nonsteroidal aromatase inhibitors, such as anastrazole (AREVIIDEX®), letrozole (FEMARA®) and aminoglutethimide, and other aromatase inhibitors including vorozole (RIVISOR®), megestrol acetate (MEGASE®), fadrozole, imidazole; lutenizing hormone-releasing hormone agonists, including leuprolide (LUPRON® and ELIGARD®), goserelin, buserelin, and tripterelin; sex steroids, including progestines, such as megestrol acetate and medroxyprogesterone acetate, estrogens, such as diethylstilbestrol and premarin, and androgens/retinoids such as fluoxymesterone, all transretionic acid and fenretinide; onapristone; anti-progesterones; estrogen receptor down-regulators (ERDs); anti-androgens, such as flutamide, nilutamide and bicalutamide; testolactone; and pharmaceutically acceptable salts, acids or derivatives of any of the above; as well as combinations of two or more of the above.

Other therapeutic agent that can be used in combination with an immunocytokine of the present invention may be a therapeutic antibody selected from the group comprising antibodies to PD1 (e.g. prolgolimab, pembrolizumab or nivolumab), antibodies to PD-L1, antibodies to CTLA4, antibodies to 4-1BB, antibodies to OX40, antibodies to GITR, antibodies to CD20 (e.g. rituximab), antibodies to HER2 (e.g. trastuzumab or pertuzumab), antibodies to VEGF (e.g. bevacizumab), or combinations thereof.

Other therapeutic agent that can be used in combination with an immunocytokine according to the invention can be a therapeutically active antitumour compound selected from the group of activators of innate or adaptive immunity.

It is understood that an immunocytokine of the invention may be used in methods for treating, as described above, in treatment, as described above, and/or in the manufacture of a medicament for the therapeutic applications described above.

Doses and routes of administration

An immunocytokine of the present invention will be administered in an amount that is effective in treatment of the condition in question, i.e. in doses and during the periods of time required to achieve the desired result. A therapeutically effective amount may vary according to factors such as the specific condition to be treated, age, sex, and weight of a patient, and whether the immunocytokine is administered alone or in combination with one or more additional drugs or treatment techniques.

Dosage regimens may be adjusted to provide the optimum desired response. For example, a single bolus may be administered, several divided doses may be administered over time or the dose may be proportionally reduced or increased as indicated by the exigencies of the therapeutic situation. It is especially advantageous to formulate parenteral compositions in a unit dosage form for ease of administration and uniformity of dosage. A unit dosage form as used herein refers to physically discrete units suited as unitary dosages for patients/subjects to be treated; each unit contains a predetermined quantity of active compound calculated to produce the desired therapeutic effect in association with the desired pharmaceutical carrier. Specification for the unit dosage forms of the present invention is typically dictated by and directly dependent on (a) the unique characteristics of a chemotherapeutic agent and particular therapeutic or prophylactic effect to be achieved, and (b) the limitations inherent in the art of compounding such an active compound for the treatment of sensitivity in the subjects.

Thus, a skilled artisan would appreciate, based upon the disclosure provided herein, that the doses and dosage regimen are adjusted in accordance with methods well-known in the therapeutic arts. That is, the maximum tolerable dose can be readily established, and the effective amount providing a detectable therapeutic effect to a patient may also be determined, as can the temporal requirements for administering each agent to provide a detectable therapeutic effect to a patient. Thus, while certain dose and administration regimens are exemplified herein, these examples in no way limit the doses and administration regimen that may be provided to a patient in practicing the embodiments of the invention.

It is to be noted that dosage values may vary with the type and severity of the condition to be alleviated, and may include single or multiple doses. Furthermore, it is to be understood that for any particular subject, specific dosage regimens should be adjusted over time according to the individual need and the judgment of a medical professional administering or supervising the administration of the compositions, and that dosage ranges set forth herein are exemplary only and are not intended to limit the scope or practice of the claimed compositions. Further, the dosage regimen with the compositions of this invention may be based on a variety of factors, including the type of disease, the age, weight, sex, medical condition of the patient, the severity of the condition, the route of administration, and the particular immunocytokine of the invention employed. Thus, the dosage regimen can vary widely, but can be determined routinely using standard methods. For example, doses may be adjusted based on pharmacokinetic or pharmacodynamic parameters, which may include clinical effects such as toxic effects and/or laboratory values. Thus, the present invention encompasses intra-patient dose-escalation as determined by the person skilled in the art. Methods for determining appropriate dosages and regimens are well-known in the art and would be understood by a skilled artisan once provided the ideas disclosed herein.

Examples of suitable administration methods are provided above.

It is contemplated that a suitable dose of an immunocytokine of the invention will be in the range of 0.1-200 mg/kg, preferably 0.1-100 mg/kg, including about 0.5-50 mg/kg, for example about 1-20 mg/kg. An immunocytokine may be administered, e.g. in a dose of at least 0.25 mg/kg, such as at least 0.5 mg/kg, including at least 1 mg/kg, e.g. at least 1.5 mg/kg, such as at least 2 mg/kg, e.g. at least 3 mg/kg, including at least 4 mg/kg, e.g. at least 5 mg/kg; and for example up to a maximum of 50 mg/kg, including up to a maximum of 30 mg/kg, e.g. up to a maximum of 20 mg/kg, including up to a maximum of 15 mg/kg. The administration will typically be repeated in appropriate time intervals, such as once a week, once every two weeks, once every three weeks or once every four weeks, and for as long as deemed appropriate by a responsible physician, who may, in some cases, increase or reduce the dose if necessary.

Examples

The following examples are provided for better understanding of the invention. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

Materials and general methods

Recombinant DNA techniques

DNA manipulations were carried out by standard techniques as described by Sambrook J. et al, Molecular cloning: A laboratory manual; Cold Spring Harbor Laboratory Press, Cold Spring Harbor, New York, 1989. The molecular biological reagents were used according to the manufacturer protocols.

Gene synthesis

Desired gene segments were prepared from oligonucleotides made by chemical synthesis. The gene segments of 300-4000 bp long, which were flanked by singular restriction sites, were assembled by annealing and ligation of oligonucleotides including PCR amplification and subsequently cloned via the specified restriction sites. The DNA sequences of the subcloned gene fragments were confirmed by DNA sequencing.

DNA sequence determination

DNA sequences were determined by Sanger sequencing.
DNA and protein sequence analysis and sequence data management
The Infomax's Vector NTI Advance suite version 8.0 and SnapGene Viewer was used for sequence creation, mapping, analysis, annotation and illustration.

Example 1

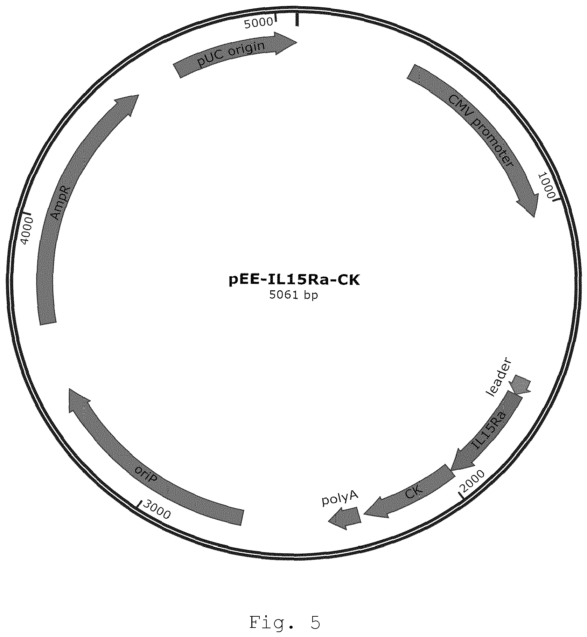

Production of recombinant proteins in suspension culture of mammalian cells
To produce the recombinant IL15 super-agonist proteins shown schematically in Fig. 1, Fig. 2, Fig. 3 and Fig. 4, we synthesized constructs comprising the fragments of the sequence of human IL15 ligand (https://www.uniprot.org/uniprot/P40933) and IL15Rα (https://www.uniprot.org/uniprot/Q13261). The sequences of the both genes were synthesized from oligonucleotides using PCR.
To produce IL15 super-agonist variants as represented by CK_IL15Ra_Hc_IL15CH1FcLALA in Fig. 1 and CK_IL15_Hc_IL15RaCH1FcLALA in Fig. 2 (hereinafter referred to as IL15SA or BCD225), said genes were cloned into plasmids as N-terminus fusion (fusion protein) with the first constant domain of kappa light chain of antibody into the vector pEE-IL15Ra-CK (or pEE-IL15-CK) at SalI/BsiWI restriction sites (Fig. 5) and with the first constant domain of antibody heavy chain into the vector pEE-IL15-CH1-Fc-LALA (or pEE-IL15Ra-CH1-Fc-LALA) IgG1 at SalI/NheI restriction sites (Fig. 6), and the both vectors were combined at the transfection step.
To produce bispecific IL15 super-agonist variants as represented by Fab-CK_IL15Ra_Hc_IL15CH1FcknobLALA (hereinafter referred to as IL15SA/PD1 or anti-PD1/IL15SA or IL15SA/PDL1 or anti-PDL1/IL15SA) in Fig. 4 and Fab-CK_IL15_Hc_IL15RaCH1FcknobLALA in Fig. 3, the IL15 ligand and receptor genes were cloned into plasmids as N-terminus fusion with the first constant domain of kappa light chain of antibody into the vector pEE-IL15Ra-CK (or pEE-IL15-CK) at SalI/BsiWI restriction sites (Fig. 5) and with the first constant domain of antibody heavy chain into the vector pEE-IL15-CH1-Fc-knob-LALA (or pEE-IL15Ra-CH1-Fc-knob-LALA) IgG1 at SalI/NheI restriction sites (Fig. 9). Furthermore, to form the Fab portion of an asymmetric antibody as shown in Fig. 3 and Fig. 4, the genes of the variable domains of anti-PD1 (prolgolimab) (hereinafter referred to as IL15SA/PD1 or anti-PD1/IL15SA) or anti-PDL1 (BCD-135) antibodies (hereinafter referred to as IL15SA/PDL1 or anti-PDL1/IL15SA) were cloned into plasmids as N-terminus fusion with the first constant domain of kappa light chain of antibody into the vector pEE-BCD100-02-VL-CK at the SalI/BsiWI restriction sites (Fig. 7) and with the first constant domain of antibody heavy chain into the vector pEE-BCD100-02-VH-CH1-Fc-hole-LALA IgG1 at SalI/NheI restriction sites (Fig. 8). As a result, the above four vectors were combined at the transfection step to synthesize 4 distinct polypeptides in one cell and form a bispecific immunocytokine.

The required quantities of all the above plasmids were produced in E.Coli cells and purified using Maxiprep Qiagen kit.

Recombinant proteins comprising IL15SA were transiently cultured in Chinese hamster ovary cell line (CHO-K1) produced according to published protocols [Biotechnol Bioeng. 2005 Sep 20; 91(6):670-677, Liao Metal., 2004; Biotechnol Lett. 2006 Jun;28(11):843-848; Biotechnol Bioeng. 2003 Nov 5;84(3):332-342]. Cells constitutively expressing the EBNA1 (Epstein-Barrvirus nuclear antigen 1) protein gene were used. Suspension culture was conducted in flasks on orbital shaker using serum-free media from Life Technologies Corporation and in accordance with manufacturer's instructions. For transient expression, cells at a concentration of $2*10^6$/ml were transfected by means of linear polyethyleneimine (PEI MAX, Polysciences). DNA/PEI ratio was 1:3-1:10. In 5-7 days after transfection, cell culture was centrifuged at 2000 g for 20 min and filtered through a 0.22 $\mu$m filter. Target proteins were isolated from culture liquid by affine HPLC.

Recombinant proteins were isolated and purified from culture liquid using a protein A affinity chromatography column. Cleared culture liquid was passed through a 5 ml HiTrap rProtein A Sepharose FF column (GE Healthcare) equilibrated with phosphate buffered saline (PBS, pH 7.4). The column was then washed with 5 column volumes of PBS to remove non-specifically binding components. Bound antigen was eluted using 0.1 M glycine buffer (pH 3). The principal protein elution peak was collected and adjusted to neutral pH with 1 M Tris buffer (pH 8). All stages were conducted under 110 cm/h flow rate. Protein was then dialyzed into PBS (pH 7.4) using SnakeSkin Dialysis Tubing technique, filtered (0.22 $\mu$m), transferred into tubes and stored at -70°C.

Purity of resulting protein solution was evaluated by SDS gel electrophoresis (see an example in Fig. 10).

Example 2

Kinetic assay of (IL15SA)2-Fc and human IL2$\beta\gamma$ receptor interactions

Affinity constants for binding of (IL15SA)2-Fc (hereinafter referred to as CK_IL15Ra_Hc_IL15CH1FcLALA in Fig. 1 and CK_IL15_Hc_IL15RaCH1FcLALA in Fig. 2) and human IL2$\beta\gamma$ were obtained using the OctetRedBio instrument according to the ForteBio instructions. AR2G biosensors were pre-rehydrated for an hour in mQ. After the activation of biosensors, the (IL15SA)2-Fc products at a concentration of 25 $\mu$g/ml in acetate buffer pH4 were non-specifically (by $NH_2$ groups) immobilized onto the biosensor. The sensors were then immersed into wells containing Human CD122/IL-2RB Protein (Fc Tag) solutions (1 and 0.5 $\mu$g/ml) at 30°C in PBS buffer supplemented with 0.1% Tween-20 and 0.1% BSA. The IL15SA/IL2$\beta\gamma$ complex was associated in this solution. The sensors were then immersed in a buffer solution for a subsequent dissociation step. The assay was conducted at 30°C using PBS supplemented with 0.1% Tween-20 and 0.1% BSA as a working buffer.

The resulting sensograms are given in Table 1 for CK_IL15Ra_Hc_IL15CH1FcLALA and for CK_IL15_Hc_IL15RaCH1FcLALA, which were analyzed after subtracting a reference signal using Octet Data Analysis software (Version 8.0) in accordance with the standard procedure and using 1:1 interaction model. The resulting affinity constants are shown in Table 1. The both variants studied showed high affinity and specificity for human IL2$\beta\gamma$.

Table 1. Affinity constants for (IL15SA)2-Fc immunocytokine interacting with human IL2$\beta\gamma$-Fc.

| Loading Sample ID | Cone. (nM) | Response | KD (M) | kon (1/Ms) | kdis (1/s) |
|---|---|---|---|---|---|
| CK_IL15Ra_Hc_IL15CH1FcLA LA | 19.4 | 0.3774 | 2.47E-10 | 3.69E+05 | 9.11E-05 |
| CK_IL15Ra_Hc_IL15CH1FcLA LA | 9.69 | 0.3301 | 3.80E-10 | 3.02E+05 | 1.15E-04 |
| CK_IL15_Hc_IL15RaCH1FcLA LA | 19.4 | 0.325 | 2.13E-10 | 3.58E+05 | 7.63E-05 |
| CK_IL15_Hc_IL15RaCH1FcLA LA | 9.69 | 0.2832 | 2.12E-10 | 3.57E+05 | 7.55E-05 |

Example 3

Kinetic assay of interactions between bispecific IL15 super-agonists and human IL2$\beta\gamma$ receptor and other cellular receptor of immune cells

The affinity constants for binding of IL15SA bispecific immunocytokines (hereinafter referred to as anti-PD1-Fab-CK_IL15Ra_Hc_IL15CH1FcknobLALA и anti-PD-Ll-Fab-CK_IL15Ra_Hc_IL15CH1FcknobLALA) to IL2$\beta\gamma$ and human PD-1/PD-L1 receptors were measured using OctetRed 96 according to the ForteBio instructions. AR2G biosensors were pre-rehydrated for an hour in mQ. After the activation of biosensors, the bispecific immunocytokines at a concentration of 25 $\mu$g/ml in acetate buffer pH4 were non-specifically (by $NH_2$ groups) immobilized onto the biosensor.

The sensors were then immersed into wells containing Human CD122/IL-2RB Protein (Fc Tag) or PD-1-Fc receptor or PD-L1-Fc receptor (1 and 0.5 µg/ml) in PBS buffer supplemented with 0.1% Tween-20 and 0.1% BSA to associate the bispecific super-agonist and the test receptor. The sensors were then immersed in a buffer solution for a subsequent dissociation step.

The instrumental data, after subtracting a reference signal, were analyzed using Octet Data Analysis software (Version 8.0) in accordance with the standard procedure and using 1:1 interaction model.

The resulting affinity constants are shown in Table 2. The both variants studied showed high affinity and specificity both for the human IL2βγ and for the paired receptors, and are deficient of nonspecific activity to the other receptor.

Table 2. Affinity constants for bispecific IL15 super-agonist antibodies interacting with human IL2βγ-Fc and the second receptor.

| Loading Sample ID | PD1 | | IL2Rb | | PDL1 | |
|---|---|---|---|---|---|---|
| | Response | KD (M) | Response | KD (M) | Response | KD (M) |
| Anti-PD-1 Fab-CK_IL15Ra_Hc_IL15CH1FcknobLALA | 0.7606 | 8.718E-10 | 0.4504 | 1.25E-10 | 0 | 0 |
| Anti-PD-L1 Fab - CK_IL15Ra_Hc_IL15CH1FcknobLALA | 0 | 0 | 0.3251 | 2.39E-11 | 0.3084 | 2.38E-11 |
| Anti-PD-1 prolgolimab | 0.9216 | 1.86E-10 | 0 | 0 | 0 | 0 |
| Anti-PDL-1 BCD-135 | 0 | 0 | 0 | 0 | 0.8183 | <1.0E-12 |

Example 4

Cellular assay for determination of proliferation activity of IL15 super-agonists on NK92 line

The NK-92 cell line was used in the assay. The assay was conducted in a 96-well culture plate. The suspension comprised NK-92 cells, a test antibody at a concentration as indicated in the graph. All suspension components were prepared in RPMI-1640 medium supplemented with fetal bovine serum and glutamine. After all components were added, the plates were incubated at 37°C, 5% $CO_2$. Alamar Blue was then added to the wells. Following incubation, the fluorescence intensity in the wells was measured. The IL15SA products (CK_IL15Ra_Hc_IL15CH1FcLALA), IL15SA/PD1 (Anti-PD-1 Fab-CK _IL15Ra_Hc _IL15CH1FcLALA) and IL15SA/PDL1 (Anti-PDL-1 Fab-CK_IL15Ra_Hc _IL15CH1FcLALA) were comparatively studied.

The results of determination of the specific activity of the above immunocytokines showed a high proliferative activity relative to the NK92 line. The estimated EC50s for all candidates were identical, and the level of activation as determined by the upper plateau was also completely the same (Fig. 11). It should be noted that the valence of the IL15SA portion, and, accordingly, a different avidity, did not significantly affect this activity.

Example 5

Cellular assay for determination of proliferation activity of IL15 super-agonists on natural killer line

The assay used isolated natural killer cells isolated from PBMCs of healthy donors by negative selection. The assay was conducted in a 96-well culture plate. The suspension comprised natural killer cells, a test antibody at a concentration as indicated in the graph. All suspension components were prepared in a culture medium supplemented with autologous human plasma. After all components were added, the plates were incubated at 37°C, 5% $CO_2$. Alamar Blue was then added to the wells. Following incubation, the fluorescence intensity in the wells was measured. The effect of IL15SA products (CK_IL15Ra_Hc_IL15CH1FcLALA), IL15SA/PD1 (Anti-PD-1 Fab-CK_IL15Ra_Hc _IL15CH1FcknobLALA) and human interleukin-2 (IL2) (Ronleukin, PCI Biotech) on the proliferation ability of natural killer cells was comparatively studied.

The results of determination of the specific activity of the above immunocytokines showed a high proliferative activity relative to the natural killer cells (Fig. 12). The estimated EC50s for all candidates were identical within the margin of error, and slightly lower than that for IL2 cytokine. Furthermore, the level of activation as defined by the upper plateau was also completely the same. It should be noted that the valence of the IL15SA portion, and, accordingly, a different avidity, did not significantly affect this activity.

Example 6

Cellular assay for determination of anti-PD1 antagonistic activity of IL15 super-agonists on reporter line

For the assay, we used the Jurkat NFAT-FLuc PD-1 cell line generated based on the Jurkat cell line and stably expressing PD-1 on the surface thereof and containing the firefly luciferase encoding gene under control of NFAT promoter; and the Raji PDL1 cell line generated based on the Raji cell line and stably expressing PDL1 on the surface thereof. The effect of IL15SA products (CK_IL15Ra_Hc_IL15CH1FcLALA), anti-PD1/IL15SA (Anti-PD-1 Fab-CK_IL15Ra_Hc _IL15CH1FcknobLALA) and anti-PD1 antagonist (prolgolimab) on the activation ability in the reporter cellular system was comparatively studied.

The assay was conducted in a 96-well culture plate. The suspension in each well contained Jurkat NFAT-FLuc PD-1 cells, Raji PDL1 cells, aCD3/aTAA1 cells at a concentration of 1 ng/ml, and a test antibody at a concentration as indicated in the graph. After adding all the components, the plates were incubated at 37°C, 5%$CO_2$ and then, using a luminescence assay kit, the luciferase intensity in the wells was measured.

The results of determination of the specific activity of the above immunocytokines showed an order of magnitude lower specific activity EC50 value for anti-PD1/IL15SA as compared to the therapeutically effective anti-PD1 monoclonal antibody prolgolimab (Fig. 13). In addition, the level of maximum activity (upper plateau) decreased by 30%. The lower specific activity of anti-PD1/IL15SA as compared to prolgolimab can only be explained by the monovalent Fab binding PD1 within the anti-PD1/IL15SA molecule as compared to the classical antibody. This is also supported by an almost order of magnitude difference in affinity for the PD-1 receptor according to the data in Example 3 (Table 2) and the value as specified for prolgolimab. It should be noted that the antagonistic activity of IL15SA was observed as background, i.e. it was substantially absent.

Example 7

Cellular assay for determination of anti-PDL1 antagonistic activity of IL15 super-agonists on reporter line

For the assay, we used the Jurkat NFAT-FLuc PD-1 cell line generated based on the Jurkat cell line and stably expressing PD-1 on the surface thereof and containing the firefly luciferase encoding gene under control of NFAT promoter; and the Raji PDL1 cell line generated based on the Raji cell line and stably expressing PDL1 on the surface thereof. The effect of IL15SA products (CK_IL15Ra_Hc_IL15CH1FcLALA), anti-PDL1/IL15SA (Anti-PD-L1 Fab-CK_IL15Ra_Hc _IL15CH1FcknobLALA) and anti-PDL1 antagonist (atezolizumab) on the activation ability in the reporter cellular system was comparatively studied.

The assay was conducted in a 96-well culture plate. The suspension in each well contained Jurkat NFAT-FLuc PD-1 cells, Raji PDL1 cells, aCD3/aTAA1 cells at a concentration of 1 ng/ml, and a test antibody at a concentration as indicated in the graph. After adding all the components, the plates were incubated at 37°C, 5%$CO_2$ and then, using a luminescence assay kit, the luciferase intensity in the wells was measured.

The results of determination of the specific activity of the above immunocytokines showed 30X lower specific activity EC50 value for anti-PDL1/IL15SA as compared to that of the therapeutically effective anti-PDL1 monoclonal antibody atezolizumab (Fig. 14). However, the level of maximum activity (upper plateau) decreased only by 10%. The lower specific activity of anti-PDL1/IL15SA as compared to that of atezolizumab can only be explained by the monovalent Fab binding PD-L1 within the anti-PDL1/IL15SA molecule as compared to the classical antibody. Thereby, no significant antagonistic activity of IL15SA was observed.

Example 8

Cellular assay for determination of effect of IL15 super-agonists in the presence of antibody specific for tumor-associated antigen (TAA) on antibody-dependent cellular cytotoxicity (ADCC) of natural killer cells against the TAA-reporter line

The assay used natural killer cells isolated from PBMCs of healthy donors by negative selection. The assay was conducted in a 96-well culture plate. The suspension contained natural killer cells and Raji cells, the effector antibody Rituximab, and a test antibody at a concentration as indicated in the graph. All suspension components were prepared in a culture medium supplemented with fetal bovine serum and glutamine. After all components were added, the plates were incubated at 37°C, 5% $CO_2$. The culture liquid was then collected, and lactate dehydrogenase content was measured using an LDH assay kit. The effect of IL15SA products (CK_IL15Ra_Hc_IL15CH1FcLALA), anti-PD1/IL15SA (Anti-PD-1 Fab-CK _IL15Ra_Hc IL15CH1FcknobLALA) on the ADCC ability of rituximab in the reporter cellular system was comparatively studied.

The results of determination of specific activity of the above immunocytokines showed a high activity relative to enhancing the ADCC ability of rituximab against the TAA-reporter line (Fig. 15 and Fig. 16). The efficacy of rituximab-dependent ADCC, as measured by the percentage of lysed Raji cells with an overexpressed CD20 receptor on the surface thereof, increased by 30% in the presence of each candidate studied as compared to rituximab alone. This effect seems to be explained by the proliferation of active natural killer cells under the influence of IL15SA and anti-PD1/IL15SA in the assay, according to the results given in Examples 4 and 5. The estimated EC50s for all candidates were identical within the margin of error. It should be noted that the valence of the IL15SA portion, and, accordingly, a different avidity, did not significantly affect this activity, which fact is further supported by Examples 4 and 5.

Example 9

Cellular assay for determination of cytotoxicity of IL15 super-agonists on PBMCs from whole blood of healthy donors

PBMCs were isolated from whole blood from healthy donors by Ficoll density gradient centrifugation.

The assay was conducted in a 96-well culture plate. The suspension contained (per well) freshly isolated PBMCs and antibody as indicated in the graph at a concentration of 0.1 $\mu$g/ml. After mixing PBMCs and antibodies, the plate was incubated for 16 h at 37°C, 5% $CO_2$. The proportion of CD56+, CD19+, CD3+, CD4+ and CD8+ subpopulations of PBMCs in suspensions was then measured by directly staining the suspensions with fluorescent-labeled antibodies against the corresponding CDs and further analyzing the cells using a flow cytofluorometer. For the CD56+, CD19+, CD3+ cells, the graphs show the proportion thereof relative to all cells of test suspension, whereas for the CD4+, CD8+ cells, the graphs show the proportion thereof relative to CD3+ cells. The cytotoxic effect of IL15SA products (CK_IL15Ra_Hc_IL15CH1FcLALA), anti-PD1/IL15SA (Anti-PD-1 Fab-CK_IL15Ra_Hc _IL15CH1FcknobLALA), anti-PD1 (prolgolimab), a mixture of anti-PD1 (prolgolimab) + IL15SA, anti-CD20 GA101 antibody on human PBMCs was comparatively studied.

The results as given in Figs. 17-21 demonstrate no significant cytotoxic effect of the candidates, i.e. no more than 10% decrease in the number of immunoreactive cells as compared to the AB negative control (i.e. absence of any exogenous antibodies). Thereby, the control anti-CD20 antibody showed a consistently complete depletion of the CD20+ B cell population. Thus, all candidates in question do not exhibit any significant non-specific in vitro cytotoxicity toward human blood cells.

Example 10

Determination of aggregative stability of IL15 super-agonist under thermostress conditions

The IL15 super-agonist product at a concentration of 9 mg/ml in PBS buffer was heated for 12 hours at a temperature of 50°C. Aggregation after thermal stress was determined by high-performance gel-filtration chromatography. Chromatography was performed on a HPLC system (Agilent) on the Tosoh TSK-Gel G3000SWXL column, 7.8 mm x 30 cm, order no. 08541 with the Tosoh TSKgel Guard SWXL precolumn, 6.0 mm x 4.0 cm, with a particle diameter of 7 $\mu$m, order no. 08543. Elution was performed in isocratic mode, mobile phase: 50 mM NaFb, 0.3 M NaCl, pH 7.0 at a flow rate of 0.5 ml/min. Detection was performed at wavelengths of 214 and 280 nm. The antibody samples were diluted with PBS buffer, pH 7.5, to a concentration of ~1 mg/ml. Injection volume was 10 microliters. The Gel filtration standard calibration mixture (Bio-Rad), order. no 151- 1901, was pre-chromatographed. Fig. 22 shows a chromatogram, in which the IL15 super-agonist demonstrates high homogeneity (the aggregate content in the solution was no more than 5%).

Example 11

Assessment of anti-tumor activity of IL15 super-agonist products in syngeneic B16F10 melanoma model

Efficacy was assessed using immunocompetent C57BL/6 mice inoculated with a B16F10 tumor line. $1{,}5{\times}10^5$ tumor cells were injected subcutaneously into the right flank of each animal in the group. On day 7 after cell inoculation (day 1 of the experiment), the animals were divided in groups as shown in Table 3. Efficacy was evaluated using one dose of the following products: BCD-225 , IL15SA/a-mPD-1 (a bispecific monoclonal antibody comprising IL-15SA and Fab fragment to mouse PD-1, a derivative of the mouse RMP1-14 antibody). The sequence of the variable domains of the RMP1-14 antibody was kindly provided by Hideo Yagita, PhD, Juntendo University School of Medicine. Genes and RMP1-14 antibody products variable domains were synthesized according to Example 1. Furthermore, we synthesized a reference product ALT-803 [Han KP et al, Cytokine. 2011 Dec;56(3):804-10.] (positive control) and placebo (negative control). The anti-tumor activity of the products in combination with the antimouse PD-1 antibody (a-mPD-1) was also assessed.

Table 3. Groups of animals in the study of anti-tumor activity of IL15 super-agonists.

| Group | Animal qty | Dose |
|---|---|---|
| BCD-225(IL15SA) | 9 | 0.2 mg/kg |
| IL15SA/a-mPD-1 | 9 | 0.2 mg/kg |
| ALT-803 | 9 | 0.2 mg/kg |
| anti-mPD-1 | 10 | 10 mg/kg |
| BCD-225 (IL15SA)+anti-mPD-1 | 9 | 0.2 mg/kg + 10 mg/kg |
| IL15SA/a-mPD-I+anti-mPD-1 | 9 | 0.2 mg/kg + 10 mg/kg |
| ALT-803 + anti-mPD-1 | 9 | 0.2 mg/kg + 10 mg/kg |
| Negative control | 10 | - |

The BCD-225 (IL15SA), IL15SA/a-mPD-1 and ALT-803 products were injected intraperitoneally in a volume of 0.2 ml on days 1, 4, 8, and 11 of the experiment. The anti-mPD-1 product was injected intraperitoneally in a volume of 0.2 ml on days 2, 5, 9, and 12 of the experiment. The negative control group was injected with sodium acetate buffer on days 1, 2, 4, 5, 8, 9, 11 and 12 of the experiment in a volume of 0.2 ml (Figure 23).

Mice body weight and tumor linear dimensions were measured throughout the experiment. The tumor volume was calculated using the following formula: $V = L \times W \times W \times \pi/6$. The efficacy of test product was assessed by the index of tumor growth inhibition (TGI) that was calculated taking into account the median tumor volume in the negative control group ($V_c$) and the group of the product (Vt) using the following formula:

$$\text{TGI } (\%) = \frac{V_c - V_t}{V_c} * 100$$

The results of the experiment are shown in Figs. 24 and 25. Provided are data as of day 11 of the experiment as animals in almost all experimental groups died from tumor burden making it impossible to reliably assess the anti-tumor activity of the products on day 15.

According to the results of the study, the BCD-225 products and the reference ALT-803 product showed comparable anti-tumor activity with TGI being 52% in the BCD-225 group, and 53% in the ALT-803 group. We observed an increasing tendency in the TGI value in the IL15SA/anti-mPD-1 bispecific antibody group as compared to the TGI values in the BCD-225 and ALT-803 groups. TGI in the IL15SA/anti-mPD-1 group was 58% on day 11 of the experiment.

The TGI values for the groups of product combinations BCD-225 + anti-mPD-1 and ALT-803 + anti-mPD-1 were 72% on day 11 of the experiment, and for the group of product combination IL15SA/a-mPD-1 and anti-mPD-1, the TGI value was 69%. The resulting TGI values indicate a more pronounced anti-tumor activity of the combination of the test products and anti-mPD-1, as compared to the activity of these products in monotherapy.

Example 12

Assessment and analysis of comparative mortality in mice with IL15 super-agonist and ALT803 products in syngeneic B16F10 melanoma model

The comparative assessment of the mortality in mice following the use of IL15 super-agonist products was performed using the model and animals as described in Example 11. The survival was assessed by counting dead or euthanized animals in a state of agony.

The results are shown in Table 4. As it follows from the resulting data, the mortality rates in the IL15SA/a-mPD-1 and IL15SA/a-mPD-1 + anti-mPD-1 product groups are, on average, less in terms of the number of mice and the duration of survival, as compared to those of IL15SA (BCD225), IL15SA + anti-mPD-1, ALT-803, ALT-803 + anti-mPD-1. The resulting mortality values show probably less pronounced toxicity of the IL15SA/a-mPD-1 bispecific immunocytokine as compared to the monospecific bivalent IL15SA and ALT-803 ones. The presence or absence of the anti-PD1 antibody did not have a statistically significant effect on mortality. Furthermore, the examination of the organs of a dead mouse from the IL15SA and ALT-803 batch revealed the presence in the liver of hydropic dystrophy with necrosis of hepatocytes, compression of the sinusoids; lymphohistiocytic infiltrate in the parenchyma, and accumulation of lymphocytes in the sinusoids. These findings are consistent with previously reported literature data on the capability of IL15-based super-agonists to cause liver damage in mice.

Table 4. Groups of animals in the study of mortality following IL15 super-agonist and ALT803 super-agonist products and combinations thereof with anti-PD1 antibody

| Group No. | Treatment | Number of dead animals | Day of experiment | Total |
|---|---|---|---|---|
| 1 | IL15SA (BCD-225) | 2<br>2 | 10<br>14 | 4 |
| 2 | IL15SA/a-mPD-1 | 2 | 15 | 2 |
| 3 | ALT-803 | 2 | 7 | 2 |
| 4 | anti-mPD-1 | 3 | 14 | 3 |
| 5 | IL15SA(BCD-225)+anti-mPD-1 | 1 | 14 | 1 |
| 6 | IL15SA/a-mPD-I+anti-mPD-1 | 1<br>1 | 11<br>14 | 2 |
| 7 | ALT-803 + anti-mPD-1 | 3 | 7 | 3 |
| 8 | Placebo | 1 | 14 | 1 |

SEQUENCE LISTING

<110> JOINT STOCK COMPANY "BIOCAD"

<120> IMMUNOCYTOKINE COMPRISING HETERODYMERIC PROTEIN COMPLEX BASED ON
      IL-15/IL-15RA

<150> RU2019129569
<151> 2019-09-19

<160> 24

<170> BiSSAP 1.3.6

<210> 1
<211> 216
<212> PRT
<213> Artificial Sequence

<220>
<223> IL15Rα-CK artificial sequence

<400> 1
Asp Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Ser Ser Leu Thr Glu Cys Val Leu Asn
            35                  40                  45
Lys Ala Thr Asn Val Ala His Trp Thr Thr Pro Ser Leu Lys Cys Ile
    50                  55                  60
Arg Asp Pro Ala Leu Val His Gln Arg Pro Ala Pro Pro Ser Thr Val
65                  70                  75                  80
Thr Thr Ala Gly Val Thr Pro Gln Pro Glu Ser Leu Ser Pro Ser Gly
                85                  90                  95
Lys Glu Pro Ala Ala Ser Ser Gly Ala Glu Gly Gly Gly Arg Thr Val
                100                 105                 110
Ala Ala Pro Ser Val Phe Ile Phe Pro Pro Ser Asp Glu Gln Leu Lys
            115                 120                 125
Ser Gly Thr Ala Ser Val Val Cys Leu Leu Asn Asn Phe Tyr Pro Arg
            130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
            195                 200                 205
Lys Ser Phe Asn Arg Gly Glu Cys
    210                 215

<210> 2
<211> 454

```
<212> PRT
<213> Artificial Sequence

<220>
<223> IL15-CH1CH2CH3 artificial sequence


<400> 2
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
                20                  25                  30
His Pro Ser Cys Lys Val Thr Ala Met Lys Cys Phe Leu Leu Glu Leu
            35                  40                  45
Gln Val Ile Ser Leu Glu Ser Gly Asp Ala Ser Ile His Asp Thr Val
        50                  55                  60
Glu Asn Leu Ile Ile Leu Ala Asn Asp Ser Leu Ser Ser Asn Gly Asn
65                  70                  75                  80
Val Thr Glu Ser Gly Cys Lys Glu Cys Glu Glu Leu Glu Glu Lys Asn
                85                  90                  95
Ile Lys Glu Phe Leu Gln Ser Phe Val His Ile Val Gln Met Phe Ile
                100                 105                 110
Asn Thr Ser Ala Gly Gly Lys Pro Gly Gly Ser Ala Gly Gly Ala Ser
            115                 120                 125
Thr Lys Gly Pro Ser Val Phe Pro Leu Ala Pro Ser Ser Lys Ser Thr
        130                 135                 140
Ser Gly Gly Thr Ala Ala Leu Gly Cys Leu Val Lys Asp Tyr Phe Pro
145                 150                 155                 160
Glu Pro Val Thr Val Ser Trp Asn Ser Gly Ala Leu Thr Ser Gly Val
                165                 170                 175
His Thr Phe Pro Ala Val Leu Gln Ser Ser Gly Leu Tyr Ser Leu Ser
            180                 185                 190
Ser Val Val Thr Val Pro Ser Ser Ser Leu Gly Thr Gln Thr Tyr Ile
        195                 200                 205
Cys Asn Val Asn His Lys Pro Ser Asn Thr Lys Val Asp Lys Arg Val
        210                 215                 220
Glu Pro Lys Ser Cys Asp Lys Thr His Thr Cys Pro Pro Cys Pro Ala
225                 230                 235                 240
Pro Glu Ala Ala Gly Gly Pro Ser Val Phe Leu Phe Pro Pro Lys Pro
                245                 250                 255
Lys Asp Thr Leu Met Ile Ser Arg Thr Pro Glu Val Thr Cys Val Val
                260                 265                 270
Val Asp Val Ser His Glu Asp Pro Glu Val Lys Phe Asn Trp Tyr Val
        275                 280                 285
Asp Gly Val Glu Val His Asn Ala Lys Thr Lys Pro Arg Glu Glu Gln
        290                 295                 300
Tyr Asn Ser Thr Tyr Arg Val Val Ser Val Leu Thr Val Leu His Gln
305                 310                 315                 320
Asp Trp Leu Asn Gly Lys Glu Tyr Lys Cys Lys Val Ser Asn Lys Ala
                325                 330                 335
Leu Pro Ala Pro Ile Glu Lys Thr Ile Ser Lys Ala Lys Gly Gln Pro
                340                 345                 350
Arg Glu Pro Gln Val Tyr Thr Leu Pro Pro Ser Arg Asp Glu Leu Thr
                355                 360                 365
```

```
Lys Asn Gln Val Ser Leu Thr Cys Leu Val Lys Gly Phe Tyr Pro Ser
    370                 375                 380
Asp Ile Ala Val Glu Trp Glu Ser Asn Gly Gln Pro Glu Asn Asn Tyr
    385                 390                 395                 400
Lys Thr Thr Pro Pro Val Leu Asp Ser Asp Gly Ser Phe Phe Leu Tyr
            405                 410                 415
Ser Lys Leu Thr Val Asp Lys Ser Arg Trp Gln Gln Gly Asn Val Phe
            420                 425                 430
Ser Cys Ser Val Met His Glu Ala Leu His Asn His Tyr Thr Gln Lys
            435                 440                 445
Ser Leu Ser Leu Ser Pro
    450
```

<210> 3
<211> 233
<212> PRT
<213> Artificial Sequence

<220>
<223> IL15-CK artificial sequence

<400> 3
```
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
            20                  25                  30
His Pro Ser Cys Lys Val Thr Ala Met Lys Cys Phe Leu Leu Glu Leu
            35                  40                  45
Gln Val Ile Ser Leu Glu Ser Gly Asp Ala Ser Ile His Asp Thr Val
    50                  55                  60
Glu Asn Leu Ile Ile Leu Ala Asn Asp Ser Leu Ser Ser Asn Gly Asn
65                  70                  75                  80
Val Thr Glu Ser Gly Cys Lys Glu Cys Glu Glu Leu Glu Glu Lys Asn
                85                  90                  95
Ile Lys Glu Phe Leu Gln Ser Phe Val His Ile Val Gln Met Phe Ile
            100                 105                 110
Asn Thr Ser Ala Gly Gly Lys Pro Gly Gly Ser Ala Gly Gly Arg Thr
            115                 120                 125
Val Ala Ala Pro Ser Val Phe Ile Phe Pro Pro Ser Asp Glu Gln Leu
    130                 135                 140
Lys Ser Gly Thr Ala Ser Val Val Cys Leu Leu Asn Asn Phe Tyr Pro
145                 150                 155                 160
Arg Glu Ala Lys Val Gln Trp Lys Val Asp Asn Ala Leu Gln Ser Gly
                165                 170                 175
Asn Ser Gln Glu Ser Val Thr Glu Gln Asp Ser Lys Asp Ser Thr Tyr
            180                 185                 190
Ser Leu Ser Ser Thr Leu Thr Leu Ser Lys Ala Asp Tyr Glu Lys His
            195                 200                 205
Lys Val Tyr Ala Cys Glu Val Thr His Gln Gly Leu Ser Ser Pro Val
    210                 215                 220
Thr Lys Ser Phe Asn Arg Gly Glu Cys
225                 230
```

<210> 4
```

<211> 436
<212> PRT
<213> Artificial Sequence

<220>
<223> IL15Rα-CH1CH2CH3 artificial sequence

<400> 4
Asp Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Ser Ser Leu Thr Glu Cys Val Leu Asn
        35                  40                  45
Lys Ala Thr Asn Val Ala His Trp Thr Thr Pro Ser Leu Lys Cys Ile
    50                  55                  60
Arg Asp Pro Ala Leu Val His Gln Arg Pro Ala Pro Pro Ser Thr Val
65                  70                  75                  80
Thr Thr Ala Gly Val Thr Pro Gln Pro Glu Ser Leu Ser Pro Ser Gly
                85                  90                  95
Lys Glu Pro Ala Ala Ser Ser Gly Ala Glu Gly Gly Gly Ala Ser Thr
                100                 105                 110
Lys Gly Pro Ser Val Phe Pro Leu Ala Pro Ser Ser Lys Ser Thr Ser
            115                 120                 125
Gly Gly Thr Ala Ala Leu Gly Cys Leu Val Lys Asp Tyr Phe Pro Glu
        130                 135                 140
Pro Val Thr Val Ser Trp Asn Ser Gly Ala Leu Thr Ser Gly Val His
145                 150                 155                 160
Thr Phe Pro Ala Val Leu Gln Ser Ser Gly Leu Tyr Ser Leu Ser Ser
                165                 170                 175
Val Val Thr Val Pro Ser Ser Ser Leu Gly Thr Gln Thr Tyr Ile Cys
            180                 185                 190
Asn Val Asn His Lys Pro Ser Asn Thr Lys Val Asp Lys Arg Val Glu
        195                 200                 205
Pro Lys Ser Cys Asp Lys Thr His Thr Cys Pro Pro Cys Pro Ala Pro
    210                 215                 220
Glu Ala Ala Gly Gly Pro Ser Val Phe Leu Phe Pro Pro Lys Pro Lys
225                 230                 235                 240
Asp Thr Leu Met Ile Ser Arg Thr Pro Glu Val Thr Cys Val Val Val
                245                 250                 255
Asp Val Ser His Glu Asp Pro Glu Val Lys Phe Asn Trp Tyr Val Asp
            260                 265                 270
Gly Val Glu Val His Asn Ala Lys Thr Lys Pro Arg Glu Glu Gln Tyr
        275                 280                 285
Asn Ser Thr Tyr Arg Val Val Ser Val Leu Thr Val Leu His Gln Asp
        290                 295                 300
Trp Leu Asn Gly Lys Glu Tyr Lys Cys Lys Val Ser Asn Lys Ala Leu
305                 310                 315                 320
Pro Ala Pro Ile Glu Lys Thr Ile Ser Lys Ala Lys Gly Gln Pro Arg
                325                 330                 335
Glu Pro Gln Val Tyr Thr Leu Pro Pro Ser Arg Asp Glu Leu Thr Asn
            340                 345                 350
Gln Val Ser Leu Thr Cys Leu Val Lys Gly Phe Tyr Pro Ser Asp Ile
            355                 360                 365

```
Ala Val Glu Trp Glu Ser Asn Gly Gln Pro Glu Asn Asn Tyr Lys Thr
370                 375                 380
Thr Pro Pro Val Leu Asp Ser Asp Gly Ser Phe Phe Leu Tyr Ser Lys
385                 390                 395                 400
Leu Thr Val Asp Lys Ser Arg Trp Gln Gln Gly Asn Val Phe Ser Cys
                405                 410                 415
Ser Val Met His Glu Ala Leu His Asn His Tyr Thr Gln Lys Ser Leu
                420                 425                 430
Ser Leu Ser Pro
            435


<210> 5
<211> 453
<212> PRT
<213> Artificial Sequence

<220>
<223> IL15-CH1CH2CH3 knob-Fc artificial sequence

<400> 5
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
                20                  25                  30
His Pro Ser Cys Lys Val Thr Ala Met Lys Cys Phe Leu Leu Glu Leu
            35                  40                  45
Gln Val Ile Ser Leu Glu Ser Gly Asp Ala Ser Ile His Asp Thr Val
        50                  55                  60
Glu Asn Leu Ile Ile Leu Ala Asn Asp Ser Leu Ser Ser Asn Gly Asn
65                  70                  75                  80
Val Thr Glu Ser Gly Cys Lys Glu Cys Glu Glu Leu Glu Glu Lys Asn
                85                  90                  95
Ile Lys Glu Phe Leu Gln Ser Phe Val His Ile Val Gln Met Phe Ile
                100                 105                 110
Asn Thr Ser Ala Gly Gly Lys Pro Gly Gly Ser Ala Gly Gly Ala Ser
            115                 120                 125
Thr Lys Gly Pro Ser Val Phe Pro Leu Ala Pro Ser Ser Lys Ser Thr
        130                 135                 140
Ser Gly Gly Thr Ala Ala Leu Gly Cys Leu Val Lys Asp Tyr Phe Pro
145                 150                 155                 160
Glu Pro Val Thr Val Ser Trp Asn Ser Gly Ala Leu Thr Ser Gly Val
                165                 170                 175
His Thr Phe Pro Ala Val Leu Gln Ser Ser Gly Leu Tyr Ser Leu Ser
            180                 185                 190
Ser Val Val Thr Val Pro Ser Ser Ser Leu Gly Thr Gln Thr Tyr Ile
            195                 200                 205
Cys Asn Val Asn His Lys Pro Ser Asn Thr Lys Val Asp Lys Arg Val
    210                 215                 220
Glu Pro Lys Ser Cys Asp Lys Thr His Thr Cys Pro Pro Cys Pro Ala
225                 230                 235                 240
Pro Glu Ala Ala Gly Gly Pro Ser Val Phe Leu Phe Pro Pro Lys Pro
                245                 250                 255
Lys Asp Thr Leu Met Ile Ser Arg Thr Pro Glu Val Thr Cys Val Val
                260                 265                 270
```

Val Asp Val Ser His Glu Asp Pro Glu Val Lys Phe Asn Trp Tyr Val
275 280 285

Asp Gly Val Glu Val His Asn Ala Lys Thr Lys Pro Arg Glu Glu Gln
290 295 300

Tyr Asn Ser Thr Tyr Arg Val Val Ser Val Leu Thr Val Leu His Gln
305 310 315 320

Asp Trp Leu Asn Gly Lys Glu Tyr Lys Cys Lys Val Ser Asn Lys Ala
325 330 335

Leu Pro Ala Pro Ile Glu Lys Thr Ile Ser Lys Ala Lys Gly Gln Pro
340 345 350

Arg Glu Pro Gln Val Tyr Thr Leu Pro Pro Arg Asp Glu Leu Thr Lys
355 360 365

Asn Gln Val Ser Leu Trp Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Lys Leu Thr Val Asp Lys Ser Arg Trp Gln Gln Gly Asn Val Phe Ser
420 425 430

Cys Ser Val Met His Glu Ala Leu His Asn His Tyr Thr Gln Lys Ser
435 440 445

Leu Ser Leu Ser Pro
450


<210> 6
<211> 436
<212> PRT
<213> Artificial Sequence

<220>
<223> IL15Rα-CH1CH2CH3 knob-Fc artificial sequence

<400> 6
Asp Thr Cys Pro Pro Pro Met Ser Val Glu His Ala Asp Ile Trp Val
1 5 10 15

Lys Ser Tyr Ser Leu Tyr Ser Arg Glu Arg Tyr Ile Cys Asn Ser Gly
20 25 30

Phe Lys Arg Lys Ala Gly Thr Ser Ser Leu Thr Glu Cys Val Leu Asn
35 40 45

Lys Ala Thr Asn Val Ala His Trp Thr Thr Pro Ser Leu Lys Cys Ile
50 55 60

Arg Asp Pro Ala Leu Val His Gln Arg Pro Ala Pro Pro Ser Thr Val
65 70 75 80

Thr Thr Ala Gly Val Thr Pro Gln Pro Glu Ser Leu Ser Pro Ser Gly
85 90 95

Lys Glu Pro Ala Ala Ser Ser Gly Ala Glu Gly Gly Gly Ala Ser Thr
100 105 110

Lys Gly Pro Ser Val Phe Pro Leu Ala Pro Ser Ser Lys Ser Thr Ser
115 120 125

Gly Gly Thr Ala Ala Leu Gly Cys Leu Val Lys Asp Tyr Phe Pro Glu
130 135 140

Pro Val Thr Val Ser Trp Asn Ser Gly Ala Leu Thr Ser Gly Val His
145 150 155 160

```
Thr Phe Pro Ala Val Leu Gln Ser Ser Gly Leu Tyr Ser Leu Ser Ser
            165                 170                 175
Val Val Thr Val Pro Ser Ser Ser Leu Gly Thr Gln Thr Tyr Ile Cys
            180                 185                 190
Asn Val Asn His Lys Pro Ser Asn Thr Lys Val Asp Lys Arg Val Glu
            195                 200                 205
Pro Lys Ser Cys Asp Lys Thr His Thr Cys Pro Pro Cys Pro Ala Pro
    210                 215                 220
Glu Ala Ala Gly Gly Pro Ser Val Phe Leu Phe Pro Pro Lys Pro Lys
225                 230                 235                 240
Asp Thr Leu Met Ile Ser Arg Thr Pro Glu Val Thr Cys Val Val Val
            245                 250                 255
Asp Val Ser His Glu Asp Pro Glu Val Lys Phe Asn Trp Tyr Val Asp
            260                 265                 270
Gly Val Glu Val His Asn Ala Lys Thr Lys Pro Arg Glu Glu Gln Tyr
            275                 280                 285
Asn Ser Thr Tyr Arg Val Val Ser Val Leu Thr Val Leu His Gln Asp
            290                 295                 300
Trp Leu Asn Gly Lys Glu Tyr Lys Cys Lys Val Ser Asn Lys Ala Leu
305                 310                 315                 320
Pro Ala Pro Ile Glu Lys Thr Ile Ser Lys Ala Lys Gly Gln Pro Arg
            325                 330                 335
Glu Pro Gln Val Tyr Thr Leu Pro Pro Arg Asp Glu Leu Thr Lys Asn
            340                 345                 350
Gln Val Ser Leu Trp Cys Leu Val Lys Gly Phe Tyr Pro Ser Asp Ile
            355                 360                 365
Ala Val Glu Trp Glu Ser Asn Gly Gln Pro Glu Asn Asn Tyr Lys Thr
            370                 375                 380
Thr Pro Pro Val Leu Asp Ser Asp Gly Ser Phe Phe Leu Tyr Ser Lys
385                 390                 395                 400
Leu Thr Val Asp Lys Ser Arg Trp Gln Gln Gly Asn Val Phe Ser Cys
            405                 410                 415
Ser Val Met His Glu Ala Leu His Asn His Tyr Thr Gln Lys Ser Leu
            420                 425                 430
Ser Leu Ser Pro
            435
```

```
<210> 7
<211> 457
<212> PRT
<213> Artificial Sequence

<220>
<223> anti-PD1 VH-CH1CH2CH3 hole-Fc artificial sequence

<400> 7
Gln Val Gln Leu Val Gln Ser Gly Gly Gly Leu Val Gln Pro Gly Gly
1                   5                   10                  15
Ser Leu Arg Leu Ser Cys Ala Ala Ser Gly Phe Thr Phe Ser Ser Tyr
            20                  25                  30
Trp Met Tyr Trp Val Arg Gln Val Pro Gly Lys Gly Leu Glu Trp Val
            35                  40                  45
Ser Ala Ile Asp Thr Gly Gly Gly Arg Thr Tyr Tyr Ala Asp Ser Val
            50                  55                  60
```

```
Lys Gly Arg Phe Ala Ile Ser Arg Val Asn Ala Lys Asn Thr Met Tyr
65              70              75                          80
Leu Gln Met Asn Ser Leu Arg Ala Glu Asp Thr Ala Val Tyr Tyr Cys
                85              90                          95
Ala Arg Asp Glu Gly Gly Gly Thr Gly Trp Gly Val Leu Lys Asp Trp
            100             105             110
Pro Tyr Gly Leu Asp Ala Trp Gly Gln Gly Thr Leu Val Thr Val Ser
            115             120             125
Ser Ala Ser Thr Lys Gly Pro Ser Val Phe Pro Leu Ala Pro Ser Ser
    130             135             140
Lys Ser Thr Ser Gly Gly Thr Ala Ala Leu Gly Cys Leu Val Lys Asp
145             150             155             160
Tyr Phe Pro Glu Pro Val Thr Val Ser Trp Asn Ser Gly Ala Leu Thr
            165             170             175
Ser Gly Val His Thr Phe Pro Ala Val Leu Gln Ser Ser Gly Leu Tyr
            180             185             190
Ser Leu Ser Ser Val Val Thr Val Pro Ser Ser Ser Leu Gly Thr Gln
    195             200             205
Thr Tyr Ile Cys Asn Val Asn His Lys Pro Ser Asn Thr Lys Val Asp
    210             215             220
Lys Arg Val Glu Pro Lys Ser Cys Asp Lys Thr His Thr Cys Pro Pro
225             230             235             240
Cys Pro Ala Pro Glu Ala Ala Gly Gly Pro Ser Val Phe Leu Phe Pro
            245             250             255
Pro Lys Pro Lys Asp Thr Leu Met Ile Ser Arg Thr Pro Glu Val Thr
            260             265             270
Cys Val Val Val Asp Val Ser His Glu Asp Pro Glu Val Lys Phe Asn
            275             280             285
Trp Tyr Val Asp Gly Val Glu Val His Asn Ala Lys Thr Lys Pro Arg
    290             295             300
Glu Glu Gln Tyr Asn Ser Thr Tyr Arg Val Val Ser Val Leu Thr Val
305             310             315             320
Leu His Gln Asp Trp Leu Asn Gly Lys Glu Tyr Lys Cys Lys Val Ser
            325             330             335
Asn Lys Ala Leu Pro Ala Pro Ile Glu Lys Thr Ile Ser Lys Ala Lys
            340             345             350
Gly Gln Pro Arg Glu Pro Gln Val Cys Thr Leu Pro Pro Ser Arg Asp
    355             360             365
Glu Leu Thr Lys Asn Gln Val Ser Leu Ser Cys Ala Val Lys Gly Phe
    370             375             380
Tyr Pro Ser Asp Ile Ala Val Glu Trp Glu Ser Asn Gly Gln Pro Glu
385             390             395             400
Asn Asn Tyr Lys Thr Thr Pro Pro Val Leu Asp Ser Asp Gly Ser Phe
            405             410             415
Phe Leu Val Ser Lys Leu Thr Val Asp Lys Ser Arg Trp Gln Gln Gly
            420             425             430
Asn Val Phe Ser Cys Ser Val Met His Glu Ala Leu His Asn His Tyr
    435             440             445
Thr Gln Lys Ser Leu Ser Leu Ser Pro
    450             455
```

<210> 8
<211> 214
<212> PRT

<213> Artificial Sequence

<220>
<223> anti-PD1 VK-CK artificial sequence

<400> 8
Gln Pro Val Leu Thr Gln Pro Leu Ser Val Ser Val Ala Leu Gly Gln
1               5                   10                  15
Thr Ala Arg Ile Thr Cys Gly Gly Asn Asn Ile Gly Ser Lys Asn Val
            20                  25                  30
His Trp Tyr Gln Gln Lys Pro Gly Gln Ala Pro Val Leu Val Ile Tyr
        35                  40                  45
Arg Asp Ser Asn Arg Pro Ser Gly Ile Pro Glu Arg Phe Ser Gly Ser
        50                  55                  60
Asn Ser Gly Asn Thr Ala Thr Leu Thr Ile Ser Arg Ala Gln Ala Gly
65                  70                  75                  80
Asp Glu Ala Asp Tyr Tyr Cys Gln Val Trp Asp Ser Ser Thr Ala Val
                85                  90                  95
Phe Gly Thr Gly Thr Lys Leu Thr Val Leu Gln Arg Thr Val Ala Ala
            100                 105                 110
Pro Ser Val Phe Ile Phe Pro Pro Ser Asp Glu Gln Leu Lys Ser Gly
            115                 120                 125
Thr Ala Ser Val Val Cys Leu Leu Asn Asn Phe Tyr Pro Arg Glu Ala
            130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
            165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
            195                 200                 205
Phe Asn Arg Gly Glu Cys
            210

<210> 9
<211> 103
<212> PRT
<213> Artificial Sequence

<220>
<223> IL15Rα artificial sequence

<400> 9
Asp Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Ser Ser Leu Thr Glu Cys Val Leu Asn
        35                  40                  45
Lys Ala Thr Asn Val Ala His Trp Thr Thr Pro Ser Leu Lys Cys Ile
        50                  55                  60
Arg Asp Pro Ala Leu Val His Gln Arg Pro Ala Pro Pro Ser Thr Val
65                  70                  75                  80

Thr Thr Ala Gly Val Thr Pro Gln Pro Glu Ser Leu Ser Pro Ser Gly
                85                    90                    95
Lys Glu Pro Ala Ala Ser Ser
                100

<210> 10
<211> 115
<212> PRT
<213> Natural Sequence

<220>
<223> IL15 natural sequence

<400> 10
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
                20                  25                  30
His Pro Ser Cys Lys Val Thr Ala Met Lys Cys Phe Leu Leu Glu Leu
            35                  40                  45
Gln Val Ile Ser Leu Glu Ser Gly Asp Ala Ser Ile His Asp Thr Val
        50                  55                  60
Glu Asn Leu Ile Ile Leu Ala Asn Asp Ser Leu Ser Ser Asn Gly Asn
65                  70                  75                  80
Val Thr Glu Ser Gly Cys Lys Glu Cys Glu Glu Leu Glu Glu Lys Asn
                85                  90                  95
Ile Lys Glu Phe Leu Gln Ser Phe Val His Ile Val Gln Met Phe Ile
                100                 105                 110
Asn Thr Ser
            115

<210> 11
<211> 9
<212> PRT
<213> Artificial Sequence

<220>
<223> HCDR1 prolgolimab artificial sequence

<400> 11
Phe Thr Phe Ser Ser Tyr Trp Met Tyr
1               5

<210> 12
<211> 17
<212> PRT
<213> Artificial Sequence

<220>
<223> HCDR2 prolgolimab artificial sequence

<400> 12
Ala Ile Asp Thr Gly Gly Gly Arg Thr Tyr Tyr Ala Asp Ser Val Lys
1               5                   10                  15

Gly


<210> 13
<211> 23
<212> PRT
<213> Artificial Sequence

<220>
<223> HCDR3 prolgolimab artificial sequence

<400> 13
Cys Ala Arg Asp Glu Gly Gly Gly Thr Gly Trp Gly Val Leu Lys Asp
1               5                   10                  15
Trp Pro Tyr Gly Leu Asp Ala
            20

<210> 14
<211> 11
<212> PRT
<213> Artificial Sequence

<220>
<223> LCDR1 prolgolimab artificial sequence

<400> 14
Gly Gly Asn Asn Ile Gly Ser Lys Asn Val His
1               5                   10

<210> 15
<211> 7
<212> PRT
<213> Artificial Sequence

<220>
<223> LCDR2 prolgolimab artificial sequence

<400> 15
Arg Asp Ser Asn Arg Pro Ser
1               5

<210> 16
<211> 10
<212> PRT
<213> Artificial Sequence

<220>
<223> LCDR3 prolgolimab artificial sequence

<400> 16
Cys Gln Val Trp Asp Ser Ser Thr Ala Val
1               5                   10

<210> 17

<211> 129
<212> PRT
<213> Artificial Sequence

<220>
<223> VH prolgolimab artificial sequence

<400> 17
Gln Val Gln Leu Val Gln Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Tyr Trp Val Arg Gln Val Pro Gly Lys Gly Leu Glu Trp Val
        35                  40                  45
Ser Ala Ile Asp Thr Gly Gly Gly Arg Thr Tyr Tyr Ala Asp Ser Val
    50                  55                  60
Lys Gly Arg Phe Ala Ile Ser Arg Val Asn Ala Lys Asn Thr Met Tyr
65                  70                  75                  80
Leu Gln Met Asn Ser Leu Arg Ala Glu Asp Thr Ala Val Tyr Tyr Cys
                85                  90                  95
Ala Arg Asp Glu Gly Gly Gly Thr Gly Trp Gly Val Leu Lys Asp Trp
            100                 105                 110
Pro Tyr Gly Leu Asp Ala Trp Gly Gln Gly Thr Leu Val Thr Val Ser
            115                 120                 125
Ser


<210> 18
<211> 107
<212> PRT
<213> Artificial Sequence

<220>
<223> VL prolgolimab artificial sequence

<400> 18
Gln Pro Val Leu Thr Gln Pro Leu Ser Val Ser Val Ala Leu Gly Gln
1               5                   10                  15
Thr Ala Arg Ile Thr Cys Gly Gly Asn Asn Ile Gly Ser Lys Asn Val
            20                  25                  30
His Trp Tyr Gln Gln Lys Pro Gly Gln Ala Pro Val Leu Val Ile Tyr
        35                  40                  45
Arg Asp Ser Asn Arg Pro Ser Gly Ile Pro Glu Arg Phe Ser Gly Ser
    50                  55                  60
Asn Ser Gly Asn Thr Ala Thr Leu Thr Ile Ser Arg Ala Gln Ala Gly
65                  70                  75                  80
Asp Glu Ala Asp Tyr Tyr Cys Gln Val Trp Asp Ser Ser Thr Ala Val
                85                  90                  95
Phe Gly Thr Gly Thr Lys Leu Thr Val Leu Gln
            100                 105


<210> 19
<211> 216
<212> PRT

<213> Artificial Sequence

<220>
<223> IL15Rα-CK-CiA artificial sequence

<400> 19
Asp Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Ser Ser Leu Thr Glu Cys Val Leu Asn
        35                  40                  45
Lys Ala Thr Asn Val Ala His Trp Thr Thr Pro Ser Leu Lys Cys Ile
    50                  55                  60
Arg Asp Pro Ala Leu Val His Gln Arg Pro Ala Pro Pro Ser Thr Val
65                  70                  75                  80
Thr Thr Ala Gly Val Thr Pro Gln Pro Glu Ser Leu Ser Pro Ser Gly
                85                  90                  95
Lys Glu Pro Ala Ala Ser Ser Gly Ala Glu Gly Gly Gly Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
        130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Ala
    210                 215

<210> 20
<211> 454
<212> PRT
<213> Artificial Sequence

<220>
<223> IL15-CH1CH2CH3- CiA artificial sequence

<400> 20
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
            20                  25                  30
His Pro Ser Cys Lys Val Thr Ala Met Lys Cys Phe Leu Leu Glu Leu
        35                  40                  45
Gln Val Ile Ser Leu Glu Ser Gly Asp Ala Ser Ile His Asp Thr Val
    50                  55                  60
Glu Asn Leu Ile Ile Leu Ala Asn Asp Ser Leu Ser Ser Asn Gly Asn
65                  70                  75                  80

```
Val Thr Glu Ser Gly Cys Lys Glu Cys Glu Glu Leu Glu Glu Lys Asn
                85                      90                      95
Ile Lys Glu Phe Leu Gln Ser Phe Val His Ile Val Gln Met Phe Ile
            100                     105                     110
Asn Thr Ser Ala Gly Gly Lys Pro Gly Gly Ser Ala Gly Gly Ala Ser
            115                     120                     125
Thr Lys Gly Pro Ser Val Phe Pro Leu Ala Pro Ser Ser Lys Ser Thr
    130                     135                     140
Ser Gly Gly Thr Ala Ala Leu Gly Cys Leu Val Lys Asp Tyr Phe Pro
145                     150                     155                 160
Glu Pro Val Thr Val Ser Trp Asn Ser Gly Ala Leu Thr Ser Gly Val
                165                     170                     175
His Thr Phe Pro Ala Val Leu Gln Ser Ser Gly Leu Tyr Ser Leu Ser
            180                     185                     190
Ser Val Val Thr Val Pro Ser Ser Ser Leu Gly Thr Gln Thr Tyr Ile
            195                     200                     205
Cys Asn Val Asn His Lys Pro Ser Asn Thr Lys Val Asp Lys Arg Val
    210                     215                     220
Glu Pro Lys Ser Ala Asp Lys Thr His Thr Cys Pro Pro Cys Pro Ala
225                     230                     235                 240
Pro Glu Ala Ala Gly Gly Pro Ser Val Phe Leu Phe Pro Pro Lys Pro
                245                     250                     255
Lys Asp Thr Leu Met Ile Ser Arg Thr Pro Glu Val Thr Cys Val Val
            260                     265                     270
Val Asp Val Ser His Glu Asp Pro Glu Val Lys Phe Asn Trp Tyr Val
            275                     280                     285
Asp Gly Val Glu Val His Asn Ala Lys Thr Lys Pro Arg Glu Glu Gln
    290                     295                     300
Tyr Asn Ser Thr Tyr Arg Val Val Ser Val Leu Thr Val Leu His Gln
305                     310                     315                 320
Asp Trp Leu Asn Gly Lys Glu Tyr Lys Cys Lys Val Ser Asn Lys Ala
                325                     330                     335
Leu Pro Ala Pro Ile Glu Lys Thr Ile Ser Lys Ala Lys Gly Gln Pro
            340                     345                     350
Arg Glu Pro Gln Val Tyr Thr Leu Pro Pro Ser Arg Asp Glu Leu Thr
            355                     360                     365
Lys Asn Gln Val Ser Leu Thr Cys Leu Val Lys Gly Phe Tyr Pro Ser
    370                     375                     380
Asp Ile Ala Val Glu Trp Glu Ser Asn Gly Gln Pro Glu Asn Asn Tyr
385                     390                     395                 400
Lys Thr Thr Pro Pro Val Leu Asp Ser Asp Gly Ser Phe Phe Leu Tyr
                405                     410                     415
Ser Lys Leu Thr Val Asp Lys Ser Arg Trp Gln Gln Gly Asn Val Phe
            420                     425                     430
Ser Cys Ser Val Met His Glu Ala Leu His Asn His Tyr Thr Gln Lys
            435                     440                     445
Ser Leu Ser Leu Ser Pro
    450
```

```
<210> 21
<211> 233
<212> PRT
<213> Artificial Sequence
```

<220>
<223> IL15-CK- CiA artificial sequence

<400> 21
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
            20                  25                  30
His Pro Ser Cys Lys Val Thr Ala Met Lys Cys Phe Leu Leu Glu Leu
            35                  40                  45
Gln Val Ile Ser Leu Glu Ser Gly Asp Ala Ser Ile His Asp Thr Val
        50                  55                  60
Glu Asn Leu Ile Ile Leu Ala Asn Asp Ser Leu Ser Ser Asn Gly Asn
65                  70                  75                  80
Val Thr Glu Ser Gly Cys Lys Glu Cys Glu Glu Leu Glu Glu Lys Asn
                85                  90                  95
Ile Lys Glu Phe Leu Gln Ser Phe Val His Ile Val Gln Met Phe Ile
            100                 105                 110
Asn Thr Ser Ala Gly Gly Lys Pro Gly Gly Ser Ala Gly Gly Arg Thr
            115                 120                 125
Val Ala Ala Pro Ser Val Phe Ile Phe Pro Pro Ser Asp Glu Gln Leu
        130                 135                 140
Lys Ser Gly Thr Ala Ser Val Val Cys Leu Leu Asn Asn Phe Tyr Pro
145                 150                 155                 160
Arg Glu Ala Lys Val Gln Trp Lys Val Asp Asn Ala Leu Gln Ser Gly
                165                 170                 175
Asn Ser Gln Glu Ser Val Thr Glu Gln Asp Ser Lys Asp Ser Thr Tyr
            180                 185                 190
Ser Leu Ser Ser Thr Leu Thr Leu Ser Lys Ala Asp Tyr Glu Lys His
        195                 200                 205
Lys Val Tyr Ala Cys Glu Val Thr His Gln Gly Leu Ser Ser Pro Val
    210                 215                 220
Thr Lys Ser Phe Asn Arg Gly Glu Ala
225                 230

<210> 22
<211> 436
<212> PRT
<213> Artificial Sequence

<220>
<223> IL15Rα-CH1CH2CH3- CiA artificial sequence

<400> 22
Asp Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Ser Ser Leu Thr Glu Cys Val Leu Asn
            35                  40                  45
Lys Ala Thr Asn Val Ala His Trp Thr Thr Pro Ser Leu Lys Cys Ile
        50                  55                  60
Arg Asp Pro Ala Leu Val His Gln Arg Pro Ala Pro Pro Ser Thr Val
65                  70                  75                  80

```
Thr Thr Ala Gly Val Thr Pro Gln Pro Glu Ser Leu Ser Pro Ser Gly
            85                  90                  95
Lys Glu Pro Ala Ala Ser Ser Gly Ala Glu Gly Gly Gly Ala Ser Thr
            100             105             110
Lys Gly Pro Ser Val Phe Pro Leu Ala Pro Ser Ser Lys Ser Thr Ser
            115             120             125
Gly Gly Thr Ala Ala Leu Gly Cys Leu Val Lys Asp Tyr Phe Pro Glu
        130             135             140
Pro Val Thr Val Ser Trp Asn Ser Gly Ala Leu Thr Ser Gly Val His
145             150             155             160
Thr Phe Pro Ala Val Leu Gln Ser Ser Gly Leu Tyr Ser Leu Ser Ser
            165             170             175
Val Val Thr Val Pro Ser Ser Ser Leu Gly Thr Gln Thr Tyr Ile Cys
            180             185             190
Asn Val Asn His Lys Pro Ser Asn Thr Lys Val Asp Lys Arg Val Glu
            195             200             205
Pro Lys Ser Ala Asp Lys Thr His Thr Cys Pro Pro Cys Pro Ala Pro
        210             215             220
Glu Ala Ala Gly Gly Pro Ser Val Phe Leu Phe Pro Pro Lys Pro Lys
225             230             235             240
Asp Thr Leu Met Ile Ser Arg Thr Pro Glu Val Thr Cys Val Val Val
            245             250             255
Asp Val Ser His Glu Asp Pro Glu Val Lys Phe Asn Trp Tyr Val Asp
            260             265             270
Gly Val Glu Val His Asn Ala Lys Thr Lys Pro Arg Glu Glu Gln Tyr
        275             280             285
Asn Ser Thr Tyr Arg Val Val Ser Val Leu Thr Val Leu His Gln Asp
    290             295             300
Trp Leu Asn Gly Lys Glu Tyr Lys Cys Lys Val Ser Asn Lys Ala Leu
305             310             315             320
Pro Ala Pro Ile Glu Lys Thr Ile Ser Lys Ala Lys Gly Gln Pro Arg
            325             330             335
Glu Pro Gln Val Tyr Thr Leu Pro Pro Ser Arg Asp Glu Leu Thr Asn
            340             345             350
Gln Val Ser Leu Thr Cys Leu Val Lys Gly Phe Tyr Pro Ser Asp Ile
            355             360             365
Ala Val Glu Trp Glu Ser Asn Gly Gln Pro Glu Asn Asn Tyr Lys Thr
    370             375             380
Thr Pro Pro Val Leu Asp Ser Asp Gly Ser Phe Phe Leu Tyr Ser Lys
385             390             395             400
Leu Thr Val Asp Lys Ser Arg Trp Gln Gln Gly Asn Val Phe Ser Cys
            405             410             415
Ser Val Met His Glu Ala Leu His Asn His Tyr Thr Gln Lys Ser Leu
            420             425             430
Ser Leu Ser Pro
            435
```

```
<210> 23
<211> 453
<212> PRT
<213> Artificial Sequence

<220>
<223> IL15-CH1CH2CH3 knob-Fc artificial sequence
```

<400> 23

```
Asp Asn Trp Val Asn Val Ile Ser Asp Leu Lys Lys Ile Glu Asp Leu
1               5                   10                  15
Ile Gln Ser Met His Ile Asp Ala Thr Leu Tyr Thr Glu Ser Asp Val
                20                  25                  30
His Pro Ser Cys Lys Val Thr Ala Met Lys Cys Phe Leu Leu Glu Leu
            35                  40                  45
Gln Val Ile Ser Leu Glu Ser Gly Asp Ala Ser Ile His Asp Thr Val
        50                  55                  60
Glu Asn Leu Ile Ile Leu Ala Asn Asp Ser Leu Ser Ser Asn Gly Asn
65                  70                  75                  80
Val Thr Glu Ser Gly Cys Lys Glu Cys Glu Glu Leu Glu Glu Lys Asn
                85                  90                  95
Ile Lys Glu Phe Leu Gln Ser Phe Val His Ile Val Gln Met Phe Ile
            100                 105                 110
Asn Thr Ser Ala Gly Gly Lys Pro Gly Gly Ser Ala Gly Gly Ala Ser
            115                 120                 125
Thr Lys Gly Pro Ser Val Phe Pro Leu Ala Pro Ser Ser Lys Ser Thr
    130                 135                 140
Ser Gly Gly Thr Ala Ala Leu Gly Cys Leu Val Lys Asp Tyr Phe Pro
145                 150                 155                 160
Glu Pro Val Thr Val Ser Trp Asn Ser Gly Ala Leu Thr Ser Gly Val
            165                 170                 175
His Thr Phe Pro Ala Val Leu Gln Ser Ser Gly Leu Tyr Ser Leu Ser
            180                 185                 190
Ser Val Val Thr Val Pro Ser Ser Ser Leu Gly Thr Gln Thr Tyr Ile
        195                 200                 205
Cys Asn Val Asn His Lys Pro Ser Asn Thr Lys Val Asp Lys Arg Val
    210                 215                 220
Glu Pro Lys Ser Ala Asp Lys Thr His Thr Cys Pro Pro Cys Pro Ala
225                 230                 235                 240
Pro Glu Ala Ala Gly Gly Pro Ser Val Phe Leu Phe Pro Pro Lys Pro
                245                 250                 255
Lys Asp Thr Leu Met Ile Ser Arg Thr Pro Glu Val Thr Cys Val Val
            260                 265                 270
Val Asp Val Ser His Glu Asp Pro Glu Val Lys Phe Asn Trp Tyr Val
        275                 280                 285
Asp Gly Val Glu Val His Asn Ala Lys Thr Lys Pro Arg Glu Glu Gln
    290                 295                 300
Tyr Asn Ser Thr Tyr Arg Val Val Ser Val Leu Thr Val Leu His Gln
305                 310                 315                 320
Asp Trp Leu Asn Gly Lys Glu Tyr Lys Cys Lys Val Ser Asn Lys Ala
            325                 330                 335
Leu Pro Ala Pro Ile Glu Lys Thr Ile Ser Lys Ala Lys Gly Gln Pro
            340                 345                 350
Arg Glu Pro Gln Val Tyr Thr Leu Pro Pro Arg Asp Glu Leu Thr Lys
        355                 360                 365
Asn Gln Val Ser Leu Trp Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
```

64

```
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
            435                 440                 445
Leu Ser Leu Ser Pro
            450


<210> 24
<211> 436
<212> PRT
<213> Artificial Sequence

<220>
<223> IL15Rα-CH1CH2CH3 knob-Fc artificial sequence

<400> 24
Asp Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Ser Ser Leu Thr Glu Cys Val Leu Asn
            35                  40                  45
Lys Ala Thr Asn Val Ala His Trp Thr Thr Pro Ser Leu Lys Cys Ile
            50                  55                  60
Arg Asp Pro Ala Leu Val His Gln Arg Pro Ala Pro Pro Ser Thr Val
65                  70                  75                  80
Thr Thr Ala Gly Val Thr Pro Gln Pro Glu Ser Leu Ser Pro Ser Gly
                85                  90                  95
Lys Glu Pro Ala Ala Ser Ser Gly Ala Glu Gly Gly Gly Ala Ser Thr
            100                 105                 110
Lys Gly Pro Ser Val Phe Pro Leu Ala Pro Ser Ser Lys Ser Thr Ser
            115                 120                 125
Gly Gly Thr Ala Ala Leu Gly Cys Leu Val Lys Asp Tyr Phe Pro Glu
            130                 135                 140
Pro Val Thr Val Ser Trp Asn Ser Gly Ala Leu Thr Ser Gly Val His
145                 150                 155                 160
Thr Phe Pro Ala Val Leu Gln Ser Ser Gly Leu Tyr Ser Leu Ser Ser
                165                 170                 175
Val Val Thr Val Pro Ser Ser Ser Leu Gly Thr Gln Thr Tyr Ile Cys
                180                 185                 190
Asn Val Asn His Lys Pro Ser Asn Thr Lys Val Asp Lys Arg Val Glu
            195                 200                 205
Pro Lys Ser Ala Asp Lys Thr His Thr Cys Pro Pro Cys Pro Ala Pro
            210                 215                 220
Glu Ala Ala Gly Gly Pro Ser Val Phe Leu Phe Pro Pro Lys Pro Lys
225                 230                 235                 240
Asp Thr Leu Met Ile Ser Arg Thr Pro Glu Val Thr Cys Val Val Val
                245                 250                 255
Asp Val Ser His Glu Asp Pro Glu Val Lys Phe Asn Trp Tyr Val Asp
                260                 265                 270
Gly Val Glu Val His Asn Ala Lys Thr Lys Pro Arg Glu Glu Gln Tyr
            275                 280                 285
Asn Ser Thr Tyr Arg Val Val Ser Val Leu Thr Val Leu His Gln Asp
            290                 295                 300
```

```
Trp Leu Asn Gly Lys Glu Tyr Lys Cys Lys Val Ser Asn Lys Ala Leu
305             310         315             320
Pro Ala Pro Ile Glu Lys Thr Ile Ser Lys Ala Lys Gly Gln Pro Arg
                325             330             335
Glu Pro Gln Val Tyr Thr Leu Pro Pro Arg Asp Glu Leu Thr Lys Asn
            340             345             350
Gln Val Ser Leu Trp Cys Leu Val Lys Gly Phe Tyr Pro Ser Asp Ile
            355             360             365
Ala Val Glu Trp Glu Ser Asn Gly Gln Pro Glu Asn Asn Tyr Lys Thr
    370             375             380
Thr Pro Pro Val Leu Asp Ser Asp Gly Ser Phe Phe Leu Tyr Ser Lys
385             390             395             400
Leu Thr Val Asp Lys Ser Arg Trp Gln Gln Gly Asn Val Phe Ser Cys
            405             410             415
Ser Val Met His Glu Ala Leu His Asn His Tyr Thr Gln Lys Ser Leu
            420             425             430
Ser Leu Ser Pro
            435
```

## Claims

1. An immunocytokine for stimulating the activation and/or proliferation of IL-15Rbeta/gamma-positive cells, comprising a heterodimeric protein complex based on IL-15/IL-15Rα comprising:

   1) IL-15Rα, which is linked to

      a) an antibody light chain constant domain, or
      b) antibody heavy chain constant domains comprising a first (CH1) heavy chain constant domain and an Fc fragment monomer comprising second (CH2) and third (CH3) heavy chain constant domains;

   2) IL-15, which is linked to

      a) antibody heavy chain constant domains comprising a first (CH1) heavy chain constant domain and an Fc fragment monomer comprising second (CH2) and third (CH3) heavy chain constant domains, or
      b) an antibody light chain constant domain;

   wherein the first antibody heavy chain constant domain and the antibody light chain constant domain within the heterodimeric complex have or do not have a covalent association through a natural S-S bridge, and wherein, if the IL-15 or the IL-15Rα is linked to the antibody light chain constant domain, then the other part of the heterodimeric protein complex selected from IL-15Rα or IL-15is linked to the antibody heavy chain constant domains.

2. The immunocytokine of Claim 1, wherein the antibody light chain constant domain is selected from CK or CL.

3. The immunocytokine of Claim 1, wherein the IL-15Rα has the amino acid sequence represented by SEQ ID NO:9, or any known mutant IL-15Rα variant with a similar biological activity.

4. The immunocytokine of Claim 1, wherein the IL-15 has the amino acid sequence represented by SEQ ID NO:10, or any known mutant IL-15 variant with a similar biological activity.

5. The immunocytokine of Claim 1, wherein the IL-15Rα is linked to the antibody light chain constant domain.

6. The immunocytokine of Claim 5, wherein the IL-15Rα linked to the antibody light chain constant domain has the amino acid sequence represented by SEQ ID NO:1 or SEQ ID NO:19.

7. The immunocytokine of Claim 1, wherein the IL-15 is linked to the antibody light chain constant domain.

EP 4 032 539 A1

8. The immunocytokine of Claim 7, wherein the IL-15 linked to the antibody light chain constant domain has the amino acid sequence represented by SEQ ID NO:3 or SEQ ID NO:21.

9. The immunocytokine of Claim 1, wherein the first (CH1) heavy chain constant domain and the Fc fragment monomer comprising second (CH2) and third (CH3) heavy chain constant domains are linked via a hinge.

10. The immunocytokine of Claim 1, wherein the IL-15 or IL-15Rα is linked to antibody heavy chain constant domains arranged in the following order: CH1-hinge-CH2-CH3.

11. The immunocytokine of Claim 10, wherein the IL-15Rα is linked to antibody heavy chain constant domains.

12. The immunocytokine of Claim 11, wherein the IL-15Rα linked to the antibody heavy chain constant domains has the amino acid sequence represented by SEQ ID NO:4 or SEQ ID NO:22.

13. The immunocytokine of Claim 10, wherein the IL-15 is linked to antibody heavy chain constant domains.

14. The immunocytokine of Claim 13, wherein the IL-15 linked to the antibody heavy chain constant domains has the amino acid sequence represented by SEQ ID NO:2 or SEQ ID NO:20.

15. The immunocytokine of Claim 1, wherein

IL-15Rα linked to the antibody light chain constant domain has the amino acid sequence represented by SEQ ID NO:1 or SEQ ID NO:19, and
IL-15 linked to the antibody heavy chain constant domains has the amino acid sequence represented by SEQ ID NO:2 or SEQ ID NO:20.

16. The immunocytokine of Claim 1, wherein

the IL-15Rα linked to the antibody heavy chain constant domains has the amino acid sequence represented by SEQ ID NO:4 or SEQ ID NO:22, and
the IL-15 linked to the antibody light chain constant domain has the amino acid sequence represented by SEQ ID NO:3 or SEQ ID NO:21.

17. The immunocytokine of Claim 1 comprising mutations in the Fc fragment monomer, which cause the deficiency of ADCC, CDC, and/or ADCP properties in said immunocytokine.

18. The immunocytokine of Claim 1, wherein the Fc fragment belongs to IgG.

19. The immunocytokine of Claim 18, wherein the Fc fragment isotype is selected from the group comprising: human IgG1, IgG2, or IgG4.

20. The immunocytokine of any of Claims 1-19 comprising the above two heterodimeric protein complexes based on IL-15/IL-15Rα.

21. The immunocytokine of Claim 20 for the use as a therapeutic agent for the treatment of cancer or an autoimmune disease.

22. An immunocytokine for stimulating the activation and/or proliferation of IL-15Rbeta/gamma-positive cells, comprising a heterodimeric protein complex based on IL-15/IL-15Rα and an immunomodulatory antibody or antigen binding fragment thereof, which specifically inhibit the PD-1 pathway,

wherein the heterodimeric protein complex based on IL-15/IL-15Rα comprises:

1) IL-15Rα, which is linked to

a) an antibody light chain constant domain, or
b) antibody heavy chain constant domains comprising a first (CH1) heavy chain constant domain and an Fc fragment monomer comprising second (CH2) and third (CH3) heavy chain constant domains;

67

2) IL-15, which is linked to

a) antibody heavy chain constant domains comprising a first (CH1) heavy chain constant domain and an Fc fragment monomer comprising second (CH2) and third (CH3) heavy chain constant domains, or
b) an antibody light chain constant domain;

wherein the first antibody heavy chain constant domain and the antibody light chain constant domain within the heterodimeric complex have or do not have a covalent association through a natural S-S bridge, and
wherein, if the IL-15 or the IL-15Rα is linked to the antibody light chain constant domain, then the other part, of the heterodimeric protein complex, selected from IL-15Rα or IL-15, is linked to the antibody heavy chain constant domains.

23. The immunocytokine of Claim wherein the antibody light chain constant domain is selected from CK or CL.

24. The immunocytokine of Claim 22, wherein the IL-15Rα has the amino acid sequence represented by SEQ ID NO:9, or any known mutant IL-15Rα variant with a similar biological activity.

25. The immunocytokine of Claim 22, wherein the IL-15 has the amino acid sequence represented by SEQ ID NO:10, or any known mutant IL-15 variant with a similar biological activity.

26. The immunocytokine of Claim 22, wherein the IL-15Rα is linked to the antibody light chain constant domain.

27. The immunocytokine of Claim 26, wherein the IL-15Rα linked to the antibody light chain constant domain has the amino acid sequence represented by SEQ ID NO:1 or SEQ ID NO:19.

28. The immunocytokine of Claim 22, wherein the IL-15 is linked to the antibody light chain constant domain.

29. The immunocytokine of Claim 28, wherein the IL-15 linked to the antibody light chain constant domain has the amino acid sequence represented by SEQ ID NO:3 or SEQ ID NO:21.

30. The immunocytokine of Claim 22, wherein the first (CH1) heavy chain constant domain and the Fc fragment monomer comprising second (CH2) and third (CH3) heavy chain constant domains are linked via a hinge.

31. The immunocytokine of Claim 22, wherein the IL-15 or IL-15Rα is linked to antibody heavy chain constant domains arranged in the following order: CH1-hinge-CH2-CH3.

32. The immunocytokine of Claim 31, wherein the IL-15Rα is linked to the antibody heavy chain constant domains.

33. The immunocytokine of Claim 32, wherein the IL-15Rα linked to the antibody heavy chain constant domains has the amino acid sequence represented by SEQ ID NO:6 or SEQ ID NO:24.

34. The immunocytokine of Claim 31, wherein the IL-15 is linked to the antibody heavy chain constant domains.

35. The immunocytokine of Claim 34, wherein the IL-15 linked to the antibody heavy chain constant domains has the amino acid sequence represented by SEQ ID NO:5 or SEQ ID NO:23.

36. The immunocytokine of Claim 22, wherein the immunomodulatory antibody or the antigen binding fragment thereof, which specifically inhibit the PD-1 pathway, is an antibody that specifically binds to PD-1.

37. The immunocytokine of Claim 22, wherein the immunomodulatory antibody comprises:

a) a light chain comprising a light chain variable domain and a light chain constant domain;
b) a heavy chain comprising a heavy chain variable domain and antibody heavy chain constant domains comprising a first (CH1) heavy chain constant domain and an Fc fragment monomer comprising second (CH2) and third (CH3) heavy chain constant domains.

38. The immunocytokine of Claim 37, wherein the light chain variable domain comprises LCDRs 1, 2, and 3 (hypervariable regions 1, 2, and 3), which are represented by the amino acid sequences of SEQ ID NO:14, SEQ ID NO:15, and SEQ ID NO:16, respectively.

39. The immunocytokine of Claim 38, wherein the light chain variable domain comprises the amino acid sequence represented by SEQ ID NO:18.

40. The immunocytokine of Claim 37, wherein the heavy chain variable domain comprises HCDRs 1, 2, and 3 (hypervariable regions 1, 2, and 3), which are represented by the amino acid sequences of SEQ ID NO:11, SEQ ID NO:12, and SEQ ID NO:13, respectively.

41. The immunocytokine of Claim 40, wherein the heavy chain variable domain comprises the amino acid sequence represented by SEQ ID NO:17.

42. The immunocytokine of Claim 37, wherein

1) the light chain variable domain comprises LCDRs 1, 2, and 3 (hypervariable regions 1, 2, and 3), which are represented by the amino acid sequences of SEQ ID NO:14, SEQ ID NO:15, and SEQ ID NO:16, respectively.
2) the heavy chain variable domain comprises HCDRs 1, 2, and 3 (hypervariable regions 1, 2, and 3), which are represented by the amino acid sequences of SEQ ID NO:11, SEQ ID NO:12, and SEQ ID NO:13, respectively.

43. The immunocytokine of Claim 42, wherein

1) the light chain variable domain comprises the amino acid sequence represented by SEQ ID NO:18;
2) the heavy chain variable domain comprises the amino acid sequence represented by SEQ ID NO:17.

44. The immunocytokine of Claim 37, wherein the immunomodulatory antibody comprises a light chain comprising the amino acid sequence represented by SEQ ID NO:8.

45. The immunocytokine of Claim 37, wherein the immunomodulatory antibody comprises a heavy chain comprising the amino acid sequence represented by SEQ ID NO:7.

46. The immunocytokine of Claim 37, wherein the immunomodulatory antibody comprises:

a heavy chain comprising the amino acid sequence represented by SEQ ID NO:7;
a light chain comprising the amino acid sequence represented by SEQ ID NO:8.

47. The immunocytokine of Claim 22, wherein the immunomodulatory antibody or the antigen binding fragment thereof, which specifically inhibit the PD-1 pathway, is an antibody that specifically binds to PD-L1.

48. The immunocytokine of Claim 22, wherein

a) the heterodimeric protein complex comprises based on IL-15/IL-15Rα:

IL-15Rα linked to the antibody light chain constant domain has the amino acid sequence represented by SEQ ID NO:1 or SEQ ID NO:19, and
IL-15 linked to the antibody heavy chain constant domains has the amino acid sequence represented by SEQ ID NO:5 or SEQ ID NO:23;

b) the immunomodulatory antibody comprises:

a heavy chain comprising the amino acid sequence represented by SEQ ID NO:7;
a light chain comprising the amino acid sequence represented by SEQ ID NO:8.

49. The immunocytokine of Claim 22, wherein

a) the IL-15Rα linked to the antibody heavy chain constant domains has the amino acid sequence represented by SEQ ID NO:6 or SEQ ID NO:24, and
the IL-15 linked to the antibody light chain constant domain has the amino acid sequence represented by SEQ ID NO:3 or SEQ ID NO:21;

b) the immunomodulatory antibody comprises:

a heavy chain comprising the amino acid sequence represented by SEQ ID NO:7;
a light chain comprising the amino acid sequence represented by SEQ ID NO:8.

50. The immunocytokine of any of Claims 22 or 37 having mutations in the antibody constant domains, which induce the heterodimerization of two different portions, one of which comprises covalently or non-covalently associated IL-15Rα linked to the antibody constant domains and IL-15 linked to the antibody constant domain, and the other portion comprises covalently or non-covalently associated light and heavy chains of antibody.

51. The immunocytokine of any of Claims 22 or 37, wherein the first Fc monomer and the second Fc monomer are selected from the following group: the first Fc monomer is a Knob modified Fc, and the second Fc monomer is a Hole modified Fc, or when the second Fc monomer is a Knob modified Fc, and the first Fc monomer is a Hole modified Fc.

52. The immunocytokine of Claim 51, wherein

a) the first Fc monomer has the amino acid substitutions S354C/T366W, and the second Fc monomer has the amino acid substitutions Y349C/T366S/L368A/Y407V.
b) first Fc monomer has the amino acid substitutions Y349C/T366S/L368A/Y407, and the second Fc monomer has the amino acid substitutions S354C/T366W.

53. The immunocytokine of any of Claims 22 or 37, wherein the Fc fragment belongs to IgG.

54. The immunocytokine of Claim 53, wherein the Fc fragment isotype is selected from the group comprising: human IgG1, IgG2, or IgG4.

55. The immunocytokine of any of Claims 22 or 37 having mutations in the Fc fragment monomer, which cause the deficiency of ADCC, CDC, and/or ADCP properties in said immunocytokine.

56. The immunocytokine of any of Claims 22-55 for the treatment of an oncological or autoimmune disease.

57. An isolated nucleic acid encoding the immunocytokine of any of Claims 1-20 or 22-55.

58. The nucleic acid of Claim 57, wherein the nucleic acid is DNA.

59. An expression vector comprising the nucleic acid of any of Claims 57-58.

60. A method for producing a host cell for producing the immunocytokine of any of Claims 1-20 or 22-55 comprising transformation of the cell with the vector of Claim 59.

61. A host cell for producing the immunocytokine of any of Claims 1-20 or 22-55 comprising the nucleic acid of any of Claims 57-58.

62. A method for producing a product comprising the immunocytokine of any of Claims 1-20 or 22-55 comprising culturing of the host cell of Claim 61 in a culture medium under conditions sufficient to produce said immunocytokine, if necessary, followed by isolation and purification of the produced immunocytokine.

63. A pharmaceutical composition for stimulating the activation and/or proliferation of IL-15Rbeta/gamma-positive cells, comprising the immunocytokine of any of Claims 1-20 or 22-55 in a therapeutically effective amount, in combination with one or more pharmaceutically acceptable excipients.

64. The pharmaceutical composition of Claim 63 for the treatment of an oncological or autoimmune disease.

65. The pharmaceutical composition of Claim 64, wherein the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

66. A method of treatment of an oncological disease, comprising administering to a subject in need of such treatment the immunocytokine of any of Claims 1-20 or 22-55, or the pharmaceutical composition of Claim 63, in a therapeutically effective amount.

67. The method of treatment of Claim 55, wherein the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

68. A method for activating the biological activity of a T cell population or NK cell population in a subject in need of such activation, comprising administering to the subject an effective amount of the immunocytokine of any of Claims 1-20 or 22-55 or the pharmaceutical composition of Claim 63.

69. A use of the immunocytokine of any of Claims 1-20 or 22-55 or the pharmaceutical composition of Claim 63 for the treatment in a subject in need of such treatment of an oncological disease.

70. The use of Claim 69, wherein the oncological disease is selected from the group comprising: HNSCC (head and neck squamous cell carcinoma), cervical cancer, cancer of unknown primary, glioblastoma, esophageal cancer, bladder cancer, TNBC (triple-negative breast cancer), CRC (colorectal cancer), hepatocellular carcinoma, melanoma, NSCLC (non-small cell lung cancer), kidney cancer, ovarian cancer, MSI CRC (colorectal cancer with microsatellite instability), leukemia (acute leukemia or myeloblastic leukemia), lymphoma, multiple myeloma, melanoma, breast cancer, colorectal cancer, prostate cancer, bladder cancer, sarcoma, hepatocellular carcinoma, glioblastoma, Hodgkin's lymphoma, T- and B-cell acute lymphoblastic leukemia, small cell lung cancer, acute myeloblastic leukemia, refractory non-Hodgkin's B-cell lymphoma, follicular lymphoma, marginal zone B-cell lymphoma, diffuse large B-cell lymphoma, head and neck squamous cell carcinoma, pancreatic cancer, ovarian cancer, acute myeloblastic leukemia and higher-risk myelodysplastic syndrome.

Fig. 1

Fig. 2

Fig. 3

Fig. 4

**pEE-IL15Ra-CK**
5061 bp

Fig. 5

**pEE-IL15-CH1-Fc-LALA**
5775 bp

pUC origin

CMV promoter

5000

1000

AmpR

leader

IL15

4000

2000

oriP

CH1

LALA

Fc-LALA

polyA

3000

Fig. 6

pEE-BCD100-02-VL-CK
4887 bp

Fig. 7

**pEE-BCD100-02-VH-CH1-Fc-hole-LALA**
5842 bp

Fig. 8

pEE-IL15-CH1-Fc-knob-LALA
5776 bp

Fig. 9

Fig. 10

Фиг.10

Fig. 11

Fig. 12

Fig. 13

Fig. 14

Fig. 15

Fig. 16

Fig. 17

EP 4 032 539 A1

Fig. 18

Fig. 19

Fig. 20

EP 4 032 539 A1

EP 4 032 539 A1

Fig. 21

Fig. 22

Fig. 23

Fig. 24

Fig. 25

## INTERNATIONAL SEARCH REPORT

| | International application No. |
|---|---|
| | PCT/RU 2020/050233 |

**A. CLASSIFICATION OF SUBJECT MATTER**
See supplemental sheet

According to International Patent Classification (IPC) or to both national classification and IPC

**B. FIELDS SEARCHED**

Minimum documentation searched (classification system followed by classification symbols)
A61K; A61P; C07K; C12N

Documentation searched other than minimum documentation to the extent that such documents are included in the fields searched

Electronic data base consulted during the international search (name of data base and, where practicable, search terms used)
ESPACENET, Google, Google Patents, PatSearch, RUPTO, USPTO

**C. DOCUMENTS CONSIDERED TO BE RELEVANT**

| Category* | Citation of document, with indication, where appropriate, of the relevant passages | Relevant to claim No. |
|---|---|---|
| A | RU 2689717 C2 (SHANKHAI KHENZHUI FARMASIUTIKAL KO., LTD.), 28.05.2019, the claims | 1-70 |
| A | WO 2019/006472 A1 (XENCOR, INC), 03.01.2019, the claims | 1-70 |
| A | WO 2012/040323 A2 (ALTOR BIOSCIEN CORPORATION et al.), 29.03.2012, the claims | 1-70 |
| A | RU 2644671 C2 (SITJUN FARMA; INSERM (ENSTITJU NASONAL DE LYA SANTE E DE LYA RESHERSH MEDIKAL), 13.02.2018, the claims | 1-70 |
| A | WO 2018/071918 A1 (XENCOR, INC), 19.04.2018, the claims | 1-70 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 November 2020 (27.11.2020) | 03 December 2020 (03.12.2020) |

| Name and mailing address of the ISA/ RU | Authorized officer |
|---|---|
| Facsimile No. | Telephone No. |

Form PCT/ISA/210 (second sheet) (April 2005)

INTERNATIONAL SEARCH REPORT

International application No.

PCT/RU 2020/050233

*A61K 38/20* (2006.01)

*A61K 39/395* (2006.01)

*A61P 35/00* (2006.01)

*C07K 14/54* (2006.01)

*C07K 14/715* (2006.01)

*C07K 16/28* (2006.01)

*C07K 19/00* (2006.01)

*C12N 15/13* (2006.01)

*C12N 15/24* (2006.01)

*C12N 15/62* (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007046006 A
- WO 2016095642 A
- WO 2015103928 A
- WO 2018071919 A
- WO 2015018528 A
- WO 2018071918 A
- WO 2012175222 A
- WO 2019006472 A
- US 5500362 A
- US 5821337 A
- WO 2018013017 A
- RU 2665790
- WO 2018194496 A
- WO 1996027011 A

- US 5168062 A
- US 4510245 A
- US 4968615 A
- US 4399216 A
- US 4634665 A
- US 5179017 A
- US 4912040 A
- US 4740461 A
- US 4959455 A
- EP 0216846 A
- EP 0256055 A
- EP 0323997 A
- EP 0338841 A

### Non-patent literature cited in the description

- **GRABSTEIN, K.H. et al.** *Science,* 1994, vol. 264, 965
- **BURTON, J.D. et al.** *Proc. Natl. Acad. Sci. USA,* 1994, vol. 91, 4935
- **BAMFORD RN. et al.** *J. Immunol.,* 1998, vol. 160, 4418-4426
- **KURYS G et al.** *J. Biol. Chem.,* 2000, vol. 275, 30653-30659
- **MUSSO et al.** *Blood,* 15 May 1999, vol. 93 (10), 3531-3539
- **BUDALGIAN et al.** *JBC,* 2004, vol. 279 (40), 42192-42201
- **KIRMAN I.** *Am. J. Gastroenterol,* 1996, vol. 91, 1789
- *Ruckert R.,* 2000, vol. 165, 2240-2250
- **YAMADA Y.** *Leukemia and Lymphoma,* 1999, vol. 35 (1-2), 37-45
- **MCLNNES I.B.** *Immunology Today,* 1998, vol. 19, 75-79
- **WILKINSON.** *J. Exp. Med.,* 1995, vol. 181, 1255-1259
- **BUDAGIAN V. et al.** *JBC,* 2004, vol. 279 (39), 40368-40375
- **MORTIER et al.** *The Journal of Immunology,* 2004, vol. 173, 1681-1688
- **RAVETCH ; KINET.** *Annu. Rev. Immunol,* 1991, vol. 9, 457-92
- **CLYNES et al.** *PNAS (USA),* 1998, vol. 95, 652-656

- **GAZZANO-SANTORO et al.** *J. Immunol. Methods,* 1996, vol. 202, 163
- **WOOF J. ; BURTON D.** *Nat Rev Immunol,* 2004, vol. 4, 89-99
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **BURKS et al.** *Proc Natl Acad Sci USA,* 1997, vol. 94, 412-417
- **WU et al.** *Proc Natl Acad Sci USA,* 1998, vol. 95, 6037-6042
- **RIDGWAY, J. B.** *Presta L G, Carter P*
- **MERCHANT, A. M. et al.** *Nature Biotech,* 1998, vol. 16, 677-681
- **ATWELL, S. ; RIDGWAY, J. B. ; WELLS, J. A. ; CARTER, P.** *J Mol Biol,* 1997, vol. 270, 26-35
- **AGNEW.** *Chem. Intl. Ed. Engl.,* 1994, vol. 33, 183-186
- Molecular cloning. **SAMBROOK J. et al.** A laboratory manual. Cold Spring Harbor Laboratory Press, Cold Spring Harbor, 1989
- *Biotechnol Bioeng.,* 20 September 2005, vol. 91 (6), 670-677
- **LIAO METAL.** *Biotechnol Lett.,* June 2006, vol. 28 (11), 843-848
- *Biotechnol Bioeng,* 05 November 2003, vol. 84 (3), 332-342
- **HAN KP et al.** *Cytokine,* December 2011, vol. 56 (3), 804-10